# EUROPEAN PATENT APPLICATION

(11) **EP 3 904 517 A1**
(43) Date of publication of application: **03.11.2021**
(21) Application number: 19906151.6
(22) Date of filing: 27.12.2019
(51) Int. Cl.: C12N 15/113, A61K 48/00, A61P 21/00, C07H 21/00

(54) **OLIGOMERIC NUCLEIC ACID MOLECULE AND APPLICATION THEREOF**

(30) Priority: 29.12.2018 CN 201811634268
(71) Applicant: Ractigen Therapeutics, Nantong, Jiangsu 226400 (CN)
(72) Inventor: LI, Longcheng, Nantong, Jiangsu 226400 (CN); KANG, Moorim, Nantong, Jiangsu 226400 (CN)
(74) Representative: Høiberg P/S
(86) International application number: PCT/CN2019/129025
(87) International publication number: WO 2020/135677

(57) **Abstract**

The present invention relates to a small activating nucleic acid molecule for treating spinal muscular atrophy and use thereof. The small activating nucleic acid molecule comprises a sense nucleic acid strand and an antisense nucleic acid strand, wherein the sense nucleic acid strand and the antisense nucleic acid strand are independently an oligonucleotide strand of 16 to 35 nucleotides in length, in which one nucleotide strand has at least 75% base homology or complementarity to a target selected from a promoter region of a target gene SMN2. The present invention also relates to a pharmaceutical composition comprising the small activating nucleic acid molecule disclosed herein and optionally, a pharmaceutically acceptable carrier, and a method for up-regulating the expression of a target gene in cells and methods for treating a disease induced by insufficient expression of a target gene with the small activating nucleic acid molecule or the pharmaceutical composition comprising the small activating nucleic acid molecule disclosed herein.

## Description

### TECHNICAL FIELD

The present invention relates to the technical field of nucleic acids, specifically as it relates to an oligomeric nucleic acid molecule associated with gene activation and use thereof.

### BACKGROUND

Spinal muscular atrophy (SMA) is a hereditary neuromuscular disease characterized by progressive weakness and atrophy of skeletal muscles and respiratory muscles, and is the leading hereditary disease causing the death of children under 2 years old. SMA is clinically classified into four types according to the age of onset and the severity, from the severest type SMA I to the mildest type SMA IV (1). SMA is one of the most common autosomal recessive disorders in childhood. The incidence of SMA in liveborn infants is 1/11,000, while the frequency of adult carriers is up to 1/67 to 1/40 (2), and the carrier rate in Chinese population is about 1/42 (3).

SMA is caused by the mutation of SMN1 (Survival of Motor Neuron 1) gene, and patients with SMA carry different copy number of highly homologous SMN2 gene (4;5). Both SMN1 and SMN2 genes are located in the chromosome 5q13 region, and encode the same protein called survival of motor neuron (SMN). Compared with the SMN1 gene, the SMN2 gene has 11 nucleotides which differ from those of SMN1, while other nucleotides are identical. Among the 11 nucleotides, only one nucleotide is located in the coding region of SMN2, that is the C (cytosine) of the sixth nucleotide of exon 7 substituted by a T (C6T), an exonic splicing enhancer region. This substitution does not change the coding sequence, but alters the effective splicing of exon 7 of SMN2, resulting in most of precursor messenger RNAs (pre-mRNAs) of SMN2 losing exon 7 during splicing and the translation of largely unstable mutant SMN protein (SMNΔ7) and a small amount of full-length SMN protein with normal function (5;6). Consequently, in patients with SMA, the function of the small amount of full-length SMN protein produced by SMN2 is insufficient to compensate for the loss of SMN protein caused by SMN1 mutation. Therefore, any method that can increase the expression level of full-length SMN protein from the SMN2 gene may be useful for treating SMA.

Strategies for modulating SMN2 splicing to increase full-length protein using small-molecule compounds or oligonucleotides have been demonstated to work in both animal models and clinical studies (7-11). Of them, the antisense oligonucleotide Spinraza has been approved by the FDA in 2016 as the first oligonucleotide drug for treating SMA in children and adults. Although regulating pre-mRNA splicing is an effective therapeutic strategy, its efficacy is limited by the available amount of SMN2 pre-mRNA. If the expression level of SMN2 pre-mRNA itself is unchanged or low, the amount of full-length SMN protein induced by the treatment is also limited. Another strategy for raising the level of SMN protein is to increase the transcriptional level of SMN2, so that the expression of full-length SMN2 protein will also be increased. Studies have shown that small-molecule histone deacetylase inhibitors (HDAC inhibitors) can activate the SMN2 gene and increase the transcriptional level of SMN2 by inhibiting histone deacetylase, resulting in the production of more SMN2 pre-mRNA, and demonstrateing good efficacy in animal SMA models (12;13). However, the small-molecule HDAC inhibitors did not show clinical efficacy in human patients with SMA. The possible reason is that HDAC inhibitors may upregulate many other genes after inhibiting histone deacetylase, and therefore do not have specificity and high activity for SMN2 promoter (14;15). The present invention provides a novel small activating nucleic acid molecule that can activate SMN2 transcription with high specificity by targeting SMN2 promoter.

### SUMMARY

In order to solve the aforementioned problem, the present invention provides a small activating nucleic acid molecule such as a small activating RNA (saRNA) molecule, which treats diseases or conditions caused by the lack of full-length SMN protein such as SMA by activating/up-regulating SMN2 transcription and increasing the expression level of full-length SMN protein via the RNA activation (RNAa) mechanism.

In one aspect of the present invention, a small activating nucleic acid molecule (such as saRNA molecule) activating/up-regulating the expression of SMN2 gene in a cell is provided, wherein one strand of the small activating nucleic acid molecule has at least 75% or more homology or complementarity to a fragment of 16-35 nucleotides in length in a promoter region of SMN2 gene, thereby activating or up-regulating the expression of the gene, wherein the promoter region comprises 2000 base pairs (bp) sequence upstream of a transcription start site. Specifically, one strand of the small activating nucleic acid molecule has at least 75% (e.g., at least about 79%, about 80%, about 85%, about 90%, about 95% or about 99%) homology or complementarity to a fragment of 16 to 35 continuous nucleotides in length in positions -1639 to -1481 (SEQ ID NO: 476), positions -1090 to -1008 (SEQ ID NO: 477), positions -994 to -180 (SEQ ID NO: 478), or positions -144 to -37 (SEQ ID NO: 479) upstream of the transcription start site in SMN2 gene promoter. More specifically, one strand of the small activating nucleic acid molecule has at least 75% (e.g., at least about 79%, about 80%, about 85%, about 90%, about 95% or about 99%) homology or complementarity to any nucleotide sequence selected from SEQ ID NOs: 315-471.

In the present invention, the small activating nucleic acid molecule comprises a sense nucleic acid fragment and an antisense nucleic acid fragment, wherein the sense nucleic acid fragment and the antisense nucleic acid fragment comprise complementary regions capable of forming a double-stranded nucleic acid structure between the two fragments, which can induce the expression of SMN2 gene in a cell via the RNAa mechanism. The sense nucleic acid fragment and the antisense nucleic acid fragment of the small activating nucleic acid molecule can exist either on two different nucleic acid strands or on one nucleic acid strand. When the sense nucleic acid fragment and the antisense nucleic acid fragment are located on two different strands, at least one strand of the small activating nucleic acid molecule has a 3' overhang of 0 to 6 nucleotides in length, and preferably, both strands have a 3' overhang of 2 or 3 nucleotides in length, wherein the nucleotide of the overhang is preferably dT. When the sense nucleic acid fragment and the antisense nucleic acid fragment are located on one nucleic acid strand, preferably the small activating nucleic acid molecule is a hairpin single-stranded nucleic acid molecule, wherein the complementary regions of the sense nucleic acid fragment and the antisense nucleic acid fragment form a double-stranded nucleic acid structure with each other. In the aforementioned small activating nucleic acid molecule, the sense nucleic acid fragment and the antisense nucleic acid fragment have 16 to 35 nucleotides (e.g., 16, 17, 18, 19, 20, 21, 22, 23, 24, 25, 26, 27, 28, 29, 30, 31, 32, 33, 34 or 35 nucleotides) in length.

In one embodiment, the sense strand of the small activating nucleic acid molecule disclosed herein has at least 75% (e.g., at least about 79%, about 80%, about 85%, about 90%, about 95% or about 99%) homology to any nucleotide sequence selected from SEQ ID NOs: 1-157, and the antisense strand of the small activating nucleic acid molecule disclosed herein has at least 75% (e.g., at least about 79%, about 80%, about 85%, about 90%, about 95% or about 99%) homology to any nucleotide sequence selected from SEQ ID NOs: 158-314. Specifically, the sense strand of the small activating nucleic acid molecule disclosed herein comprises, consists of or is any nucleotide sequence selected from SEQ ID NOs: 1-157; and the antisense strand of the small activating nucleic acid molecule disclosed herein comprises, consists of or is any nucleotide sequence selected from SEQ ID NOs: 158-314.

In the small activating nucleic acid molecule disclosed herein, all nucleotides may be natural or non-chemically modified nucleotides, or at least one nucleotide is a chemically modified nucleotide, and the chemical modification is one or a combination of the following modifications:
(1) modification of a phosphodiester bond of nucleotides in the nucleotide sequence of the small activating nucleic acid molecule;
(2) modification of 2'-OH of the ribose in the nucleotide sequence of the small activating nucleic acid molecule; and
(3) modification of a base in the nucleotide of the small activating nucleic acid molecule.

The chemical modification described herein is well-known to those skilled in the art, wherein the modification of the phosphodiester bond refers to the modification of oxygen in the phosphodiester bond, including phosphorothioate modification and boranophosphate modification. Both types of modifications can stabilize an saRNA structure and maintain the high specificity and high affinity for base pairing.

The ribose modification refers to the modification of 2'-OH in pentose of a nucleotide, i.e., the introduction of certain substituents into hydroxyl positions of the ribose, such as 2'-fluoro modification, 2'-oxymethyl modification, 2'-oxyethylidene methoxy modification, 2,4'-dinitrophenol modification, locked nucleic acid (LNA), 2'-amino modification and 2'-deoxy modification.

The base modification refers to the modification of the base of a nucleotide, such as 5'-bromouracil modification, 5'-iodouracil modification, N-methyluracil modification and 2,6-diaminopurine modification.

These modifications can increase the bioavailability of the small activating nucleic acid molecule, improve affinity to a target sequence, and enhance resistance to nuclease hydrolysis in a cell.

In addition, in order to promote cellular update of the small activating nucleic acid, on the basis of the aforementioned modifications, a lipophilic group, such as cholesterol, can be conjugated onto the terminus of the sense strand or antisense strand of the small activating nucleic acid molecule to facilitate transmembrance trafficking across the lipid bilayer cell membrane and nuclear envelope to finally bind with its gene promoter target in the cell nucleus.

After contacting a cell, the small activating nucleic acid molecule disclosed herein can effectively activate or upregulate the expression of SMN2 gene in a cell, preferably upregulate the expression by at least 10%.

One aspect of the present invention provides a nucleic acid encoding the small activating nucleic acid molecule disclosed herein. In one embodiment, the small activating nucleic acid molecule disclosed herein is a small activating RNA (saRNA) molecule. In one embodiment, the nucleic acid is a DNA molecule.

One aspect of the present invention provides a cell comprising the small activating nucleic acid molecule disclosed herein or the nucleic acid encoding the small activating nucleic acid molecule disclosed herein. In one embodiment, the cell is a mammalian cell, preferably a human cell. The aforementioned cell may be *in vitro,* such as a cell line or a cell strain, or may exist in a mammalian body, such as a human body.

Another aspect of the present invention provides a composition, such as a pharmaceutical composition, comprising the aforementioned small activating nucleic acid molecule or nucleic acid encoding the small activating nucleic acid molecule disclosed herein and optionally, a pharmaceutically acceptable carrier. In one embodiment, the pharmaceutically acceptable carrier includes an aqueous carrier, a liposome, a high-molecular polymer or a polypeptide. In one embodiment, the pharmaceutically acceptable carrier is selected from an aqueous carrier, a liposome, a high-molecular polymer and a polypeptide. In one embodiment, the aqueous carrier may be, for example, RNase-free water or RNase-free buffer. The composition may comprise 1-150 nM (e.g., 1-100 nM, 1-50 nM, 1-20 nM, 10-100 nM, 10-50 nM, 20-50 nM, 20-100 nM or 50 nM) of the aforementioned small activating nucleic acid molecule or nucleic acid encoding the small activating nucleic acid molecule disclosed herein.

Another aspect of the present invention relates to use of the aforementioned small activating nucleic acid molecule, nucleic acid encoding the small activating nucleic acid molecule disclosed herein or the composition comprising the aforementioned small activating nucleic acid molecule or nucleic acid encoding the small activating nucleic acid molecule disclosed herein in preparing a preparation for activating/up-regulating the expression of SMN2 gene in a cell.

The present invention also relates to a method for activating/up-regulating the expression of SMN2 gene in a cell, wherein the method comprises administering the aforementioned small activating nucleic acid molecule, the nucleic acid encoding the small activating nucleic acid molecule disclosed herein or the composition comprising the aforementioned small activating nucleic acid molecule or nucleic acid encoding the small activating nucleic acid molecule disclosed herein to the cell.

The aforementioned small activating nucleic acid molecule, the nucleic acid encoding the small activating nucleic acid molecule disclosed herein or the composition comprising the aforementioned small activating nucleic acid molecule or nucleic acid encoding the small activating nucleic acid molecule disclosed herein may be directly introduced into a cell or may be produced in the cell after a nucleotide sequence encoding the small activating nucleic acid molecule is introduced into the cell. The cell is preferably a mammalian cell, more preferably a human cell. The aforementioned cell may be *in vitro,* such as a cell line or a cell strain, or may exist in a mammalian body, such as a human body. The human body is a patient suffering from a disease or symptom caused by decreased expression of full-length SMN protein due to SMN1 gene mutation or deletion or insufficient expression, and/or insufficient expression of full-length SMN2 protein, and the small activating nucleic acid molecule, the nucleic acid encoding the small activating nucleic acid molecule disclosed herein or the composition comprising the aforementioned small activating nucleic acid molecule or the nucleic acid encoding the small activating nucleic acid molecule disclosed herein is administered in a sufficient amount to treat the disease or symptom. Specifically, the symptom caused by lack of full-length SMN protein due to SMN1 gene mutation or deletion, and/or insufficient expression of full-length SMN2 protein includes, for example, spinal muscular atrophy. In one embodiment, the disease caused by insufficient expression of full-length SMN protein or SMN1 gene mutation or deletion or insufficient expression of full-length protein is spinal muscular atrophy. In one embodiment, the spinal muscular atrophy described herein includes type I, type II, type III and type IV SMA.

Another aspect of the present invention provides an isolated target site of small activating nucleic acid molecule on SMN2 gene, wherein the target site is a continuous nucleotide sequence having a length of 16 to 35 nucleotides in the promoter region of the SMN2 gene, preferably, the target site is any sequence selected from SEQ ID NOs 476-479, having a length of 16 to 35 continuous nucleotides. Specifically, the target site comprises or is selected from any of the nucleotide sequences set forth in SEQ ID NOs: 315-471.

Another aspect of the present invention relates to a method for treating a disease caused by insufficient expression of full-length SMN protein due to SMN1 gene mutation or deletion, and/or insufficient expression of full-length SMN2 protein in an individual, which comprises administering a therapeutically effective dose of the aforementioned small activating nucleic acid molecule, the nucleic acid encoding the small activating nucleic acid molecule disclosed herein or the composition comprising the small activating nucleic acid molecule disclosed herein or the nucleic acid encoding the small activating nucleic acid molecule disclosed herein to the individual. The individual may be a mammal, such as a human. In one embodiment, the disease caused by insufficient expression of full-length SMN protein due to SMN1 gene mutation may include, for example, spinal muscular atrophy. In one embodiment, the disease caused by insufficient expression of full-length SMN protein due to SMN1 gene mutation or deletion and/or insufficient expression of full-length SMN2 protein is spinal muscular atrophy. In one embodiment, the spinal muscular atrophy described herein includes type I, type II, type III and type IV SMA.

Another aspect of the present invention relates to use of the small activating nucleic acid molecule disclosed herein, the nucleic acid encoding the small activating nucleic acid molecule disclosed herein or the composition comprising the small activating nucleic acid molecule disclosed herein or the nucleic acid encoding the small activating nucleic acid molecule disclosed herein in preparing a medicament for treating a disease or condition caused by insufficient expression of full-length SMN protein due to SMN1 gene mutation or deletion or insufficient expression of full-length protein and/or insufficient expression of full-length SMN2 protein. The individual may be a mammal, such as a human. In one embodiment, the disease caused by insufficient expression of full-length SMN protein due to SMN1 gene mutation or deletion or insufficient expression of full-length protein and/or insufficient expression of full-length SMN2 protein may include, for example, spinal muscular atrophy. In one embodiment, the disease caused by insufficient expression of full-length SMN protein due to SMN1 gene mutation or deletion or insufficient expression of full-length protein and/or insufficient expression of full-length SMN2 protein is spinal muscular atrophy. In one embodiment, the spinal muscular atrophy described herein includes type I, type II, type III and type IV SMA.

### Advantages of the Present Invention

The small activating nucleic acid molecule activating/upregulating the expression of SMN2 gene provided herein (such as an saRNA molecule) can efficiently and specifically upregulate the expression of SMN2 gene and increase the expression level of full-length SMN2 mRNA with low toxic and adverse effects, and can be used in preparing a drug for treating disorders associated with insufficient expression of full-length SMN protein and diseases or conditions caused by a SMN1 gene mutation or deletion or insufficient expression of full-length protein, and/or insufficient expression of full-length SMN2 protein.

### BRIEF DESCRIPTION OF THE DRAWINGS

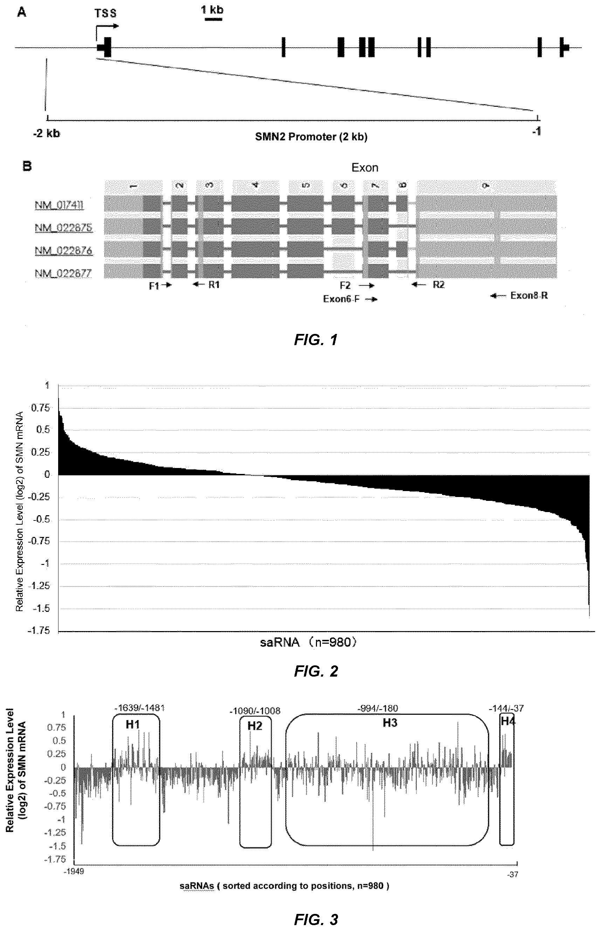

**FIG. 1** is a schematic showing SMN2 gene structure with a 2-kb promoter region used for designing saRNAs and the location of PCR primers. **FIG. 1A** shows SMN2 gene structure and a 2-kb promoter region used for designing saRNAs. **FIG. 1B** shows the location of RT-PCR primers for SMN2 mRNA amplification. SMN F1 + SMN R1 is a RT-qPCR primer pair used for high throughput saRNA screening; SMN F2 + SMN R2 is an RT-qPCR primer pair used for saRNA lead validation; and SMN-exon6-F + SMN-exon8-R is a primer pair for regular RT-PCR.
**FIG. 2** shows changes in expression level of SMN mRNA mediated by small activating nucleic acid molecules. 980 SMN2 promoter-targeting saRNAs were individually transfected into human embryonic kidney cells HEK293T. 72 h later, the expression level of SMN mRNA was analyzed by one-step RT-qPCR. The drawing shows the relative fold changes in expression level of SMN mRNA caused by each of the 980 saRNAs sorted by fold change in a descending order.
**FIG. 3** shows saRNA hotspot regions on SMN2 promoter. 980 SMN2 promoter-targeting saRNAs were individually transfected into human embryonic kidney cells HEK293T and 72 h later the expression level of SMN mRNA was analyzed by one-step RT-qPCR. The drawing shows fold changes in expression level of SMN mRNA caused by each saRNA relative to a control (Mock) treatment sorted by saRNA's location on the promoter from -1949 to -37 upstream of SMN2 transcription start site (TSS). The drawing also shows the location of 4 saRNA hotspot regions (H1-H4, rectangular boxes). The numbers above the boxes indicate the boundaries of the hotspot regions (relative to the SMN2 TSS).

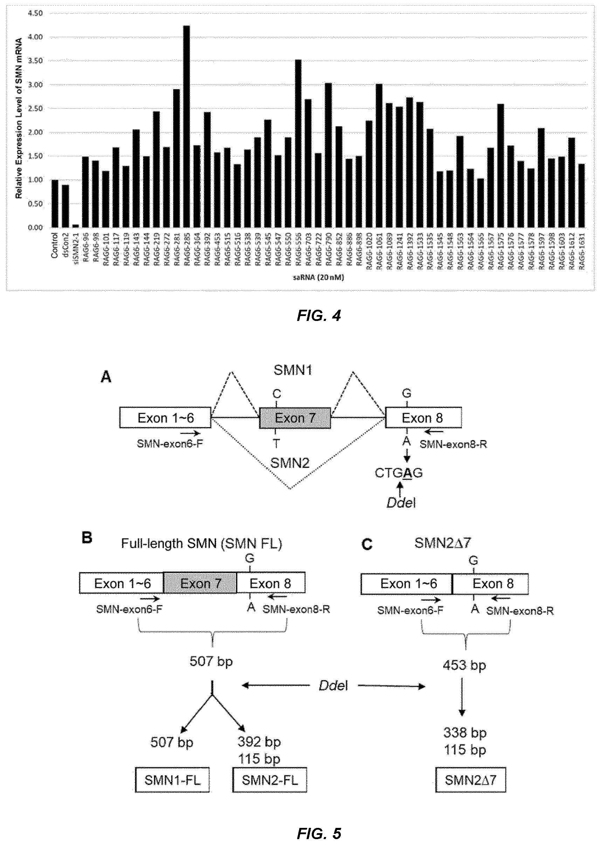

**FIG. 4** shows a quantitative assessment of 50 randomly selected saRNAs for their activity in activating the expression of SMN gene in HEK293T cells. HEK293T cells were transfected with the indicated saRNAs (n=50, final concentration: 20 nM). 72 h later, RNAs were extracted from the transfected cells using a Qiagen RNeasy kit. After reverse transcription, qPCR amplification of SMN genes was performed using a 7500FAST real-time PCR system, and mRNA levels of HPRT1 and TBP genes were determined and their geometric means was used as internal references for RNA loading normalization. Y axis represents values of changes in SMN mRNA expression level in each saRNA-treated sample relative to control (Mock) treatment after normalization by that of internal reference genes. dsCon2 and siSMN2-1 are a control duplex and an SMN2 siRNA control respectively.
**FIG. 5** is a schematic for the method of PCR amplification followed by *Dde*I restriction enzyme digestion for the detection of SMN expression. **FIG. 5A** shows the differences between SMN1 gene and SMN2 gene. A G→A variation in exon 8 of SMN2 gene creates a *Dde*l restriction enzyme cutting site. PCR amplification of cDNA using primers SMN-exon6-F and SMN-exon8-R generated full-length SMN (SMN2FL) product (507 bp) (**FIG. 5B**) and/or an exon 7-skipped/deleted (SMN2Δ7) product (453 bp) (**FIG. 5C**). In order to differtiate SMN2 products from SMN1 products, the PCR products were digested with *Dde*I enzyme and separated on an argarose gel. Product derived from full-length SMN1 could not be digested, whereas product derived from full-length SMN2 was digested into two fragements (392 bp and 115 bp) (**FIG. 5B**), and the SMN2Δ7 product was digested into a 338 bp and 115 bp fragments (**FIG. 5C**).

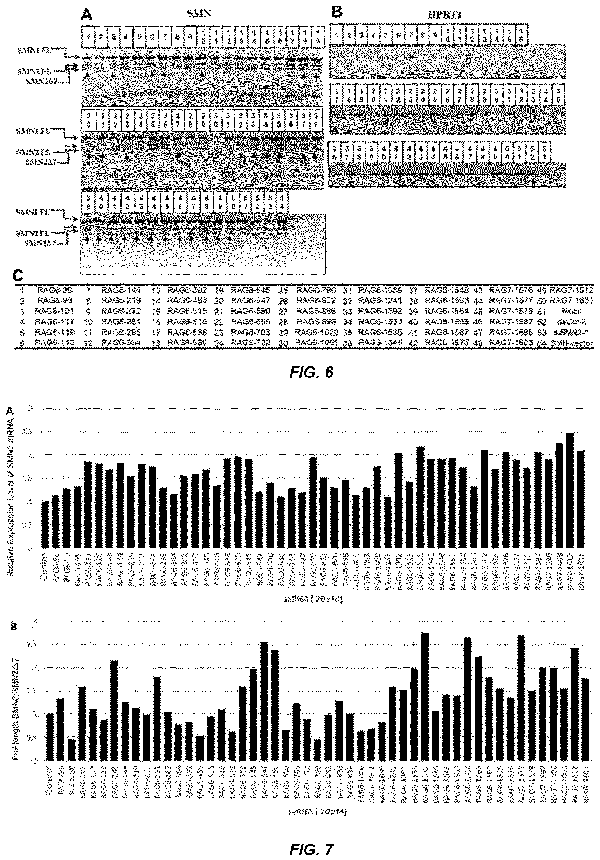

**FIG. 6** are electrophoretograms showing the activity of 50 randomly selected saRNAs in increasing the expression level of full-length SMN2 mRNA in HEK293T cells. HEK293T cells were transfected with the indicated saRNAs (n=50, final concentration: 20 nM). Control treatment included a Mock, dsCon2, siSMN2-1 and vector-mediated overexpression (SMN-vector) (bands 51, 52, 53 and 54, respectively). 72 h later, RNAs were extracted from the transfected cells using a Qiagen RNeasy kit. After reverse transcription, regular RT-PCR amplification was performed, and HPRT1 was amplified as an internal reference for RNA loading. The amplification product of SMN gene was digested with *Dde*I and then subjected to gel electrophoresis, and band intensity was quantified. The amplification product of HPRT1 was directly subjected to gel electrophoresis without digestion. **FIG. 6A** is a gel electrophoretogram of the amplification product of SMN gene after *Dde*I digestion; **FIG. 6B** is a gel electrophoretogram of the amplification product of HPRT1; and **FIG. 6C** lists the sample names of the bands in **FIG. 6A** and **FIG. 6B****.** FL: full-length amplification product; SMN2Δ7: exon 7-skipped/deleted product; SMN2 partial: SMN2-specific digested fragment. Black arrows indicate saRNAs that can increase the ratio of the full-length SMN2 product to the exon 7-skipped/deleted product.
**FIG. 7** shows the quantitative activity of 50 randomly selected saRNAs in inducing levels of total SMN2 mRNAs and full-length SMN2. The intensity of the electrophoresis bands in **FIG. 6** was quantified to derive the total mRNA levels of SMN2 (**FIG. 7A**) and the ratio of full-length SMN2 mRNA to SMN2Δ7 mRNA levels **(****FIG. 7B****).** The values were normalized by the band intensity of HPRT1 for each sample and presented as values relative to Mock treatment.

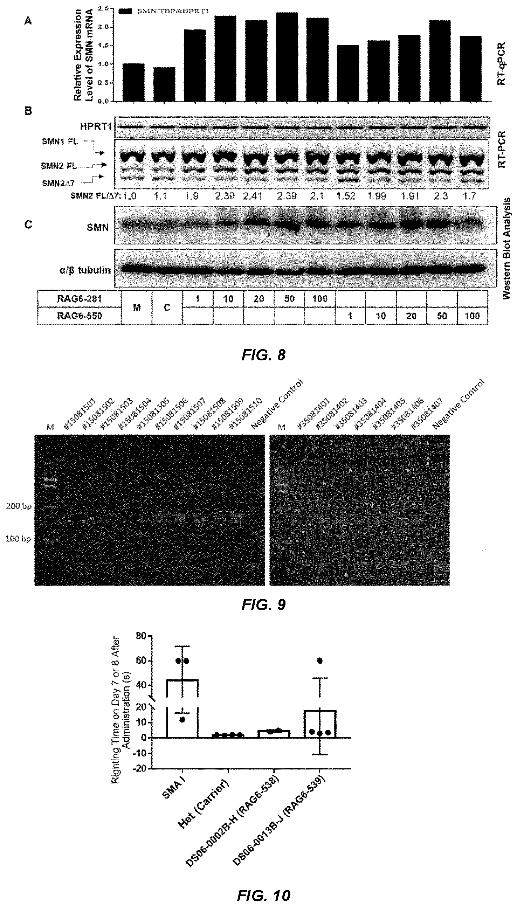

**FIG. 8** shows the dose-response relationship of saRNAs in activating the expression of SMN and SMN2FL mRNA and protein. Two SMN2 saRNAs (RAG6-281 and RAG6-550) were individually transfected into HEK293T cells at the indicated concentrations (1 nM, 10 nM, 20 nM, 50 nM and 100 nM) for 72 h. Total RNAs were extracted from the transfected cells and reverse transcripted, and protein was isolated for western blotting analysis. **FIG. 8A** shows the relative expression level of SMN total mRNAs as detected by RT-qPCR. TBP and HPRT1 were also amplified and their geometric means was used as internal references. **FIG. 8B** shows SMN mRNA levels amplified by regular RT-PCR followed by *Dde*I digestion and electrophoresis. HPRT1 was amplified as an internal reference control. The values (labeled as SMN2 FL/Δ7) listed under the electrophoretogram represent quantified ratio of full-length SMN2 to SMN2Δ7 for each treatment relative to the ratio of Mock treatment. **FIG. 8C** shows the expression of SMN protein detected by Western blotting, and α/β tubulin was assayed as an internal reference protein. M: mock transfected control; C: dsCon2 dsRNA control; FL: full-length amplification product; SMN2Δ7: exon 7-skipped/deleted product.
**FIG. 9** shows genotyping result for newborn SMA model mice. Pups were derived by corssing Smn1^{+/-}, SMN2^{-/-} mice and Smn1^{-/-},SMN2^{+/+} mice. Genotype was determined by genomic PCR. Mice with the following two genotypes were used: type 1 SMA (SMA 1) mice which carried homozygous deletion of mouse Smn gene and human SMN2 heterzygous knockin with the genotype of Smn1^{-/-}, SMN2^{+/-}; Normal control mice (Het) which carried mouse Smn heterozygous deletion and human SMN2 heterzygous knockin with the genotype of Smn1^{+/-}, SMN2^{+/-}. The PCR product for SMA I and Het mice was a single band of 160 bp and two band of 160 bp and 180 respectively.
**FIG. 10** shows improvement of motor function of SMA I mice after treatment with SMN2-saRNA. Newborn mice were divided into four groups, i.e. normal control group (Het), SMA I control group (untreated), *in vivo*-jetPEI-formulated SMN2-saRNA RAG6-539 (DS06-0013B, 1 mg/mL) group, and HKP-formulated SMN2-saRNA RAG6-538 (DS06-0002B, 2 mg/mL) group. The newborn mice were administrated by intracerebroventricular injection (ICV) on postnatal day 1 (P1). motor function of mice was assessed by righting reflex test on P7 or P8.

### DETAILED DESCRIPTION

In the present invention, the related terms are defined as follows:
The term "complementary" as used herein refers to the capability of forming base pairs between two oligonucleotide strands. The base pairs are generally formed through hydrogen bonds between nucleotides in the antiparallel oligonucleotide strands. The bases of the complementary oligonucleotide strands can be paired in the Watson-Crick manner (such as A to T, A to U, and C to G) or in any other manner allowing the formation of a duplex (such as Hoogsteen or reverse Hoogsteen base pairing).

Complementarity includes complete complementarity and incomplete complementarity. "Complete complementarity" or "100% complementarity" means that each nucleotide from the first oligonucleotide strand can form a hydrogen bond with a nucleotide at a corresponding position in the second oligonucleotide strand in the double-stranded region of the double-stranded oligonucleotide molecule, with no base pair being "mispaired". "Incomplete complementarity" means that not all the nucleotide units of the two strands are bound with each other by hydrogen bonds. For example, for two oligonucleotide strands each of 20 nucleotides in length in the double-stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. Substantial complementarity refers to at least about 75%, about 79%, about 80%, about 85%, about 90%, about 95% or 99% complementarity.

The term "oligonucleotide" as used herein refers to polymers of nucleotides, and includes, but is not limited to, single-stranded or double-stranded molecules of DNA, RNA, or DNA/RNA hybrid, oligonucleotide strands containing regularly and irregularly alternating deoxyribosyl portions and ribosyl portions, as well as modified and naturally or unnaturally existing frameworks for such oligonucleotides. The oligonucleotide for activating target gene transcription described herein is a small activating nucleic acid molecule.

The terms "oligonucleotide strand" and "oligonucleotide sequence" as used herein can be used interchangeably, referring to a generic term for short nucleotide sequences having less than 35 bases (including nucleotides in deoxyribonucleic acid (DNA) or ribonucleic acid (RNA)). In the present invention, the length of an oligonucleotide strand can be any length from 16 to 35 nucleotides.

The term "gene" as used herein refers to all nucleotide sequences required to encode a polypeptide chain or to transcribe a functional RNA. "Gene" can be an endogenous or fully or partially recombinant gene for a host cell (for example, because an exogenous oligonucleotide and a coding sequence for encoding a promoter are introduced into a host cell, or a heterogeneous promoter adjacent to an endogenous coding sequence is introduced into a host cell). For example, the term "gene" includes a nucleic acid sequence composed of exons and introns. Protein-coding sequences are, for example, sequences contained within exons in an open reading frame between an initiation codon and a termination codon, and as used herein, "gene" can comprise a gene regulatory sequence, such as a promoter, an enhancer, and all other sequences known in the art for controlling the transcription, expression or activity of another gene, no matter whether the gene contains a coding sequence or a non-coding sequence. In one case, for example, "gene" can be used to describe a functional nucleic acid containing a regulatory sequence such as a promoter or an enhancer. The expression of a recombinant gene can be controlled by one or more types of heterogeneous regulatory sequences.

The term "target gene" as used herein can refer to nucleic acid sequences, transgenes, viral or bacterial sequences, chromosomes or extrachromosomal genes that are naturally present in organisms, and/or can be transiently or stably transfected or incorporated into cells and/or chromatins thereof. The target gene can be a protein-coding gene or a non-protein-coding gene (such as a microRNA gene and a long non-coding RNA gene). The target gene generally contains a promoter sequence, and the positive regulation for the target gene can be achieved by designing a small activating nucleic acid molecule having sequence identity (also called homology) to the promoter sequence, characterized as the up-regulation of expression of the target gene. "Sequence of a target gene promoter" refers to a non-coding sequence of the target gene, and the reference of the sequence of a target gene promoter in the phrase "complementary with the sequence of a target gene promoter" of the present invention means a coding strand of the sequence, also known as a non-template strand, i.e. a nucleic acid sequence having the same sequence as the coding sequence of the gene. "Target sequence" refers to a sequence fragment in the sequence of a target gene promoter, which is homologous or complementary with a sense oligonucleotide strand or an antisense oligonucleotide strand of a small activating nucleic acid molecule.

As used herein, the terms "sense strand" and "sense oligonucleotide strand" can be used interchangeably, and the sense oligonucleotide strand of a small activating nucleic acid molecule refers to the first nucleic acid strand having sequence homology with the coding strand of the sequence of a target gene promoter in the small activating nucleic acid molecule duplex.

As used herein, the terms "antisense strand" and "antisense oligonucleotide strand" can be used interchangeably, and the antisense oligonucleotide strand of a small activating nucleic acid molecule refers to the second nucleic acid strand which is complementary with the sense oligonucleotide strand in the small activating nucleic acid molecule duplex.

The term "coding strand" as used herein refers to a DNA strand in the target gene which cannot be used for transcription, and the nucleotide sequence of this strand is the same as that of a RNA produced from transcription (in the RNA, T in DNA is replaced by U). The coding strand of the double-stranded DNA sequence of the target gene promoter described herein refers to a promoter sequence on the same DNA strand as the DNA coding strand of the target gene.

The term "template strand" as used herein refers to the other strand complementary with the coding strand in the double-stranded DNA of the target gene, i.e. the strand that, as a template, can be transcribed into RNA, and this strand is complementary with the transcribed RNA (A to U and G to C). In the process of transcription, RNA polymerase binds to the template strand, moves along the 3'→5' direction of the template strand, and catalyzes the synthesis of the RNA along the 5'→3' direction. The template strand of the double-stranded DNA sequence of the target gene promoter described herein refers to a promoter sequence on the same DNA strand as the DNA template strand of the target gene.

The term "promoter" as used herein refers to a sequence which is spatially associated with a protein-coding or RNA-coding nucleoic acid sequence and plays a regulatory role for the transcription of the protein-coding or RNA-coding nucleic acid sequence. Generally, a eukaryotic gene promoter contains 100 to 5000 base pairs, although this length range is not intended to limit the term "promoter" as used herein. Although the promoter sequence is generally located at the 5' terminus of a protein-coding or RNA-coding sequence, it may also exist in exon and intron sequences.

The term "transcription start site" as used herein refers to a nucleotide marking the transcription start on the template strand of a gene. The transcription start site can appear on the template strand of the promoter region. A gene can have more than one transcription start site.

The term "identity" or "homology" as used herein means that one oligonucleotide strand (sense or antisense strand) of an saRNA has sequence similarity with a coding strand or template strand in a region of the promoter sequence of a target gene. As used herein, the "identity" or "homology" may be at least about 75%, about 79%, about 80%, about 85%, about 90%, about 95% or 99%.

The term "overhang" as used herein refers to non-base-paired nucleotides at the terminus (5' or 3') of an oligonucleotide strand, which is formed by one strand extending out of the other strand in a double-stranded oligonucleotide. A single-stranded region extending out of the 3' terminus and/or 5' terminus of a duplex is referred to as an overhang.

As used herein, the terms "gene activation" or "activating gene expression" and "gene up-regulation" or "up-regulating gene expression" can be used interchangeably, and mean an increase in transcription, translation, expression or activity of a certain nucleic acid as determined by measuring the transcriptional level, mRNA level, protein level, enzymatic activity, methylation state, chromatin state or configuration, translation level or the activity or state in a cell or biological system of a gene. These activities or states can be determined directly or indirectly. In addition, "gene activation", "activating gene expression", "gene up-regulation" or "up-regulating gene expression" refers to an increase in activity associated with a nucleic acid sequence, regardless of the mechanism of such activation. For example, gene activation occurs at the transcriptional level to increase transcription into RNA and the RNA is translated into a protein, thereby increasing the expression of the protein.

As used herein, the terms "small activating RNA", "saRNA", and "small activating nucleic acid molecule" can be used interchangeably, and refer to a nucleic acid molecule that can upregulate target gene expression and can be composed of a first nucleic acid fragment (antisense strand, also referred to as antisense oligonucleotide strand) containing a nucleotide sequence having sequence identity to the non-coding nucleic acid sequence (e.g., a promoter and an enhancer) of a target gene and a second nucleic acid fragment (sense strand, also referred to as sense oligonucleotide strand) containing a nucleotide sequence complementary with the first nucleic acid fragment, wherein the first nucleic acid fragment and the second nucleic acid fragment form a duplex. The small activating nucleic acid molecule can also be comprised of a synthesized or vector-expressed single-stranded RNA molecule that can form a hairpin structure by two complementary regions within the molecule, wherein the first region contains a nucleotide sequence having sequence identity to the target sequence of a promoter of a gene, and the second region contains a nucleotide sequence which is complementary with the first region. The length of the duplex region of the small activating nucleic acid molecule is typically about 10 to about 50, about 12 to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45 or about 50 base pairs. In addition, the terms "saRNA", "small activating RNA", and "small activating nucleic acid molecule" also contain nucleic acids other than the ribonucleotide, including, but not limited to, modified nucleotides or analogues.

As used herein, the term "hotspot" refers to a gene promoter region of at least 30 bp in length where functional small activating nucleic acid molecules are enriched, i.e., at least 30% of the small activating nucleic acid molecules designed to target this region is functional and can induce a 1.2-fold or more change in the mRNA expression of the target gene.

As used herein, the term "synthesis" refers to a method for synthesis of an oligonucleotide, including any method allowing RNA synthesis, such as chemical synthesis, *in vitro* transcription, and/or vector-based expression.

In the present invention, the expression of SMN2 gene is upregulated by RNA activation, and a related disease (particularly spinal muscular atrophy) is treated by increasing the expression level of full-length SMN protein. The SMN2 gene is sometimes also called a target gene in the present invention.

The present invention provides a method for preparing the small activating nucleic acid molecule, which comprises sequence design and synthesis.

Small activating nucleic acid molecules can be chemically synthesized or can be obtained from a biotechnology company specialized in nucleic acid synthesis.

Generally speaking, chemical synthesis of nucleic acids comprises the following four steps: (1) synthesis of oligomeric ribonucleotides; (2) deprotection; (3) purification and isolation; (4) desalination and annealing.

For example, the specific steps for chemically synthesizing saRNAs described herein are as follows:

### (1) Synthesis of oligomeric ribonucleotides

Synthesis of 1 µM RNA was set in an automatic DNA/RNA synthesizer (e.g., Applied Biosystems EXPEDITE8909), and the coupling time of each cycle was set as 10 to 15 min. With a solid phase-bonded 5'-O-p-dimethoxytriphenylmethyl-thymidine substrate as an initiator, one base was bonded to the solid phase substrate in the first cycle, and then, in the nth (19 ≥ n ≥ 2) cycle, one base was bonded to the base bonded in the n-1th cycle. This process was repeated until the synthesis of the whole nucleic acid sequence was completed.

### (2). Deprotection

The solid phase substrate bonded with the saRNA was put into a test tube, and 1 mL of a solution of the mixture of ethanol and ammonium hydroxide (volume ratio: 1:3) was added to the test tube. The test tube was then sealed and placed in an incubator, and the mixture was incubated at 25-70 °C for 2 to 30 h. The solution containing the solid phase substrate bonded with the saRNA was filtered, and the filtrate was collected. The solid phase substrate was rinsed with double distilled water twice (1 mL each time), and the filtrate was collected. The collected eluents were combined and dried under vacuum for 1 to 12 h. Then the solution was added with 1 mL of a solution of tetrabutylammonium fluoride in tetrahydrofuran (1 M), let stand at room temperature for 4 to 12 h, followed by addition of 2 mL of *n*-butanol. Precipitate was collected to give a single-stranded crude product of saRNA by high-speed centrifugation.

### (3) Purification and isolation

The resulting crude product of saRNA was dissolved in 2 mL of aqueous ammonium acetate solution with a concentration of 1 mol/mL, and the solution was separated by a reversed-phase C18 column of high-pressure liquid chromatography to give a purified single-stranded product of saRNA.

### (4) Desalination and annealing

Salts were removed by gel filtration (size exclusion chromatography). A single sense oligomeric ribonucleic acid strand and a single antisense oligomeric ribonucleic acid strand were mixed into 1 to 2 mL of buffer (10 mM Tris, pH = 7.5-8.0, 50 mM NaCl) at a molar ratio of 1:1. The solution was heated to 95 °C, and was then slowly cooled to room temperature to give a solution containing saRNA.

It was discovered in this study that after being introduced into a cell, the aforementioned saRNA can effectively increase the expression level of full-length SMN2 mRNA and protein.

The present invention will be further illustrated with reference to specific examples and drawings below. It should be understood that these examples are merely intended to illustrate the present invention rather than limit the scope of the present invention. In the following examples, study methods without specific conditions were generally in accordance with conventional conditions, such as conditions described in Sambrook, et al., Molecular Cloning: Laboratory Manual (New York: Cold Spring Harbor Laboratory Press, 1989), or conditions recommended by the manufacturer.

### EXAMPLES

### Example 1: Design and Synthesis of Small Activating Nucleic Acid Molecules Targeting SMN2 Promoter

The sense promoter sequence of SMN2 gene from the transcription start site (TSS) to upstream -2000 bp was retrieved from the UCSC genome database.

In order to identify functional small activating RNAs (saRNAs) capable of activating the expression of SMN2 gene, a series of 19-nt saRNA targets were selected on a 2000-bp SMN2 promoter sequence (**FIG. 1**), starting from -2000 bp upstream of the TSS, and moving toward the TSS by 1 bp each time, resulting in a total of 1982 targets. The target sequences were then filtered to keep those which met the following criteria: (1) having a GC content between 35% and 65%; (2) with less than 5 consecutive identical nucleotides; (3) with 3 or less dinucleotide repeats; (4) with 3 or less trinucleotide repeats. After the filtration, 980 target sequences remained and their corresponding double-stranded saRNAs were chemically synthesized. Each of the sense strand and antisense strand in the saRNA used in the study were 21 nt in length. The 19 nucleotides in the 5' region of the first ribonucleic acid strand (sense strand) of the saRNA had 100% sequence identity to the target sequence of the promoter, and the 3' terminus of the first ribonucleic acid strand was a dTdT sequence. The 19 nucleotides in the 5' region of the second ribonucleic acid strand were complementary with the first ribonucleic acid strand sequence, and the 3' terminus of the second ribonucleic acid strand was a dTdT sequence. The aforementioned two strands of the saRNA were mixed at a molar ratio of 1:1 and annealed to obtain a duplex saRNA.

### Example 2: High Throughput Screening of saRNAs Targeting SMN2 Promoter

### 1) Cell culture and transfection

Human embryonic kidney cell line HEK293T (ATCC® CRL-3216™) was cultured in DMEM media (Gibco) containing 10% of fetal bovine serum (Gibco) and 1% of penicillin/streptomycin (Gibco). The cells were cultured in 5% CO₂ at 37°C. The HEK293T cells were seeded into 96-well plates at 5000 cells/well. saRNAs were individually transfected into the HEK293T cells in each well at a final concentration of 10 nM (unless otherwise stated) with 0.3 µL of RNAiMAX (Invitrogen, Carlsbad, CA) by following the revserse transfection protocol, and the transfection duration was 72 hours. Control treatment included a blank control (Mock), a sequence nonspecific oligonucleotide duplex (dsCon2, sense strand 5'-ACUACUGAGUGACAGUAGA[dT][dT]-3' (SEQ ID NO: 472), antisense strand 5'-UCUACUGUCACUCAGUAGU[dT][dT]-3' (SEQ ID NO: 473)), an SMN2 small interference RNA (siMSN2-1, sense strand 5'-GGGAUGAUACAGCACUGAU[dT][dT]-3' (SEQ ID NO: 474), antisense strand 5'AUCAGUGCUGUAUCAUCCC[dT][dT]-3' (SEQ ID NO: 475)), wherein blank control (Mock) treatment was transfection omitting nucleic acid.

### 2) One-step RT-qPCR

At the end of transfection, the media were discarded, and each well was washed with 150 µL of PBS once. After discarding the PBS, 100 µL of cell lysis buffer (Power SYBR® Green Cells-to-Ct™ Kit, Life Technologies) was added into each well and incubated at room temperature for 5 min. 0.5 µL of the resulted cell lysis was taken from each well and analyzed by RT-qPCR using One Step TB Green™ PrimeScrip™ RT-PCR kit II (Takara, RR086A) in a Roche Lightcycler 480 real-time PCR machine. PCR reactions was parepared using Bravo Automated Liquid Handling Platform (Agilent). Each transfection sample was amplified in 3 repeat wells. PCR reaction conditions are shown in Table 1.

**Table 1. PCR reaction preparation**

| Reagent | Volume/Reaction |
|---|---|
| 2 × One Step TB Green RT-PCR buffer 4 | 2.5 µL |
| PrimeScript 1 step enzyme mixture 2 | 0.2 µL |
| Mixture of forward and reverse primers (5 µM) | 0.4 µL |
| dH₂O without RNase | 1.4 µL |
| Crude lysate (RNA) | 0.5 µL |
| Total volume | 5 µL |

Reaction conditions were as follows: reverse transcription reaction (stage 1): 5 min at 42°C, 10 s at 95°C; PCR reaction (stage 2): 5 s at 95°C, 20 s at 60°C, 45 cycles of amplification. HPRT1 and TBP were also amplified as internal reference genes. PCR primers used for amplifying SMN, HPRT1 and TBP genes are shown in Table 4, wherein SMN was amplified using the SMN F1/R1 primer pair.

To calculate the expression level (*Eᵣₑₗ*) of SMN2 (target gene) in an saRNA-transfected sample relative to control treatment (Mock), the Ct values of the target gene and the two internal reference genes were substituted into formula 1, ${E}_{rel} = {2}^{\left(CtTm-CtTs\right)} / \left({\left({2}^{\left(CtR1m-CtR1s\right)}∗{2}^{\left(CtR2m-CtR2s\right)}\right)}^{\left(1/2\right)}\right)$ wherein CtTm was the Ct value of the target gene from the mock-treated sample; CtTs was the Ct value of the target gene from the saRNA-treated sample; CtR1ₘ was the Ct value of the internal reference gene 1 from the mock-treated sample; CtR1ₛ was the Ct value of the internal reference gene 1 from the saRNA-treated sample; CtR2m was the Ct value of the internal reference gene 2 from the mock-treated sample; and CtR2s was the Ct value of the internal reference gene 2 from the saRNA-treated sample.

### 3) Screening of functional saRNAs

In order to identify saRNAs capable of activating SMN2 transcription, HEK293T cells were transfected with each of the aforementioned 980 saRNAs with a transfection concentration of 10 nM. Seventy-two hours later, the lysed cells were analyzed by one-step RT-qPCR to obtain relative (compared with Mock treatment) expression level of SMN2 gene for each saRNA-treated sample. As shown in Table 2, 157 (16.02%) and 416 (42.45%) saRNAs exhibited activating and inhibiting activities respectively, and 407 (41.53%) saRNAs had no obvious effect on the expression of SMN2. The observed maximum activation and maximum inhibition were of 1.82 fold and of 0.33 fold, respectively. saRNAs with activating activity are referred to as functional saRNAs. Their target sequences, sense and antisense sequences and corresponding relative SMN expression levels are listed in Table 3.

**Table 2. Summary of high-throughput screening of SMN2 saRNAs**

| saRNA functional catetory | log₂ value (fold change) for SMN mRNA level | Number of saRNAs | Percentage |
|---|---|---|---|
| High activation | ≥ 0.49 (1.50) — ≤ 0.86 (1.82) | 10 | 1.0 |
| Moderate activation | ≥ 0.26 (1.20) — < 0.49 (1.50) | 54 | 5.5 |
| Mild activation | ≥ 0.13 (1.10) — < 0.26 (1.20) | 93 | 9.5 |
| No effect | < 0.13 (1.10) — > ―0.13 (0.91) | 407 | 41.5 |
| Mild inhibition | ≤ -0.13 (0.91) — ≥ -0.26 (0.84) | 201 | 20.5 |
| Moderate inhibition | ≤ -0.26 (0.84) — > -0.49 (0.71) | 171 | 17.4 |
| High inhibition | ≤ -0.49 (0.71) — ≥ ―1.58 (0.33) | 44 | 4.5 |
| Total | | 980 | 100 |

**Table 3. List of functional saRNA sequences, their target sequences and expression of SMN**

| **saRNA** | **Target sequence (5'-3')** | **Sense sequence (5'-3')** | **Antisense sequence (5'-3')** | **Relative expression of SMN mRNA (fold change)** | **Relative expression of SMN mRNA (log2)** |
|---|---|---|---|---|---|
| RAG6-1763 | ATCTGTGAGATGTACCTTT (SEQ ID NO:315) | AUCUGUGAGAUGUACCUUU[dT][dT] (SEQ ID NO:1) | AAAGGUACAUCUCACAGAU[dT][dT] (SEQ ID NO:158) | 1.20 | 0.26 |
| RAG6-1634 | CACTCTGTCACTCAGGCTG (SEQ ID NO:316) | CACUCUGUCACUCAGGCUG[dT][dT] (SEQ ID NO:2) | CAGCCUGAGUGACAGAGUG[dT][dT] (SEQ ID NO:159) | 1.11 | 0.16 |
| RAG6-1633 | ACTCTGTCACTCAGGCTGG (SEQ ID NO:317) | ACUCUGUCACUCAGGCUGG[dT][dT] (SEQ ID NO:3) | CCAGCCUGAGU GACAGAGU [dT][dT] (SEQ ID NO:160) | 1.13 | 0.17 |
| RAG6-1631 | TCTGTCACTCAGGCTGGAG (SEQ ID NO:318) | UCUGUCACUCAGGCUGGAG[dT][dT] (SEQ ID NO:4) | CUCCAGCCUGAGUGACAGA[dT][dT] (SEQ ID NO:161) | 1.29 | 0.36 |
| RAG6-1623 | TCAGGCTGGAGTGCAGTGG (SEQ ID NO:319) | UCAGGCUGGAGUGCAGUGG[dT][dT] (SEQ ID NO:5) | CCACUGCACUCCAGCCUGA[dT][dT] (SEQ ID NO:162) | 1.11 | 0.15 |
| RAG6-1615 | GAGTGCAGTGGCGTGATCT (SEQ ID NO:320) | GAGUGCAGUGGCGUGAUCU[dT][dT] (SEQ ID NO:6) | AGAUCACGCCACUGCACUC[dT][dT] (SEQ ID NO:163) | 1.16 | 0.22 |
| RAG6-1612 | TGCAGTGGCGTGATCTTGG (SEQ ID NO:321) | UGCAGUGGCGUGAUCUUGG[dT][dT] (SEQ ID NO:7) | CCAAGAUCACGCCACUGCA[dT][dT] (SEQ ID NO:164) | 1.25 | 0.33 |
| RAG6-1611 | GCAGTGGCGTGATCTTGGC (SEQ ID NO:322) | GCAGUGGCGUGAUCUUGGC[dT][dT] (SEQ ID NO:8) | GCCAAGAUCACGCCACUGC[dT][dT] (SEQ ID NO:165) | 1.13 | 0.17 |
| RAG6-1606 | GGCGTGATCTTGGCTCACT (SEQ ID NO:323) | GGCGUGAUCUUGGCUCACU[dT][dT] (SEQ ID NO:9) | AGUGAGCCAAGAUCACGCC[dT][dT] (SEQ ID NO:166) | 1.11 | 0.15 |
| RAG6-1603 | GTGATCTTGGCTCACTGCA (SEQ ID NO:324) | GUGAUCUUGGCUCACUGCA[dT][dT] (SEQ ID NO:10) | UGCAGUGAGCCAAGAUCAC[dT][dT] (SEQ ID NO:167) | 1.24 | 0.31 |
| RAG6-1602 | TGATCTTGGCTCACTGCAA (SEQ ID NO:325) | UGAUCUUGGCUCACUGCAA[dT][dT] (SEQ ID NO:11) | UUGCAGUGAGCCAAGAUCA[dT][dT] (SEQ ID NO:168) | 1.12 | 0.16 |
| RAG6-1600 | ATCTTGGCTCACTGCAACC (SEQ ID NO:326) | AUCUUGGCUCACUGCAACC[dT][dT] (SEQ ID NO:12) | GGUUGCAGUGAGCCAAGAU[dT][dT] (SEQ ID NO:169) | 1.13 | 0.18 |
| RAG6-1598 | CTTGGCTCACTGCAACCTC (SEQ ID NO:327) | CU UGGCUCACUGCAACCUC [dT][dT] (SEQ ID NO:13) | GAGGUUGCAGUGAGCCAAG[dT][dT] (SEQ ID NO:170) | 1.50 | 0.59 |
| RAG6-1597 | TTGGCTCACTGCAACCTCC (SEQ ID NO:328) | UUGGCUCACUGCAACCUCC[dT][dT] (SEQ ID NO:14) | GGAGGUUGCAGUGAGCCAA[dT][dT] (SEQ ID NO:171) | 1.34 | 0.42 |
| RAG6-1578 | GCCTCCCGAGTTCAAGTGA (SEQ ID NO:329) | GCCUCCCGAGUUCAAGUGA[dT][dT] (SEQ ID NO:15) | UCACUUGAACUCGGGAGGC[dT][dT] (SEQ ID NO:172) | 1.36 | 0.45 |
| RAG6-1577 | CCTCCCGAGTTCAAGTGAT (SEQ ID NO:330) | CCUCCCGAGUUCAAGUGAU[dT] [dT] (SEQ ID NO:16) | AUCACUUGAACUCGGGAGG[dT][dT] (SEQ ID NO:173) | 1.25 | 0.32 |
| RAG6-1576 | CTCCCGAGTTCAAGTGATT (SEQ ID NO:331) | CUCCCGAGUUCAAGUGAUU[dT] [dT] (SEQ ID NO:17) | AAUCACUUGAACUCGGGAG[dT][dT] (SEQ ID NO:174) | 1.31 | 0.39 |
| RAG6-1575 | TCCCGAGTTCAAGTGATTC (SEQ ID NO:332) | UCCCGAGUUCAAGUGAUUC[dT][dT] (SEQ ID NO:18) | GAAUCACUUGAACUCGGGA[dT][dT] (SEQ ID NO:175) | 1.24 | 0.31 |
| RAG6-1567 | TCAAGTGATTCTCCTGGCT (SEQ ID NO:333) | UCAAGUGAUUCUCCUGGCU[dT][dT] (SEQ ID NO:19) | AGCCAGGAGAAUCACUUGA[dT][dT] (SEQ ID NO:176) | 1.25 | 0.33 |
| RAG6-1565 | AAGTGATTCTCCTGGCTCA (SEQ ID NO:334) | AAGUGAUUCUCCUGGCUCA[dT][dT] (SEQ ID NO:20) | UGAGCCAGGAGAAUCACUU[dT][dT] (SEQ ID NO:177) | 1.24 | 0.31 |
| RAG6-1564 | AGTGATTCTCCTGGCTCAG (SEQ ID NO:335) | AGUGAUUCUCCUGGCUCAG[dT][dT] (SEQ ID NO:21) | CUGAGCCAGGAGAAUCACU[dT][dT] (SEQ ID NO:178) | 1.30 | 0.38 |
| RAG6-1563 | GTGATTCTCCTGGCTCAGC (SEQ ID NO:336) | GUGAUUCUCCUGGCUCAGC[dT][dT] (SEQ ID NO:22) | GCUGAGCCAGGAGAAUCAC[dT][dT] (SEQ ID NO:179) | 1.64 | 0.72 |
| RAG6-1548 | CAGCCTCCCAAGCAGCTGT (SEQ ID NO:337) | CAGCCUCCCAAGCAGCUGU [dT][dT] (SEQ ID NO:23) | ACAGCUGCUUGGGAGGCUG[dT][dT] (SEQ ID NO:180) | 1.29 | 0.37 |
| RAG6-1545 | CCTCCCAAGCAGCTGTCAT (SEQ ID NO:338) | CCUCCCAAGCAGCUGUCAU[dT][dT] (SEQ ID NO:24) | AUGACAGCUGCUUGGGAGG[dT][dT] (SEQ ID NO:181) | 1.59 | 0.67 |
| RAG6-1543 | TCCCAAGCAGCTGTCATTA (SEQ ID NO:339) | U CCCAAGCAGCU GU CAU UA [dT][dT] (SEQ ID NO:25) | UAAUGACAGCUGCUUGGGA[dT][dT] (SEQ ID NO:182) | 1.10 | 0.14 |
| RAG6-1535 | AGCTGTCATTACAGGCCTG (SEQ ID NO:340) | AGCUGUCAUUACAGGCCUG[dT][dT] (SEQ ID NO:26) | CAGGCCUGUAAUGACAGCU[dT][dT] (SEQ ID NO:183) | 1.59 | 0.66 |
| RAG6-1534 | GCTGTCATTACAGGCCTGC (SEQ ID NO:341) | GCUGUCAUUACAGGCCUGC[dT][dT] (SEQ ID NO:27) | GCAGGCCUGUAAUGACAGC[dT][dT] (SEQ ID NO:184) | 1.14 | 0.19 |
| RAG6-1533 | CTGTCATTACAGGCCTGCA (SEQ ID NO:342) | CUGUCAUUACAGGCCUGCA[dT][dT] (SEQ ID NO:28) | UGCAGGCCUGUAAUGACAG[dT][dT] (SEQ ID NO:185) | 1.28 | 0.36 |
| RAG6-1483 | GGAGAAACAGGGTTTCACC (SEQ ID NO:343) | GGAGAAACAGGGUUUCACC[dT][dT] (SEQ ID NO:29) | GGUGAAACCCUGUUUCUCC[dT][dT] (SEQ ID NO:186) | 1.12 | 0.17 |
| RAG6-1481 | AGAAACAGGGTTTCACCAT (SEQ ID NO:344) | AGAAACAGGGUUUCACCAU[dT][dT] (SEQ ID NO:30) | AUGGUGAAACCCUGUUUCU[dT][dT] (SEQ ID NO:187) | 1.14 | 0.19 |
| RAG6-1403 | AAGTGCTGGGATTATAGGC (SEQ ID NO:345) | AAGUGCUGGGAUUAUAGGC[dT][dT] (SEQ ID NO:31) | GCCUAUAAUCCCAGCACUU [dT][dT] (SEQ ID NO:188) | 1.21 | 0.28 |
| RAG6-1392 | TTATAGGCATGAGCCACCG (SEQ ID NO:346) | UUAUAGGCAUGAGCCACCG[dT][dT] (SEQ ID NO:32) | CGGUGGCUCAUGCCUAUAA[dT][dT] (SEQ ID NO:189) | 1.23 | 0.30 |
| RAG6-1241 | ATTCTCCCCTTCCTCCACA (SEQ ID NO:347) | AUUCUCCCCUUCCUCCACA[dT][dT] (SEQ ID NO:33) | UGUGGAGGAAGGGGAGAAU[dT][dT] (SEQ ID NO:190) | 1.26 | 0.33 |
| RAG6-1239 | TCTCCCCTTCCTCCACAGA (SEQ ID NO:348) | UCUCCCCUUCCUCCACAGA[dT][dT] (SEQ ID NO:34) | UCUGUGGAGGAAGGGGAGA[dT][dT] (SEQ ID NO:191) | 1.10 | 0.13 |
| RAG6-1119 | CATTTAGCAACCCTAGATG (SEQ ID NO:349) | CAUUUAGCAACCCUAGAUG[dT][dT] (SEQ ID NO:35) | CAUCUAGGGUUGCUAAAUG[dT][dT] (SEQ ID NO:192) | 1.11 | 0.14 |
| RAG6-1118 | ATTTAGCAACCCTAGATGC (SEQ ID NO:350) | AUUUAGCAACCCUAGAUGC[dT][dT] (SEQ ID NO:36) | GCAUCUAGGGUUGCUAAAU [dT][dT] (SEQ ID NO:193) | 1.13 | 0.18 |
| RAG6-1117 | TTTAGCAACCCTAGATGCT (SEQ ID NO:351) | UUUAGCAACCCUAGAUGCU [dT][dT] (SEQ ID NO:37) | AGCAUCUAGGGUUGCUAAA[dT][dT] (SEQ ID NO:194) | 1.14 | 0.19 |
| RAG6-1116 | TTAGCAACCCTAGATGCTT (SEQ ID NO:352) | UUAGCAACCCUAGAUGCUU [dT][dT] (SEQ ID NO:38) | AAGCAUCUAGGGUUGCUAA[dT][dT] (SEQ ID NO:195) | 1.19 | 0.26 |
| RAG6-1115 | TAGCAACCCTAGATGCTTA (SEQ ID NO:353) | UAGCAACCCUAGAUGCUUA[dT][dT] (SEQ ID NO:39) | U AAGCAUCUAGGGUUGCUA[dT][dT] (SEQ ID NO:196) | 1.22 | 0.29 |
| RAG6-1089 | ATACTGGAGGCCCGGTGTG (SEQ ID NO:354) | AUACUGGAGGCCCGGUGUG[dT][dT] (SEQ ID NO:40) | CACACCGGGCCUCCAGUAU [dT][dT] (SEQ ID NO:197) | 1.61 | 0.69 |
| RAG6-1075 | GTGTGGTGGCTCACACCTG (SEQ ID NO:355) | GUGUGGUGGCUCACACCUG[dT][dT] (SEQ ID NO:41) | CAGGUGUGAGCCACCACAC[dT][dT] (SEQ ID NO:198) | 1.11 | 0.15 |
| RAG6-1072 | TGGTGGCTCACACCTGTAA (SEQ ID NO:356) | UGGUGGCUCACACCUGUAA[dT][dT] (SEQ ID NO:42) | UUACAGGUGUGAGCCACCA[dT][dT] (SEQ ID NO:199) | 1.14 | 0.20 |
| RAG6-1071 | GGTGGCTCACACCTGTAAT (SEQ ID NO:357) | GGUGGCUCACACCUGUAAU [dT][dT] (SEQ ID NO:43) | AUUACAGGUGUGAGCCACC[dT][dT] (SEQ ID NO:200) | 1.15 | 0.21 |
| RAG6-1070 | GTGGCTCACACCTGTAATC (SEQ ID NO:358) | GUGGCUCACACCUGUAAUC[dT][dT] (SEQ ID NO:44) | GAUUACAGGUGUGAGCCAC[dT][dT] (SEQ ID NO:201) | 1.17 | 0.23 |
| RAG6-1068 | GGCTCACACCTGTAATCCC (SEQ ID NO:359) | GGCUCACACCUGUAAUCCC [dT][dT] (SEQ ID NO:45) | GGGAUUACAGGUGUGAGCC[dT][dT] (SEQ ID NO:202) | 1.22 | 0.29 |
| RAG6-1064 | CACACCTGTAATCCCAGCA (SEQ ID NO:360) | CACACCUGUAAUCCCAGCA[dT][dT] (SEQ ID NO:46) | UGCUGGGAUUACAGGUGUG[dT][dT] (SEQ ID NO:203) | 1.19 | 0.26 |
| RAG6-1063 | ACACCTGTAATCCCAGCAC (SEQ ID NO:361) | ACACCUGUAAUCCCAGCAC[dT][dT] (SEQ ID NO:47) | GUGCUGGGAUUACAGGUGU[dT][dT] (SEQ ID NO:204) | 1.16 | 0.21 |
| RAG6-1061 | ACCTGTAATCCCAGCACTT (SEQ ID NO:362) | ACCUGUAAUCCCAGCACUU[dT][dT] (SEQ ID NO:48) | AAGUGCUGGGAUUACAGGU [dT][dT] (SEQ ID NO:205) | 1.34 | 0.42 |
| RAG6-1057 | GTAATCCCAGCACTTTGGG (SEQ ID NO:363) | GUAAUCCCAGCACUUUGGG[dT][dT] (SEQ ID NO:49) | CCCAAAGUGCUGGGAUUAC[dT][dT] (SEQ ID NO:206) | 1.10 | 0.14 |
| RAG6-1056 | TAATCCCAGCACTTTGGGA (SEQ ID NO:364) | UAAUCCCAGCACUUUGGGA[dT][dT] (SEQ ID NO:50) | UCCCAAAGUGCUGGGAUUA[dT][dT] (SEQ ID NO:207) | 1.17 | 0.22 |
| RAG6-1055 | AATCCCAGCACTTTGGGAG (SEQ ID NO:365) | AAUCCCAGCACUUUGGGAG[dT][dT] (SEQ ID NO:51) | CUCCCAAAGUGCUGGGAUU [dT][dT] (SEQ ID NO:208) | 1.14 | 0.18 |
| RAG6-1050 | CAGCACTTTGGGAGGCCGA (SEQ ID NO:366) | CAGCACUUUGGGAGGCCGA[dT][dT] (SEQ ID NO:52) | UCGGCCUCCCAAAGUGCUG[dT][dT] (SEQ ID NO:209) | 1.14 | 0.19 |
| RAG6-1033 | GAGGCGGTCGGATTACGAG (SEQ ID NO:367) | GAGGCGGUCGGAUUACGAG[dT][dT] (SEQ ID NO:53) | CUCGUAAUCCGACCGCCUC[dT][dT] (SEQ ID NO:210) | 1.15 | 0.20 |
| RAG6-1031 | GGCGGTCGGATTACGAGGT (SEQ ID NO:368) | GGCGGUCGGAUUACGAGGU[dT][dT] (SEQ ID NO:54) | ACCUCGUAAUCCGACCGCC[dT][dT] (SEQ ID NO:211) | 1.11 | 0.16 |
| RAG6-1030 | GCGGTCGGATTACGAGGTC (SEQ ID NO:369) | GCGGUCGGAUUACGAGGUC[dT][dT] (SEQ ID NO:55) | GACCUCGUAAUCCGACCGC[dT][dT] (SEQ ID NO:212) | 1.11 | 0.15 |
| RAG6-1029 | CGGTCGGATTACGAGGTCA (SEQ ID NO:370) | CGGUCGGAUUACGAGGUCA[dT][dT] (SEQ ID NO:56) | UGACCUCGUAAUCCGACCG[dT][dT] (SEQ ID NO:213) | 1.15 | 0.20 |
| RAG6-1028 | GGTCGGATTACGAGGTCAG (SEQ ID NO:371) | GGUCGGAUUACGAGGUCAG[dT][dT] (SEQ ID NO:57) | CUGACCUCGUAAUCCGACC[dT][dT] (SEQ ID NO:214) | 1.16 | 0.22 |
| RAG6-1027 | GTCGGATTACGAGGTCAGG (SEQ ID NO:372) | GUCGGAUUACGAGGUCAGG[dT][dT] (SEQ ID NO:58) | CCUGACCUCGUAAUCCGAC[dT][dT] (SEQ ID NO:215) | 1.16 | 0.21 |
| RAG6-1026 | TCGGATTACGAGGTCAGGA (SEQ ID NO:373) | UCGGAUUACGAGGUCAGGA[dT][dT] (SEQ ID NO:59) | UCCUGACCUCGUAAUCCGA[dT][dT] (SEQ ID NO:216) | 1.22 | 0.28 |
| RAG6-1025 | CGGATTACGAGGTCAGGAG (SEQ ID NO:374) | CGGAUUACGAGGUCAGGAG[dT][dT] (SEQ ID NO:60) | CUCCUGACCUCGUAAUCCG[dT][dT] (SEQ ID NO:217) | 1.10 | 0.14 |
| RAG6-1022 | ATTACGAGGTCAGGAGTTC (SEQ ID NO:375) | AUUACGAGGUCAGGAGUUC[dT][dT] (SEQ ID NO:61) | GAACUCCUGACCUCGUAAU [dT][dT] (SEQ ID NO:218) | 1.18 | 0.24 |
| RAG6-1021 | TTACGAGGTCAGGAGTTCA (SEQ ID NO:376) | UUACGAGGUCAGGAGUUCA[dT][dT] (SEQ ID NO:62) | UGAACUCCUGACCUCGUAA[dT][dT] (SEQ ID NO:219) | 1.17 | 0.22 |
| RAG6-1020 | TACGAGGTCAGGAGTTCAA (SEQ ID NO:377) | UACGAGGUCAGGAGUUCAA[dT][dT] (SEQ ID NO:63) | UUGAACUCCUGACCUCGUA[dT][dT] (SEQ ID NO:220) | 1.26 | 0.34 |
| RAG6-1019 | ACGAGGTCAGGAGTTCAAG (SEQ ID NO:378) | ACGAGGUCAGGAGUUCAAG[dT][dT] (SEQ ID NO:64) | CUUGAACUCCUGACCUCGU[dT][dT] (SEQ ID NO:221) | 1.13 | 0.17 |
| RAG6-1016 | AGGTCAGGAGTTCAAGACC (SEQ ID NO:379) | AGGUCAGGAGUUCAAGACC[dT][dT] (SEQ ID NO:65) | GGUCUUGAACUCCUGACCU[dT][dT] (SEQ ID NO:222) | 1.15 | 0.20 |
| RAG6-1008 | AGTTCAAGACCAGCCTGGC (SEQ ID NO:380) | AGUUCAAGACCAGCCUGGC[dT][dT] (SEQ ID NO:66) | GCCAGGCUGGUCUUGAACU[dT][dT] (SEQ ID NO:223) | 1.14 | 0.19 |
| RAG6-980 | GAAACCCCATCTTTACTAA (SEQ ID NO:381) | GAAACCCCAUCUUUACUAA[dT][dT] (SEQ ID NO:67) | UUAGUAAAGAUGGGGUUUC[dT][dT] (SEQ ID NO:224) | 1.10 | 0.14 |
| RAG6-951 | ATTAGCCGGGTGTGGTGGT (SEQ ID NO:382) | AUUAGCCGGGUGUGGUGGU[dT][dT] (SEQ ID NO:68) | ACCACCACACCCGGCUAAU[dT] [dT] (SEQ ID NO:225) | 1.13 | 0.18 |
| RAG6-937 | GTGGTGGGCGCCTGTAATC (SEQ ID NO:383) | GUGGUGGGCGCCUGUAAUC[dT][dT] (SEQ ID NO:69) | GAUUACAGGCGCCCACCAC[dT][dT] (SEQ ID NO:226) | 1.21 | 0.27 |
| RAG6-931 | GGCGCCTGTAATCCCAGCT (SEQ ID NO:384) | GGCGCCUGUAAUCCCAGCU[dT][dT] (SEQ ID NO:70) | AGCUGGGAUUACAGGCGCC[dT][dT] (SEQ ID NO:227) | 1.13 | 0.17 |
| RAG6-923 | TAATCCCAGCTACTCGGGG (SEQ ID NO:385) | UAAUCCCAGCUACUCGGGG[dT][dT] (SEQ ID NO:71) | CCCCGAGUAGCUGGGAUUA[dT][dT] (SEQ ID NO:228) | 1.13 | 0.18 |
| RAG6-905 | GGGCTGAGGCAGAATTGCT (SEQ ID NO:386) | GGGCUGAGGCAGAAUUGCU[dT][dT] (SEQ ID NO:72) | AGCAAUUCUGCCUCAGCCC[dT][dT] (SEQ ID NO:229) | 1.17 | 0.23 |
| RAG6-898 | GGCAGAATTGCTTGAACCT (SEQ ID NO:387) | GGCAGAAUUGCUUGAACCU[dT][dT] (SEQ ID NO:73) | AGGUUCAAGCAAUUCUGCC[dT[[dT] (SEQ ID NO:230) | 1.23 | 0.30 |
| RAG6-896 | CAGAATTGCTTGAACCTGG (SEQ ID NO:388) | CAGAAUUGCUUGAACCUGG[dT][dT] (SEQ ID NO:74) | CCAGGUUCAAGCAAUUCUG[dT][dT] (SEQ ID NO:231) | 1.10 | 0.13 |
| RAG6-886 | TGAACCTGGGAGGCAGAGG (SEQ ID NO:389) | UGAACCUGGGAGGCAGAGG[dT][dT] (SEQ ID NO:75) | CCUCUGCCUCCCAGGUUCA[dT][dT] (SEQ ID NO:232) | 1.24 | 0.31 |
| RAG6-885 | GAACCTGGGAGGCAGAGGT (SEQ ID NO:390) | GAACCUGGGAGGCAGAGGU[dT][dT] (SEQ ID NO:76) | ACCUCUGCCUCCCAGGUUC[dT][dT] (SEQ ID NO:233) | 1.14 | 0.19 |
| RAG6-883 | ACCTGGGAGGCAGAGGTTG (SEQ ID NO:391) | ACCUGGGAGGCAGAGGUUG[dT][dT] (SEQ ID NO:77) | CAACCUCUGCCUCCCAGGU[dT][dT] (SEQ ID NO:234) | 1.12 | 0.17 |
| RAG6-866 | TGCAGTGAGCTGAGATCAC (SEQ ID NO:392) | UGCAGUGAGCUGAGAUCAC[dT][dT] (SEQ ID NO:78) | GUGAUCUCAGCUCACUGCA[dT][dT] (SEQ ID NO:235) | 1.15 | 0.20 |
| RAG6-857 | CTGAGATCACGCCACTGCA (SEQ ID NO:393) | CUGAGAUCACGCCACUGCA[dT][dT] (SEQ ID NO:79) | UGCAGUGGCGUGAUCUCAG[dT][dT] (SEQ ID NO:236) | 1.16 | 0.22 |
| RAG6-852 | ATCACGCCACTGCATTCCA (SEQ ID NO:394) | AUCACGCCACUGCAUUCCA[dT][dT] (SEQ ID NO:80) | UGGAAUGCAGUGGCGUGAU[dT][dT] (SEQ ID NO:237) | 1.59 | 0.67 |
| RAG6-829 | GGGTGACAGAGCAATACTC (SEQ ID NO:395) | GGGUGACAGAGCAAUACUC[dT][dT] (SEQ ID NO:81) | GAGUAUUGCUCUGUCACCC[dT][dT] (SEQ ID NO:238) | 1.22 | 0.28 |
| RAG6-826 | TGACAGAGCAATACTCTGT (SEQ ID NO:396) | UGACAGAGCAAUACUCUGU[dT][dT] (SEQ ID NO:82) | ACAGAGUAUUGCUCUGUCA[dT][dT] (SEQ ID NO:239) | 1.16 | 0.21 |
| RAG6-822 | AGAGCAATACTCTGTCGCA (SEQ ID NO:397) | AGAGCAAUACUCUGUCGCA[dT][dT] (SEQ ID NO:83) | UGCGACAGAGUAUUGCUCU[dT][dT] (SEQ ID NO:240) | 1.10 | 0.14 |
| RAG6-796 | AAAAGAATACTGGAGGCTG (SEQ ID NO:398) | AAAAGAAUACUGGAGGCUG[dT][dT] (SEQ ID NO:84) | CAGCCUCCAGUAUUCUUUU[dT][dT] (SEQ ID NO:241) | 1.17 | 0.23 |
| RAG6-795 | AAAGAATACTGGAGGCTGG (SEQ ID NO:399) | AAAGAAUACUGGAGGCUGG[dT][dT] (SEQ ID NO:85) | CCAGCCUCCAGUAUUCUUU[dT][dT] (SEQ ID NO:242) | 1.11 | 0.15 |
| RAG6-790 | ATACTGGAGGCTGGGCGAG (SEQID NO:400) | AUACUGGAGGCUGGGCGAG[dT][dT] (SEQ ID NO:86) | CUCGCCCAGCCUCCAGUAU[dT][dT] (SEQ ID NO:243) | 1.50 | 0.58 |
| RAG6-775 | CGAGGTGGCTCACACCTGT (SEQID NO:401) | CGAGGUGGCUCACACCUGU[dT][dT] (SEQ ID NO:87) | ACAGGUGUGAGCCACCUCG[dT][dT] (SEQ ID NO:244) | 1.10 | 0.14 |
| RAG6-772 | GGTGGCTCACACCTGTAAT (SEQID NO:402) | GGUGGCUCACACCUGUAAU[dT][dT] (SEQ ID NO:88) | AUUACAGGUGUGAGCCACC[dT][dT] (SEQ ID NO:245) | 1.16 | 0.21 |
| RAG6-769 | GGCTCACACCTGTAATCCC (SEQ ID NO:403) | GGCUCACACCUGUAAUCCC[dT][dT] (SEQ ID NO:89) | GGGAUUACAGGUGUGAGCC[dT][dT] (SEQ ID NO:246) | 1.19 | 0.25 |
| RAG6-768 | GCTCACACCTGTAATCCCA (SEQID NO:404) | GCUCACACCUGUAAUCCCA[dT][dT] (SEQ ID NO:90) | UGGGAUUACAGGUGUGAGC[dT][dT] (SEQ ID NO:247) | 1.12 | 0.16 |
| RAG6-765 | CACACCTGTAATCCCAGCA (SEQID NO:405) | CACACCUGUAAUCCCAGCA[dT][dT] (SEQ ID NO:91) | UGCUGGGAUUACAGGUGUG[dT][dT] (SEQ ID NO:248) | 1.11 | 0.14 |
| RAG6-757 | TAATCCCAGCATTTTGGGA (SEQ ID NO:406) | UAAUCCCAGCAUUUUGGGA[dT][dT] (SEQ ID NO:92) | UCCCAAAAUGCUGGGAUUA[dT][dT] (SEQ ID NO:249) | 1.11 | 0.16 |
| RAG6-728 | GGGCGGAATATCTTGAGCT (SEQ ID NO:407) | GGGCGGAAUAUCUUGAGCU[dT][dT] (SEQ ID NO:93) | AGCUCAAGAUAUUCCGCCC[dT][dT] (SEQ ID NO:250) | 1.11 | 0.15 |
| RAG6-722 | AATATCTTGAGCTCAGGAG (SEQID NO:408) | AAUAUCUUGAGCUCAGGAG[dT][dT] (SEQ ID NO:94) | CUCCUGAGCUCAAGAUAUU[dT][dT] (SEQ ID NO:251) | 1.41 | 0.49 |
| RAG6-703 | TTCGAGACCAGCCTACACA (SEQID NO:409) | UUCGAGACCAGCCUACACA[dT][dT] (SEQ ID NO:95) | UGUGUAGGCUGGUCUCGAA[dT][dT] (SEQ ID NO:252) | 1.36 | 0.45 |
| RAG6-696 | CCAGCCTACACAATATGCT (SEQID NO:410) | CCAGCCUACACAAUAUGCU [dT][dT] (SEQ ID NO:96) | AGCAUAUUGUGUAGGCUGG[dT][dT] (SEQ ID NO:253) | 1.14 | 0.19 |
| RAG6-689 | ACACAATATGCTCCAAACG (SEQ ID NO:411) | ACACAAUAUGCUCCAAACG[dT][dT] (SEQ ID NO:97) | CGUUUGGAGCAUAUUGUGU[dT][dT] (SEQ ID NO:254) | 1.12 | 0.16 |
| RAG6-688 | CACAATATGCTCCAAACGC (SEQID NO:412) | CACAAUAUGCUCCAAACGC[dT] [dT] (SEQ ID NO:98) | GCGUUUGGAGCAUAUUGUG[dT][dT] (SEQ ID NO:255) | 1.21 | 0.28 |
| RAG6-687 | ACAATATGCTCCAAACGCC (SEQ ID NO:413) | ACAAUAUGCUCCAAACGCC[dT]dT] (SEQ ID NO:99) | GGCGUUUGGAGCAUAUUGU[dT] [dT] (SEQ ID NO:256) | 1.10 | 0.14 |
| RAG6-676 | CAAACGCCGCCTCTACAAA (SEQID NO:414) | CAAACGCCGCCUCUACAAA[dT][dT] (SEQ ID NO:100) | UUUGUAGAGGCGGCGUUUG[dT][dT] (SEQ ID NO:257) | 1.10 | 0.14 |
| RAG6-622 | CTGTGGTCCTAGCTACTTG (SEQID NO:415) | CUGUGGUCCUAGCUACUUG[dT][dT] (SEQ ID NO:101) | CAAGUAGCUAGGACCACAG[dT][dT] (SEQ ID NO:258) | 1.21 | 0.27 |
| RAG6-591 | GGGAGGATCGCTTGAGCTC (SEQID NO:416) | GGGAGGAUCGCUUGAGCUC[dT][dT] (SEQ ID NO:102) | GAGCUCAAGCGAUCCUCCC[dT][dT] (SEQ ID NO:259) | 1.14 | 0.18 |
| RAG6-589 | GAGGATCGCTTGAGCTCGG (SEQ ID NO:417) | GAGGAUCGCUUGAGCUCGG[dT][dT] (SEQ ID NO:103) | CCGAGCUCAAGCGAUCCUC[dT][dT] (SEQ ID NO:260) | 1.13 | 0.17 |
| RAG6-571 | GGAGGTCGAGGCTGCAATG (SEQID NO:418) | GGAGGUCGAGGCUGCAAUG[dT][dT] (SEQ ID NO:104) | CAUUGCAGCCUCGACCUCC[dT][dT] (SEQ ID NO:261) | 1.10 | 0.14 |
| RAG6-568 | GGTCGAGGCTGCAATGAGC (SEQID NO:419) | GGUCGAGGCUGCAAUGAGC[dT][dT] (SEQ ID NO:105) | GCUCAUUGCAGCCUCGACC[dT][dT] (SEQ ID NO:262) | 1.15 | 0.21 |
| RAG6-557 | CAATGAGCCGAGATGGTGC (SEQ ID NO:420) | CAAUGAGCCGAGAUGGUGC[dT][dT] (SEQ ID NO:106) | GCACCAUCUCGGCUCAUUG[dT][dT] (SEQ ID NO:263) | 1.11 | 0.16 |
| RAG6-556 | AATGAGCCGAGATGGTGCC (SEQID NO:421) | AAUGAGCCGAGAUGGUGCC[dT][dT] (SEQ ID NO:107) | GGCACCAUCUCGGCUCAUU [dT][dT] (SEQ ID NO:264) | 1.37 | 0.45 |
| RAG6-550 | CCGAGATGGTGCCACTGCA (SEQID NO:422) | CCGAGAUGGUGCCACUGCA[dT][dT] (SEQ ID NO:108) | UGCAGUGGCACCAUCUCGG[dT][dT] (SEQ ID NO:265) | 1.23 | 0.30 |
| RAG6-548 | GAGATGGTGCCACTGCACT (SEQ ID NO:423) | GAGAUGGUGCCACUGCACU[dT][dT] (SEQ ID NO:109) | AGUGCAGUGGCACCAUCUC[dT][dT] (SEQ ID NO:266) | 1.15 | 0.20 |
| RAG6-547 | AGATGGTGCCACTGCACTC (SEQID NO:424) | AGAUGGUGCCACUGCACUC[dT][dT] (SEQ ID NO:110) | GAGUGCAGUGGCACCAUCU[dT][dT] (SEQ ID NO:267) | 1.24 | 0.31 |
| RAG6-545 | ATGGTGCCACTGCACTCTG (SEQID NO:425) | AUGGUGCCACUGCACUCUG[dT][dT] (SEQ ID NO:111) | CAGAGUGCAGUGGCACCAU[dT][dT] (SEQ ID NO:268) | 1.37 | 0.46 |
| RAG6-539 | CCACTGCACTCTGACGACA (SEQ ID NO:426) | CCACUGCACUCUGACGACA[dT][dT] (SEQ ID NO:112) | UGUCGUCAGAGUGCAGUGG[dT][dT] (SEQ ID NO:269) | 1.41 | 0.50 |
| RAG6-538 | CACTGCACTCTGACGACAG (SEQ ID NO:427) | CACUGCACUCUGACGACAG[dT][dT] (SEQ ID NO:113) | CUGUCGUCAGAGUGCAGUG[dT][dT] (SEQ ID NO:270) | 1.37 | 0.46 |
| RAG6-530 | TCTGACGACAGAGCGAGAC (SEQID NO:428) | UCUGACGACAGAGCGAGAC[dT][dT] (SEQ ID NO:114) | GUCUCGCUCUGUCGUCAGA[dT][dT] (SEQ ID NO:271) | 1.15 | 0.20 |
| RAG6-529 | CTGACGACAGAGCGAGACT (SEQID NO:429) | CUGACGACAGAGCGAGACU[dT][dT] (SEQ ID NO:115) | AGUCUCGCUCUGUCGUCAG[dT][dT] (SEQ ID NO:272) | 1.13 | 0.17 |
| RAG6-516 | GAGACTCCGTCTCAAAACA (SEQ ID NO:430) | GAGACUCCGUCUCAAAACA[dT][dT] (SEQ ID NO:116) | UGUUUUGAGACGGAGUCUC[dT][dT] (SEQ ID NO:273) | 1.23 | 0.30 |
| RAG6-515 | AGACTCCGTCTCAAAACAA (SEQ ID NO:431) | AGACUCCGUCUCAAAACAA[dT][dT] (SEQ ID NO:117) | UUGUUUUGAGACGGAGUCU[dT][dT] (SEQ ID NO:274) | 1.28 | 0.35 |
| RAG6-465 | TCTAGTGTTTAAGGATCTG (SEQ ID NO:432) | UCUAGUGUUUAAGGAUCUG[dT][dT] (SEQ ID NO:118) | CAGAUCCUUAAACACUAGA[dT][dT] (SEQ ID NO:275) | 1.20 | 0.26 |
| RAG6-463 | TAGTGTTTAAGGATCTGCC (SEQ ID NO:433) | UAGUGUUUAAGGAUCUGCC[dT][dT] (SEQ ID NO:119) | GGCAGAUCCUUAAACACUA[dT][dT] (SEQ ID NO:276) | 1.11 | 0.14 |
| RAG6-460 | TGTTTAAGGATCTGCCTTC (SEQ ID NO:434) | UGUUUAAGGAUCUGCCUUC[dT][dT] (SEQ ID NO:120) | GAAGGCAGAUCCUUAAACA[dT][dT] (SEQ ID NO:277) | 1.13 | 0.17 |
| RAG6-453 | GGATCTGCCTTCCTTCCTG (SEQ ID NO:435) | GGAUCUGCCUUCCUUCCUG[dT][dT] (SEQ ID NO:121) | CAGGAAGGAAGGCAGAUCC[dT][dT] (SEQ ID NO:278) | 1.39 | 0.48 |
| RAG6-425 | TTGTCTTTCCTTGTTTGTC (SEQ ID NO:436) | UUGUCUUUCCUUGUUUGUC[dT][dT] (SEQ ID NO:122) | GACAAACAAGGAAAGACAA[dT][dT] (SEQ ID NO:279) | 1.13 | 0.17 |
| RAG6-423 | GTCTTTCCTTGTTTGTCTT (SEQ ID NO:437) | GUCUUUCCUUGUUUGUCUU[dT][dT] (SEQ ID NO:123) | AAGACAAACAAGGAAAGAC[dT][dT] (SEQ ID NO:280) | 1.11 | 0.15 |
| RAG6-395 | CAAGCAGGTTTTAAATTCC (SEQ ID NO:438) | CAAGCAGGUUUUAAAUUCC[dT][dT] (SEQ ID NO:124) | GGAAUUUAAAACCUGCUUG[dT][dT] (SEQ ID NO:281) | 1.10 | 0.14 |
| RAG6-392 | GCAGGTTTTAAATTCCTAG (SEQ ID NO:439) | GCAGGUUUUAAAUUCCUAG[dT][dT] (SEQ ID NO:125) | CUAGGAAUUUAAAACCUGC[dT][dT] (SEQ ID NO:282) | 1.31 | 0.39 |
| RAG6-364 | ACATTTACTTTTCCAAGGG (SEQID NO:440) | ACAUUUACUUUUCCAAGGG[dT][dT] (SEQ ID NO:126) | CCCUUGGAAAAGUAAAUGU[dT][dT] (SEQ ID NO:283) | 1.27 | 0.35 |
| RAG6-294 | ACACTGGAGTTCGAGACGA (SEQID NO:441) | ACACUGGAGUUCGAGACGA[dT][dT] (SEQ ID NO:127) | UCGUCUCGAACUCCAGUGU[dT][dT] (SEQ ID NO:284) | 1.13 | 0.17 |
| RAG6-291 | CTGGAGTTCGAGACGAGGC (SEQID NO:442) | CUGGAGUUCGAGACGAGGC[dT][dT] (SEQ ID NO:128) | GCCUCGUCUCGAACUCCAG[dT][dT] (SEQ ID NO:285) | 1.19 | 0.26 |
| RAG6-285 | TTCGAGACGAGGCCTAAGC (SEQ ID NO:443) | UUCGAGACGAGGCCUAAGC[dT][dT] (SEQ ID NO:129) | GCUUAGGCCUCGUCUCGAA[dT][dT] (SEQ ID NO:286) | 1.30 | 0.38 |
| RAG6-282 | GAGACGAGGCCTAAGCAAC (SEQ ID NO:444) | GAGACGAGGCCUAAGCAAC[dT][dT] (SEQ ID NO:130) | GUUGCUUAGGCCUCGUCUC[dT][dT] (SEQ ID NO:287) | 1.14 | 0.19 |
| RAG6-281 | AGACGAGGCCTAAGCAACA (SEQ ID NO:445) | AGACGAGGCCUAAGCAACA[dT][dT] (SEQ ID NO:131) | UGUUGCUUAGGCCUCGUCU[dT][dT] (SEQ ID NO:288) | 1.82 | 0.86 |
| RAG6-280 | GACGAGGCCTAAGCAACAT (SEQID NO:446) | GACGAGGCCUAAGCAACAU[dT][dT] (SEQ ID NO:132) | AUGUUGCUUAGGCCUCGUC[dT][dT] (SEQ ID NO:289) | 1.11 | 0.16 |
| RAG6-273 | CCTAAGCAACATGCCGAAA (SEQ ID NO:447) | CCUAAGCAACAUGCCGAAA[dT][dT] (SEQ ID NO:133) | UUUCGGCAUGUUGCUUAGG[dT][dT] (SEQ ID NO:290) | 1.17 | 0.22 |
| RAG6-272 | CTAAGCAACATGCCGAAAC (SEQID NO:448) | CUAAGCAACAUGCCGAAAC[dT][dT] (SEQ ID NO:134) | GUUUCGGCAUGUUGCUUAG[dT][dT] (SEQ ID NO:291) | 1.30 | 0.38 |
| RAG6-271 | TAAGCAACATGCCGAAACC (SEQID NO:449) | UAAGCAACAUGCCGAAACC[dT][dT] (SEQ ID NO:135) | GGUUUCGGCAUGUUGCUUA[dT][dT] (SEQ ID NO:292) | 1.19 | 0.26 |
| RAG6-219 | TGGTGGCGCACGCCTATAG (SEQID NO:450) | UGGUGGCGCACGCCUAUAG[dT][dT] (SEQ ID NO:136) | CUAUAGGCGUGCGCCACCA[dT][dT] (SEQ ID NO:293) | 1.33 | 0.42 |
| RAG6-218 | GGTGGCGCACGCCTATAGT (SEQID NO:451) | GGUGGCGCACGCCUAUAGU [dT][dT] (SEQ ID NO:137) | ACUAUAGGCGUGCGCCACC[dT][dT] (SEQ ID NO:294) | 1.19 | 0.26 |
| RAG6-206 | CTATAGTCCTAGCTACTGG (SEQ ID NO:452) | CUAUAGUCCUAGCUACUGG[dT][dT] (SEQ ID NO:138) | CCAGUAGCUAGGACUAUAG[dT][dT] (SEQ ID NO:295) | 1.22 | 0.29 |
| RAG6-205 | TATAGTCCTAGCTACTGGG (SEQ ID NO:453) | UAUAGUCCUAGCUACUGGG[dT][dT] (SEQ ID NO:139) | CCCAGUAGCUAGGACUAUA[dT][dT] (SEQ ID NO:296) | 1.10 | 0.13 |
| RAG6-181 | TGAGGTGGGAGGATCGCTT (SEQID NO:454) | UGAGGUGGGAGGAUCGCUU[dT][dT] (SEQ ID NO:140) | AAGCGAUCCUCCCACCUCA[dT][dT] (SEQ ID NO:297) | 1.19 | 0.24 |
| RAG6-144 | CTGCAGTGAGCCGAGATCG (SEQ ID NO:455) | CUGCAGUGAGCCGAGAUCG[dT][dT] (SEQ ID NO:141) | CGAUCUCGGCUCACUGCAG[dT][dT] (SEQ ID NO:298) | 1.23 | 0.30 |
| RAG6-143 | TGCAGTGAGCCGAGATCGC (SEQ ID NO:456) | UGCAGUGAGCCGAGAUCGC[dT] [dT] (SEQ ID NO:142) | GCGAUCUCGGCUCACUGCA[dT] [dT] (SEQ ID NO:299) | 1.53 | 0.62 |
| RAG6-119 | TGCACTCCAGCCTGAGCGA (SEQ ID NO:457) | UGCACUCCAGCCUGAGCGA[dT][dT] (SEQ ID NO:143) | UCGCUCAGGCUGGAGUGCA[dT][dT] (SEQ ID NO:300) | 1.24 | 0.31 |
| RAG6-117 | CACTCCAGCCTGAGCGACA (SEQ ID NO:458) | CACU CCAGCCUGAGCGACA[dT] [dT] (SEQ ID NO:144) | UGUCGCUCAGGCUGGAGUG[dT][dT] (SEQ ID NO:301) | 1.26 | 0.34 |
| RAG6-101 | ACAGGGCGAGGCTCTGTCT (SEQ ID NO:459) | ACAGGGCGAGGCUCUGUCU[dT][dT] (SEQ ID NO:145) | AGACAGAGCCUCGCCCUGU[dT][dT] (SEQ ID NO:302) | 1.29 | 0.37 |
| RAG6-98 | GGGCGAGGCTCTGTCTCAA (SEQ ID NO:460) | GGGCGAGGCUCUGUCUCAA[dT] [dT] (SEQ ID NO:146) | UUGAGACAGAGCCUCGCCC[dT][dT] (SEQ ID NO:303) | 1.56 | 0.64 |
| RAG6-97 | GGCGAGGCTCTGTCTCAAA (SEQ ID NO:461) | GGCGAGGCUCUGUCUCAAA[dT][dT] (SEQ ID NO:147) | UUUGAGACAGAGCCUCGCC[dT][dT] (SEQ ID NO:304) | 1.18 | 0.24 |
| RAG6-96 | GCGAGGCTCTGTCTCAAAA (SEQ ID NO:462) | GCGAGGCUCUGUCUCAAAA[dT][dT] (SEQ ID NO:148) | UUUUGAGACAGAGCCUCGC[dT][dT] (SEQ ID NO:305) | 1.26 | 0.34 |
| RAG6-93 | AGGCTCTGTCTCAAAACAA (SEQ ID NO:463) | AGGCUCUGUCUCAAAACAA[dT][dT] (SEQ ID NO:149) | UUGUUUUGAGACAGAGCCU[dT][dT] (SEQ ID NO:306) | 1.14 | 0.20 |
| RAG6-92 | GGCTCTGTCTCAAAACAAA (SEQ ID NO:464) | GGCUCUGUCUCAAAACAAA[dT][dT] (SEQ ID NO:150) | UUUGUUUUGAGACAGAGCC[dT][dT] (SEQ ID NO:307) | 1.16 | 0.22 |
| RAG6-91 | GCTCTGTCTCAAAACAAAC (SEQ ID NO:465) | GCUCUGUCUCAAAACAAAC[dT][dT] (SEQ ID NO:151) | GUUUGUUUUGAGACAGAGC[dT][dT] (SEQ ID NO:308) | 1.15 | 0.20 |
| RAG6-90 | CTCTGTCTCAAAACAAACA (SEQ ID NO:466) | CUCUGUCUCAAAACAAACA[dT][dT] (SEQ ID NO:152) | UGUUUGUUUUGAGACAGAG[dT][dT] (SEQ ID NO:309) | 1.18 | 0.24 |
| RAG6-45 | AACACAGTGAAATGAAAGG (SEQ ID NO:467) | AACACAGUGAAAUGAAAGG[DT][dT] (SEQ ID NO:153) | CCUUUCAUUUCACUGUGUU[dT] [dT] (SEQ ID NO:310) | 1.22 | 0.29 |
| RAG6-44 | ACACAGTGAAATGAAAGGA (SEQ ID NO:468) | ACACAGUGAAAUGAAAGGA[dT][dT] (SEQ ID NO:154) | UCCUUUCAUUUCACUGUGU[dT][dT] (SEQ ID NO:311) | 1.16 | 0.22 |
| RAG6-41 | CAGTGAAATGAAAGGATTG (SEQ ID NO:469) | CAGUGAAAUGAAAGGAUUG[dT][dT] (SEQ ID NO:155) | CAAUCCUUUCAUUUCACUG[DT][DT] (SEQ ID NO:312) | 1.23 | 0.30 |
| RAG6-39 | GTGAAATGAAAGGATTGAG (SEQ ID N0:470) | GUGAAAUGAAAGGAUUGAG[dT] [dT] (SEQ ID NO:156) | CUCAAUCCUUUCAUUUCAC[dT][dT] (SEQ ID NO:313) | 1.15 | 0.20 |
| RAG6-37 | GAAATGAAAGGATTGAGAG (SEQ ID NO:471) | GAAAUGAAAGGAUUGAGAG[DT] [dT] (SEQ ID NO:157) | CUCUCAAUCCUUUCAUUUC[dT][dT] (SEQ ID NO:314) | 1.21 | 0.27 |

**Table 4. Primer sequences for RT-qPCR analysis**

| **Primer** | **Sequence No.** | **Sequence (5'-3')** |
|---|---|---|
| SMN F1 | SEQ ID NO: 480 | CACAGGCCAGAGCGATGA |
| SMN R1 | SEQ ID NO: 481 | CGAAGTTTCACAAATGTCACCAT |
| HPRT1 F | SEQ ID NO: 482 | ATGGACAGGACTGAACGTCTT |
| HPRT1 R | SEQ ID NO: 483 | TCCAGCAGGTCAGCAAAGAA |
| TBP F | SEQ ID NO: 484 | ATAATCCCAAGCGGTTTGCT |
| TBP R | SEQ ID NO: 485 | CTGCCAGTCTGGACTGTTCT |
| SMN F2 | SEQ ID NO: 486 | CCACCACCTCCCATATGTCC |
| SMN R2 | SEQ ID NO: 487 | GCTCTATGCCAGCATTTCTCCT |
| SMN-exon6-F | SEQ ID NO: 488 | CCCCCACCACCTCCCATATG |
| SMN-exon8-R | SEQ ID NO: 489 | CCCTTCTCACAGCTCATAAAATTAC |

**FIG. 2** further shows the activity distribution of the SMN2 saRNAs sorted from highly activation to highly inhibition. When the 980 saRNAs were sorted by their location on SMN2 promoter, it can be clearly seen that functional saRNAs were distributed across the promoter in a clustered fashion, i.e., at certain promoter regions, there were "hotspots" where functional saRNAs were enriched **(****FIG. 3****).** As shown in **FIG. 3****,** there are 4 hotspots occurring in regions -1639 to - 1481 (H1), -1090 to -1008 (H2), -994 to -180 (H3), and -144 to -37 (H4) of the promoter and having highly enriched functional saRNAs. This result indicates that functional saRNAs were not randomly distributed on the promoter but were enriched in the specific hotspot regions.
**Hotspot H1 (-1639 to -1481) sequence** (SEQ ID NO: 476): 
agtcgcactctgtcactcaggctggagtgcagtggcgtgatcttggctcactgcaacctccgcctcccgagttcaagtgattctcctggctcagcc

tcccaagcagctgtcattacaggcctgcaccaccacacccggctgatttttgtatttttagga (SEQ ID NO: 476)
**Hotspot H2 (-1090 to -1008) sequence** (SEQ ID NO: 477):
   aatactggaggcccggtgtggtggctcacacctgtaatcccagcactttgggaggccgaggcggtcggattacgaggtcagg (SEQ ID
NO: 477) **Hotspot H3 (-994 to -180) sequence** (SEQ ID NO: 478): 
ctggccaacatggtgaaaccccatctttactaaaaatacaaaaattagccgggtgtggtggtgggcgcctgtaatcccagctactcgggggct
gaggcagaattgcttgaacctgggaggcagaggttgcagtgagctgagatcacgccactgcattccagcctgggtgacagagcaatactctgt
cgcaaaaaaaaaaagaatactggaggctgggcgaggtggctcacacctgtaatcccagcattttgggatgccagaggcgggcggaatatctt
gagctcaggagttcgagaccagcctacacaatatgctccaaacgccgcctctacaaaacatacagaaactagccgggtgtggtggcgtgcccc
 
tgtggtcctagctacttgggaggttgaggcgggaggatcgcttgagctcgggaggtcgaggctgcaatgagccgagatggtgccactgcactc
tgacgacagagcgagactccgtctcaaaacaaacaacaaataaggttgggggatcaaatatcttctagtgtttaaggatctgccttccttcctgcc
cccatgtttgtctttccttgtttgtctttatatagatcaagcaggttttaaattcctagtaggagcttacatttacttttccaagggggaggggaataaat
atctacacacacacacacacacacacacacacacacacactggagttcgagacgaggcctaagcaacatgccgaaaccccgtctctactaaat
acaaaaaatagctgagcttggtggcgcacgcctatagtcctagctactggggaggctg (SEQ ID NO: 478)
**Hotspot H4 (-144 to -37) sequence** (SEQ ID NO: 479):

ctgcagtgagccgagatcgcgccgctgcactccagcctgagcgacagggcgaggctctgtctcaaaacaaacaaacaaaaaaaaaaggaaa
ggaaatataacacagtg(SEQ ID NO: 479).

### Example 3: Further Screening and Validation of Functional saRNAs Capable of Activating SMN Gene

In order to further screen and verify the functional saRNAs capable of activating SMN gene, based on the high-throughput screening result, 50 saRNAs were randomly selected from 157 functional saRNAs to further verify their activating effect on the expression of SMN genes in HEK293T, HS27 (human skin fibroblast cell line) and NHDF (normal human dermal fibroblast cells) cells. HEK293T cells, HS27 cells and NHDF cells were transfected with individual saRNAs (n=50, final concentration: 20 nM) shown in Table 5 with the transfection method described in Example 2. After 72 hours, RNAs were extracted from the transfected cells using a Qiagen RNeasy kit. After reverse transcription, qPCR amplification of SMN was performed using a 7500FAST real-time PCR system, and HPRT1 and TBP genes were amplified with their geometric means of expression as internal references. **FIG. 4** shows the activating effect of the saRNAs on the expression of SMN gene in HEK293T cells, and Table 5 shows the activating effect of the saRNAs on the expression of SMN gene in HS27 and NHDF cells. It can be seen from these results that the these saRNAs can activate the expression of SMN genes in the different cells to different degrees, up to 19-fold.

**Table 5. Randomly selected 50 saRNAs for Validation**

| **saRNA** | **293T** | **HS27** | **NHDF** | **Mean** |
|---|---|---|---|---|
| RAG6-1061 | 2.10 | 1.51 | 19.07 | 7.56 |
| RAG6-219 | 1.78 | 3.45 | 4.19 | 3.14 |
| RAG6-790 | 2.40 | 2.67 | 2.79 | 2.62 |
| RAG6-1392 | 1.79 | 3.19 | 2.80 | 2.59 |
| RAG6-392 | 1.77 | 2.82 | 2.81 | 2.47 |
| RAG6-550 | 1.60 | 2.55 | 2.50 | 2.22 |
| RAG6-556 | 2.08 | 2.30 | 2.06 | 2.15 |
| RAG6-1612 | 1.28 | 2.70 | 2.45 | 2.14 |
| RAG6-1089 | 1.64 | 2.28 | 2.39 | 2.10 |
| RAG6-281 | 2.21 | 1.99 | 1.86 | 2.02 |
| RAG6-143 | 1.46 | 2.42 | 2.11 | 2.00 |
| RAG6-545 | 1.84 | 1.85 | 1.98 | 1.89 |
| RAG6-852 | 1.69 | 1.65 | 2.08 | 1.81 |
| RAG6-1535 | 1.56 | 1.80 | 1.98 | 1.78 |
| RAG6-1020 | 1.56 | 1.71 | 2.05 | 1.77 |
| RAG6-1533 | 1.63 | 1.74 | 1.88 | 1.75 |
| RAG6-285 | 2.10 | 1.54 | 1.54 | 1.73 |
| RAG6-722 | 1.54 | 1.59 | 2.05 | 1.73 |
| RAG6-272 | 1.51 | 1.77 | 1.88 | 1.72 |
| RAG6-98 | 1.69 | 1.51 | 1.89 | 1.70 |
| RAG6-117 | 1.17 | 2.12 | 1.73 | 1.68 |
| RAG6-539 | 1.37 | 1.84 | 1.75 | 1.65 |
| RAG6-538 | 1.27 | 1.72 | 1.93 | 1.64 |
| RAG6-703 | 1.44 | 1.46 | 1.70 | 1.53 |
| RAG6-364 | 1.39 | 1.57 | 1.58 | 1.51 |
| RAG6-144 | 1.07 | 1.76 | 1.60 | 1.48 |
| RAG6-1575 | 1.35 | 1.70 | 1.26 | 1.44 |
| RAG6-1598 | 1.02 | 1.93 | 1.31 | 1.42 |
| RAG6-1576 | 1.15 | 1.25 | 1.86 | 1.42 |
| RAG6-1563 | 1.38 | 1.17 | 1.67 | 1.40 |
| RAG6-1597 | 1.22 | 1.35 | 1.39 | 1.32 |
| RAG6-1603 | 1.24 | 0.87 | 1.80 | 1.30 |
| RAG6-886 | 1.13 | 1.22 | 1.42 | 1.26 |
| RAG6-898 | 1.07 | 1.18 | 1.50 | 1.25 |
| RAG6-1241 | 1.53 | 1.09 | 1.12 | 1.25 |
| RAG6-1578 | 0.85 | 1.35 | 1.49 | 1.23 |
| RAG6-515 | 1.19 | 1.12 | 1.36 | 1.22 |
| RAG6-547 | 1.04 | 1.38 | 1.24 | 1.22 |
| RAG6-1567 | 0.96 | 1.32 | 1.31 | 1.20 |
| RAG6-1548 | 0.80 | 1.35 | 1.21 | 1.12 |
| RAG6-1631 | 0.93 | 1.26 | 1.16 | 1.12 |
| RAG6-516 | 0.82 | 1.24 | 1.16 | 1.08 |
| RAG6-1564 | 0.94 | 1.31 | 0.96 | 1.07 |
| RAG6-101 | 0.90 | 1.24 | 1.08 | 1.07 |
| RAG6-1577 | 0.91 | 1.00 | 1.28 | 1.06 |
| RAG6-453 | 1.10 | 1.03 | 0.79 | 0.97 |
| RAG6-119 | 0.97 | 0.99 | 0.95 | 0.97 |
| RAG6-1545 | 0.82 | 1.12 | 0.97 | 0.97 |
| RAG6-96 | 1.15 | 0.70 | 0.72 | 0.86 |
| RAG6-1565 | 0.69 | 1.35 | 0.24 | 0.76 |

### Example 4: Assessment of Expression of SMN2 by RT-PCR and Restriction Enzyme Digestion

As SMN2 gene is highly homolgous to SMN1 gene, the RT-qPCR primers described above are insufficient to distinguish the mRNA sequences of SMN2 and SMN1. In order to specifically detect the expression of SMN2 mRNAs after saRNA treatment and also differentiate SMN2 mRNA with exon 7 included and skipped, cDNA from the saRNA-treated cells was amplified with a primer pair SMN-exon6-F and SMN-exon8-R, and the PCR products were then digested with *Dde*I restriction enzyme. After gel electrophoresis of the digestion products, expression levels of full-length mRNA and exon 7-deleted (SMN2Δ7) mRNA of SMN2 gene were assessed by the density of specific DNA bands. Briefly, HEK293T cells were inoculated into 6-well plates at 2-3 × 10⁵ cells/well and reversely transfected with the saRNA at a final concentration of 10 nM. At the end of the transfection, total cellular RNAs were extracted using an RNeasy Plus Mini kit (Qiagen; Hilden, Germany) as per the kit's instructions. RNA (1 µg) was reversely transcribed into cDNA using a Prime Script RT kit containing gDNA Eraser (Takara, Shiga, Japan) and PCR amplified with SMN-exon6-F and SMN-exon8-R primers and Takara (RR010A) PCR reagent under conditions of 10 s at 98 °C, 15 s at 60 °C, 32 s at 72 °C and 28 cycles of amplification (see Table 6 for details). After PCR amplification, the PCR products were digested with *Dde*I to distinguish SMN1 and SMN2, and the digested products were then separated by 2.5% agarose gel electrophoresis. The band intensity of each PCR product or restriction enzyme digestion bands were analyzed using Image Lab (BIO-RAD, Chemistry Doc^{tm} MP imaging system). A 500 bp band of a Takara 100 bp DNA ladder (3407A) (approximately 150 ng DNA contained in a 5-µL sample loaded in a gel well) was used as a reference to normalize the band intensity of test samples. The normalized band intensity was then expressed as relative values to that of Mock treatment. The restriction enzyme digestion reaction and and conditions are shown in Table 7. HPRT1 was used as an internal reference gene, and primer sequences used are listed in Table 4.

The SMN2 overexpression vector used in this example was constructed and transfected as follows:

Total cellular RNAs was extracted from HEK293T cells and reverse transcribed into cDNA with OligodT primers. SMN2 full-length open reading frame (ORF) was amplified with PCR cloning primers cSMN2-F2 (TAAGCA GGATCC ATG GCG ATG AGC AGC GGC GGC (SEQ ID NO: 490)) and cSMN2-R2 (TAAGCA GAATTC TTA ATT TAA GGA ATG TGA GCA (SEQ ID NO: 491)). The resulted products were digested with BamHI and EcoRI enzymes. pcDNA3.1 plasmids (Invitrogen) were digested with the same enzymes. The digested plasmids and the digested PCR products were ligated with T4 ligase. Competent cells DH5α were transformed with the ligation reaction products. After the cells were grown overnight, plasmids were extracted using a Qiagen Miniprep kit. The resulting plasmids (1 µg) were transfected into HEK293T cells using Lipofectamine 3000 (Invitrogen). 72 hours later, total RNAs were extracted from the transfected cells and analyzed by RT-PCR and a restriction enzyme digestion.

**Table 6. RT-PCR reaction and conditions**

| Reagent (Takara, R010A) | Volume (µL) | Final concentration |
|---|---|---|
| 5 × primeSTAR buffer | 5 | 1× |
| dNTP mixture | 2 | 200 µM each |
| Primers F + R (5 uM) | 1 | 0.2-0.3 µM each |
| Template | 2 | |
| PrimeSTAR HS DNA polymerase | 0.25 | |
| Double distilled water | 14.75 | |
| PCR conditions | | |
| 98°C | 10 s | 28 cycles |
| 60°C | 15 s | |
| 72°C | 32 s | |

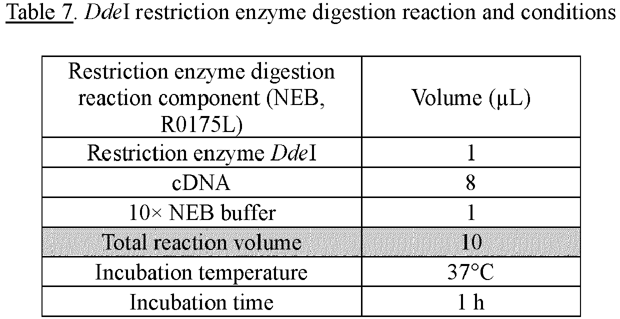

As shown in **FIG. 6** and **FIG. 7A****,** compared with blank control (Mock) and oligonucleotide duplex (dsCON2) control treatment (bands 51-52), the total expression level of SMN2 mRNAs was increased in all saRNA-treated cells, with the highest induction reaching 2.49-fold. Moreover, most of the saRNAs (28, 56%) led to an increase in the ratio of level full-length mRNA to that of exon 7-deleted mRNA of SMN2 gene (indicated by black arrows in **FIG. 6,** and **FIG. 7B****).**

### Example 5: Study on Dose-effect Relationship of saRNAs in Activating SMN Expression and Increasing Expression of Full-length SMN2 mRNA and Protein

In order to determine the dose-effect relationship between saRNA treatment and SMN activation, 2 saRNAs (RAG6-281 and RAG6-550) were selected to transfect HEK293T cells at concentrations from 1 nM, 10 nM, 20 nM, 50 nM to 100 nM. Seventy-two hours later, RNA and protein were extracted from the treated cells. The RNA sample was reversely transcribed into cDNA, and the resulted cDNA was amplified by RT-qPCR and by RT-PCR followed by *Dde*I restriction enzyme digestion. Protein samples were analyzed by western blotting with an SMN-specific antibody to determine the expression level of SMN protein. Briefly, treated cells were lysed with cell lysis buffer (1× RIPA buffer, Cell Signaling Technology (CST), Danvers, MA, USA, #9806). Protease inhibitor (Sigma, Lot#126M4015v) was added to the lysis buffer. The protein sample was quantified by the BCA method, and separated by polyacrylamide gel electrophoresis. After electrophoresis, the protein samples were transferred to a 0.45 µm PVDF membrane. The blots were assayed with a mouse monoclonal anti-SMN antibody (CST, #12976) or a rabbit polyclonal anti-α/β-tubulin antibody (CST, #2148), and an anti-mouse IgG HRP-linked antibody (CST, #7076) or an anti-rabbit IgG HRP-linked antibody (CST, #7074) was used as a secondary antibody. The membrane was scanned using Image Lab to detect protein signals.

As shown in **FIG. 8A****,** RAG6-281 and RAG6-550 significantly activated the expression of SMN mRNA by more than 1.5-fold even at the transfection concentration of 1 nM, and caused a peak increase in SMN expression (2.38 fold and 2.16 fold respectively) at the concentration of 50 nM. When they were transfected at 100 nM, there was no furtuer increase in SMN expression. In addition, RT-PCR/*Dde*I digestion analysis showed that both RAG6-281 and RAG6-550 upregulated the mRNA expressions of SMN1 and SMN2, which is consistent with the results of RT-qPCR. By quantifying the ratio of full-length SMN2 bands to SMN2Δ7 bands, it was found that RAG6-281 and RAG6-550 significantly increased the expression of full-length SMN2 mRNA at all concentrations tested (**FIG. 8B**). Compared with Mock treatment, RAG6-281 increased the ratio of full-length SMN2 mRNA to SMN2Δ7 mRNA by 1.9 fold, 2.39 fold, 2.41 fold, 2.39 fold and 2.1 fold when transfected at concentrations of 1 nM, 10 nM, 20 nM, 50 nM and 100 nM, respectively; and RAG6-550 increased the ratio of full-length SMN2 mRNA to SMN2Δ7 mRNA by 1.52 fold, 1.99 fold, 1.91 fold, 2.3 fold and 1.7 fold at the same concentrations as above, respectively. The changes induced by RAG6-281 and RAG6-550 was in a dose-dependent manner at the transfection concentrations ranging from 1 nM to 50 nM (**FIG. 8B**). The change in SMN protein expression was further assayed by Western blot analysis and was highly consistent with that of mRNA as assessed by RT-qPCR, indicating that both RAG6-281 and RAG6-550 significantly upregulated the expression level of full-length SMN protein in a dose-dependent manner (**FIG. 8C**)**.** However, exon 7-deleted SMN protein (SMN2Δ7) bands were not detected by Western blot probably due to its high instability, which is consistent with that reported in the literature (Hua et al, PLoS Biol 2007;5(4)e73).

### Example 6: In Vivo Efficacy of saRNAs in Improving Mortor Function of Type I SMA Mice

### 1. Breeding and genotyping of type I SMA (SMA I) mice

Newborn mice were derived by crossing Smn1^{+/-}, SMN2^{-/-} mice (with mouse Smn1 gene heterozygous knockout) and Smn1^{-/-}, SMN2^{+/+}mice (type III SMA mice with two copies of human SMN2 transgene) (provided by Beijing Ruicy Gene Therapy Institute for Rare Diseases) and genotyped by genomic PCR assay (**FIG. 9**). Pups with the following genotypes were used in this study: SMA I mice which carried hmologyous deletion of mouse Smn gene and a heterozygous transgene of human SMN2 with the genotype of Smn1^{-/-}, SMN2^{+/-}, SMA heterozygous (Het) mice (normal control) which carried heterozygous deletion of mouse Smn1 and a heterozygous transgene of human SMN2 with the genotype of Smn^{+/-}, SMN2^{+/-}.

### 2. Preparation of in vivo-jetPEI- and HKP-formulated oligonucleotides

Preparation of *in-vivo* jetPEI formulation: To prepare 5 mg/mL stock solution, SMN2-saRNA RAG6-539 (DS06-0013B) was dissolved in RNase-free water (Invitrogen, 2063810). 5 µL of DS06-0013B and 12.5 µL of 10% glucose solution (Polyplus-transfection, G181106) were gently mixed with 3.5 µL of RNase-free water to prepare DS06-0013B working solution. The working solution was added to 4 µL of *in-vivo*-jetPEI (Polyplus-transfection, 26031A1C) and mixed, and the mixture was incubated at room temperature for 15 min, with a final RNA concentration of 1 mg/mL.

Preparation of HKP formulation: SMN2-saRNA RAG6-538 (DS06-0002B) was dissolved in RNase-free water to prepare 4 mg/mL stock solution. Histidine-Lysine co-Polymer (HKP) (Suzhou Sirnaomics Biopharmaceuticals Co. Ltd., AKF271/042-79-11) was dissolved in RNase-free water to prepare 16 mg/mL stock solution. 7.5 µL of HKP stock solution was rapidly mixed with 7.5 µL of DS06-0002B stock solution, and the mixture was placed at room temperature for 30 min, with a final RNA concentration of 2 mg/mL.

### 3. Intracerebroventricular injection of in-vivo-jetPEI- and HKP-formulated oligonucleotides in SMA I mice

Pup mice as above genotyped were divided into four groups and they were: Het control mice, SMA control (SMA I mice, untreated), DS06-0013B-J (SMA I mice treated with *in vivo-*jetPEI-formulated DS06-0013B, 1 mg/mL), and DS06-0002B-H (SMA I mice treated with HKP-formulated DS06-0002B, 2 mg/mL). The newborn mice were administrated by intracerebroventricular injection (ICV) on postnatal day 1 (P1) with an injection volume of 5 µL. Animal grouping, administration route, injection volume and time are shown in Table 8.

**Table 8. Administration of saRNAs and control groups**

| Group | Number of animals | Administration route | Injection volume | Administration time |
|---|---|---|---|---|
| DS06-0002B-H (RAG6-538) | 4 | ICV | 5 µL | P1 |
| DS06-0013B-J (RAG6-539) | 2 | ICV | 5 µL | P1 |
| SMA control | 3 | N/A | N/A | N/A |
| Het control | 4 | N/A | N/A | N/A |

### 4. Assessment of motor function of SMA I mice

Motor function of mice was assessed on P7 or P8 by the righting reflex test. Briefly, mice in the normal standing posture were placed in supine position with their their backs touching the experimental tabletop and limbs facing up and were then released to let them return to normal standing posture. The time (in second) needed for them to return to normal position was recorded and was described as righting reflex time or righting time. If a mouse failed to return to the normal posture within 60 s, then the righting time was recorded as > 60 s. The righting time reflects the motor capacity of the mice, the shorter the righting time, the better the motor capability of the mice. Table 9 lists the righting time of mice in this study.

**Table 9. Righting time of mice**

| Group | Number of animals | Animal No. | Date of assessment | Righting time (second) |
|---|---|---|---|---|
| DS06-0002B-H (RAG6-538) | 2 | 15081503 | P7 | 4 |
| | | 15081505 | P7 | 5 |
| DS06-0013B-J (RAG6-539) | 4 | 15081509 | P7 | 3.5 |
| | | 35081405 | P8 | 3 |
| | | 35081406 | P8 | 4 |
| | | 35081407 | P8 | > 60 |
| SMA control | 3 | 35081403 | P7 | > 60 |
| | | 15081502 | P7 | 12 |
| | | 35081404 | P8 | > 60 |
| Het control | 4 | 15081506 | P7 | 2 |
| | | 15081507 | P7 | 2 |
| | | 15081510 | P7 | 2 |
| | | 35081402 | P8 | 1.5 |

FIG. 10 shows the motor function of the SMA I mice after administration of SMN2-saRNAs. As shown in Table 9, the righting time of the mice in the normal control group (Het) was within 2 s, while the righting time of the mice in the SMA I control group (untreated) was at least 12 s, and two of which completely lost their ability to right themselves (righting time > 60 s). The righting time of the two saRNA-treated groups [RAG6-538 (DS06-0002B-H) and DS06-0013B (DS06-0013B-J)] was close to that of normal mice, particularly the mice in the DS06-0002B-H group. Compared with SMA I control group, the righting time of the mice in DS06-0002B-H group was shortened by nearly 10-fold, and the righting time of the mice in the DS06-0013B-J group was shortened by 2.5-fold (Table 9, **FIG. 10****).** This result demonstrates that the motor function of the SMA I mice was significantly improved after administering SMN2-saRNAs, suggesting that saRNA therapy can delay the onset of the disease.

In summary, based on high throughput screening of SMN promoter-targeting saRNAs, a plurality of saRNAs capable of significantly activating the expression of SMN gene were identified. These saRNAs not only can upregulate the expression of SMN2 gene in a dose-dependent manner, but also can significantly increase the ratio of full-length SMN2 protein to SMN2Δ7 protein in cells. Moreover, an *in vivo* efficacy study proved that the saRNA disclosed herein can significantly improve the motor capability of SMA I mice. These results clearly suggested that activating SMN2 expression at the transcriptional level with the saRNAs targeting MSN2 promoter to increase full-length SMN protein expression is a promising strategy to treat SMA.

### References

1. Kolb SJ, Coffey CS, Yankey JW, Krosschell K, Arnold WD, et al. 2017. Natural history of infantile-onset spinal muscular atrophy. Ann Neurol 82:883-91
2. Sugarman EA, Nagan N, Zhu H, Akmaev VR, Zhou Z, et al. 2012. Pan-ethnic carrier screening and prenatal diagnosis for spinal muscular atrophy: clinical laboratory analysis of>72,400 specimens. Eur J Hum Genet 20:27-32
3. Zhang C, Lang Q, 2017. New Therapeutic medication for treating Spinal Muscular Atrophy— —SPINRAZA. Journal of Clinical Phamacology 15:83-4
4. Lefebvre S, Burglen L, Reboullet S, Clermont O, Burlet P, et al. 1995. Identification and characterization of a spinal muscular atrophy-determining gene. Cell 80:155-65
5. Lorson CL, Hahnen E, Androphy EJ, Wirth B. 1999. A single nucleotide in the SMN gene regulates splicing and is responsible for spinal muscular atrophy. Proc Natl Acad Sci USA 96:6307-11
6. Monani UR, Lorson CL, Parsons DW, Prior TW, Androphy EJ, et al. 1999. A single nucleotide difference that alters splicing patterns distinguishes the SMA gene SMN1 from the copy gene SMN2. Hum Mol Genet 8:1177-83
7. Hua Y, Sahashi K, Hung G, Rigo F, Passini MA, et al. 2010. Antisense correction of SMN2 splicing in the CNS rescues necrosis in a type III SMA mouse model. Genes Dev 24:1634-44
8. Hua Y, Sahashi K, Rigo F, Hung G, Horev G, et al. 2011. Peripheral SMN restoration is essential for long-term rescue of a severe spinal muscular atrophy mouse model. Nature 478:123-6
9. Naryshkin NA, Weetall M, Dakka A, Narasimhan J, Zhao X, et al. 2014. Motor neuron disease. SMN2 splicing modifiers improve motor function and longevity in mice with spinal muscular atrophy. Science 345:688-93
10. Palacino J, Swalley SE, Song C, Cheung AK, Shu L, et al. 2015. SMN2 splice modulators enhance U1-pre-mRNA association and rescue SMA mice. Nat Chem Biol 11:511-7
11. Michelson D, Ciafaloni E, Ashwal S, Lewis E, Narayanaswami P, et al. 2018. Evidence in focus: Nusinersen use in spinal muscular atrophy: Report of the Guideline Development, Dissemination, and Implementation Subcommittee of the American Academy of Neurology. Neurology
12. Avila AM, Burnett BG, Taye AA, Gabanella F, Knight MA, et al. 2007. Trichostatin A increases SMN expression and survival in a mouse model of spinal muscular atrophy. J Clin Invest 117:659-71
13. Somers E, Riessland M, Schreml J, Wirth B, Gillingwater TH, Parson SH. 2013. Increasing SMN levels using the histone deacetylase inhibitor SAHA ameliorates defects in skeletal muscle microvasculature in a mouse model of severe spinal muscular atrophy. Neurosci Lett 544:100-4
14. Swoboda KJ, Scott CB, Crawford TO, Simard LR, Reyna SP, et al. 2010. SMA CARNI-VAL trial part I: double-blind, randomized, placebo-controlled trial of L-carnitine and valproic acid in spinal muscular atrophy. PLoS One 5:e12140
15. Kissel JT, Scott CB, Reyna SP, Crawford TO, Simard LR, et al. 2011. SMA CARNIVAL TRIAL PART II: a prospective, single-armed trial of L-carnitine and valproic acid in ambulatory children with spinal muscular atrophy. PLoS One 6:e21296_{∘}

## Claims

1. A small activating nucleic acid molecule, wherein one strand of the small activating nucleic acid molecule has at least 75% homology or complementarity to a continuous sequence of 16 to 35 nucleotides in length in the promoter of SMN2 gene in the region -1639 to -1481 (SEQ ID NO: 476), region -1090 to -1008 (SEQ ID NO: 477), region -994 to -180 (SEQ ID NO: 478) or region -144 to -37 (SEQ ID NO: 479) upstream of the transcription start site of SMN2 gene.

2. The small activating nucleic acid molecule of claim 1, comprising a sense nucleic acid fragment and an antisense nucleic acid fragment, wherein the sense nucleic acid fragment and the antisense nucleic acid fragment comprise complementary regions, wherein the complementary regions form a double-stranded nucleic acid structure that can activate the expression of the SMN2 gene in cells.

3. The small activating nucleic acid molecule of claim 2, wherein the sense nucleic acid fragment and the antisense nucleic acid fragment are located on two different nucleic acid strands.

4. The small activating nucleic acid molecule of claim 2, wherein the sense nucleic acid fragment and the antisense nucleic acid fragment are located on an identical nucleic acid strand, preferably a hairpin single-stranded nucleic acid molecule, wherein the complementary regions of the sense nucleic acid fragment and the antisense nucleic acid fragment form a double-stranded nucleic acid structure.

5. The small activating nucleic acid molecule of claim 3, wherein at least one strand has a 3' overhang of 0 to 6 nucleotides in length.

6. The small activating nucleic acid molecule of claim 5, wherein both strands have a 3' overhang of 2 to 3 nucleotides in length.

7. The small activating nucleic acid molecule of any of claims 2-6, wherein the sense nucleic acid fragment and the antisense nucleic acid fragment independently have 16 to 35 nucleotides in length.

8. The small activating nucleic acid molecule of claim 1, wherein one strand of the small activating nucleic acid molecule has at least 75% homology or complementarity to a nucleotide sequence selected from SEQ ID NOs: 315-471.

9. The small activating nucleic acid molecule of claim 8, wherein a sense fragment of the small activating nucleic acid molecule has at least 75% homology to a nucleotide sequence selected from SEQ ID NOs: 1-157, and an antisense fragment of the small activating nucleic acid molecule has at least 75% homology to a nucleotide sequence selected from SEQ ID NOs: 158-314.

10. The small activating nucleic acid molecule of claim 9, wherein the sense fragment of the small activating nucleic acid molecule comprises or is a nucleotide sequence selected from SEQ ID NOs: 1-157, and the antisense fragment of the small activating nucleic acid molecule comprises or is a nucleotide sequence selected from SEQ ID NOs: 158-314.

11. The small activating nucleic acid molecule of any of claims 1-10, wherein at least one nucleotide is a chemically modified nucleotide.

12. The small activating nucleic acid molecule of claim 11, wherein the chemical modified nucleotide is chosen from at least one of the following modifications:
a) modification of a phosphodiester bond connecting nucleotides in the nucleotide sequence of the small activating nucleic acid molecule;
b) modification of 2'-OH of a ribose in the nucleotide sequence of the small activating nucleic acid molecule; and
c) modification of a base in the nucleotide sequence of the small activating nucleic acid molecule.

13. The small activating nucleic acid molecule of any of claims 1-12, wherein the small activating nucleic acid molecule activates/up-regulates the expression of SMN2 gene by at least 10%.

14. A nucleic acid encoding the small activating nucleic acid molecule of any one of claims 1-10.

15. The nucleic acid of claim 14, wherein the nucleic acid is a DNA molecule.

16. A cell comprising the small activating nucleic acid molecule of any one of claims 1-13 or the nucleic acid of claim 14 or claim 15.

17. A composition comprising the small activating nucleic acid molecule of any one of claims 1-13 or the nucleic acid of claim 14 or claim 15 and optionally, a pharmaceutically acceptable carrier.

18. The composition of claim 17, wherein the pharmaceutically acceptable carrier is chosen from an aqueous carrier, a liposome, a high-molecular polymer and/or a polypeptide.

19. The composition of claim 17 or 18, wherein the composition comprises 1-150 nM of the small activating nucleic acid molecule.

20. A method for treating a disease or condition induced by insufficient expression of SMN protein, SMN1 gene mutation or deletion or insufficient expression of full-length protein, and/or insufficient expression of full-length SMN2 protein in an individual comprising: administering a therapeutic dose of the small activating nucleic acid molecule of any one of claims 1-13, the nucleic acid of claim 14 or claim 15, or the composition of any one of claims 17-19 to the individual.

21. The method of claim 20, wherein the disease or condition is a hereditary neuromuscular disease, preferably spinal muscular atrophy.

22. The method of claim 20 or claim 21, wherein the individual is a mammal, preferably a human.

23. The use of the small activating nucleic acid molecule of any one of claims 1-13, the nucleic acid of claim 14 or claim 15, or the composition of any of claims 17-19 in preparing a medicament for treating a disease or condition induced by insufficient expression of SMN protein, SMN 1 gene mutation or deletion or insufficient expression of full-length protein, and/or insufficient expression of full-length SMN2 protein in an individual.

24. The use of claim 23, wherein the disease or condition is a hereditary neuromuscular disease, preferably spinal muscular atrophy.

25. The use of claim 23 or claim 24, wherein the individual is a mammal, preferably a human.

26. The use of the small activating nucleic acid molecule of any one of claims 1-13, the nucleic acid of claim 14 or claim 15, or the composition of any of claims 17-19 in preparing a preparation for activating/up-regulating expression of SMN2 gene in a cell.

27. The use of claim 26, wherein the small activating nucleic acid molecule, the nucleic acid of claim 14 or claim 15, or the composition of any of claims 17-19 is directly introduced into the cell.

28. The use of claim 26, wherein the small activating nucleic acid molecule is produced in the cell after a nucleotide sequence encoding the small activating nucleic acid molecule is introduced into the cell.

29. The use of any of claims 26-28, wherein the cell is a mammalian cell, preferably a human cell.

30. The use of claim 29, wherein the cell is in a human body.

31. The use of claim 30, wherein the human body is a patient suffering from a symptom induced by the insufficient expression of SMN protein, a SMN1 gene mutation or SMN1 gene deletion or insufficient expression of full-length protein, and/or insufficient expression of full-length SMN2 protein, wherein the small activating nucleic acid molecule, the nucleic acid or the composition is administered in a sufficient amount to treat the symptom.

32. The use of claim 31, wherein the symptom induced by insufficient expression of full-length SMN protein is a hereditary neuromuscular disease, preferably spinal muscular atrophy.

33. An isolated target site of small SMN2-activating nucleic acid molecule, wherein the target site comprises 16 to 35 continuous nucleotides in a sequence selected from SEQ ID NOs: 476-479.

34. The small activating nucleic acid molecule target site of claim 33, wherein the target site is in a nucleotide sequence selected from SEQ ID NOs: 315-471.

35. A method for activating/up-regulating expression of SMN2 gene in a cell comprising: administering the small activating nucleic acid molecule of any of claims 1-13, the nucleic acid of claim 14 or claim 15, or the composition of any of claims 17-19 to the cell.

36. The method of claim 35, wherein the small activating nucleic acid molecule of any of claims 1-13, the nucleic acid of claim 14 or claim 15, or the composition of any of claims 17-19 is directly introduced into the cell.

37. The method of claim 35, wherein the small activating nucleic acid molecule is produced in the cell after a nucleotide sequence encoding the small activating nucleic acid molecule is introduced into the cell.

38. The method of any of claims 35-37, wherein the cell is a mammalian cell, preferably a human cell.

39. The method of claim 38, wherein the cell is in a human body.

40. The method of claim 39, wherein the human body is a patient suffering from a symptom induced by insufficient expression of the full-length SMN protein, a SMN1 gene mutation or deletion or insufficient expression of full-length protein, and/or insufficient expression of full-length SMN2 protein, wherein the small activating nucleic acid molecule, the nucleic acid or the composition is administered in a sufficient amount to treat the symptom.

41. The method of claim 40, wherein the symptom caused by insufficient expression of full-length SMN protein is hereditary neuromuscular disease, preferably spinal muscular atrophy.


Sequence Listing

<110>  Ractigen Therapeutics

<120>  OLIGONUCLEOTIDE MOLECULE AND USE THEREOF IN TUMOR TREATMENT

<130>  20191126

<160>  491

<170>  PatentIn version 3.5

<210>  1
<211>  21
<212>  DNA/RNA
<213>  Artificially synthesized

<400>  1
aucugugaga uguaccuuut t                                                21


<210>  2
<211>  21
<212>  DNA/RNA
<213>  Artificially synthesized

<400>  2
cacucuguca cucaggcugt t                                                21


<210>  3
<211>  21
<212>  DNA/RNA
<213>  Artificially synthesized

<400>  3
acucugucac ucaggcuggt t                                                21


<210>  4
<211>  21
<212>  DNA/RNA
<213>  Artificially synthesized

<400>  4
ucugucacuc aggcuggagt t                                                21


<210>  5
<211>  21
<212>  DNA/RNA
<213>  Artificially synthesized

<400>  5
ucaggcugga gugcaguggt t                                                21


<210>  6
<211>  21
<212>  DNA/RNA
<213>  Artificially synthesized

<400>  6

gagugcagug gcgugaucut t                                                21

<210>  7
<211>  21
<212>  DNA/RNA
<213>  Artificially synthesized

<400>  7
ugcaguggcg ugaucuuggt t                                                21

<210>  8
<211>  21
<212>  DNA/RNA
<213>  Artificially synthesized

<400>  8
gcaguggcgu gaucuuggct t                                                21

<210>  9
<211>  21
<212>  DNA/RNA
<213>  Artificially synthesized

<400>  9
ggcgugaucu uggcucacut t                                                21

<210>  10
<211>  21
<212>  DNA/RNA
<213>  Artificially synthesized

<400>  10
gugaucuugg cucacugcat t                                                21

<210>  11
<211>  21
<212>  DNA/RNA
<213>  Artificially synthesized

<400>  11
ugaucuuggc ucacugcaat t                                                21

<210>  12
<211>  21
<212>  DNA/RNA
<213>  Artificially synthesized

<400>  12
aucuuggcuc acugcaacct t                                                21

<210>  13
<211>  21
<212>  DNA/RNA
<213>  Artificially synthesized

<400>  13

cuuggcucac ugcaaccuct t                                                21

<210>  14
<211>  21
<212>  DNA/RNA
<213>  Artificially synthesized

<400>  14
uuggcucacu gcaaccucct t                                                21

<210>  15
<211>  21
<212>  DNA/RNA
<213>  Artificially synthesized

<400>  15
gccucccgag uucaagugat t                                                21

<210>  16
<211>  21
<212>  DNA/RNA
<213>  Artificially synthesized

<400>  16
ccucccgagu ucaagugaut t                                                21

<210>  17
<211>  21
<212>  DNA/RNA
<213>  Artificially synthesized

<400>  17
cucccgaguu caagugauut t                                                21

<210>  18
<211>  21
<212>  DNA/RNA
<213>  Artificially synthesized

<400>  18
ucccgaguuc aagugauuct t                                                21

<210>  19
<211>  21
<212>  DNA/RNA
<213>  Artificially synthesized

<400>  19
ucaagugauu cuccuggcut t                                                21

<210>  20
<211>  21
<212>  DNA/RNA
<213>  Artificially synthesized

<400>  20

aagugauucu ccuggcucat t                                                21

<210>  21
<211>  21
<212>  DNA/RNA
<213>  Artificially synthesized

<400>  21
agugauucuc cuggcucagt t                                                21

<210>  22
<211>  21
<212>  DNA/RNA
<213>  Artificially synthesized

<400>  22
gugauucucc uggcucagct t                                                21

<210>  23
<211>  21
<212>  DNA/RNA
<213>  Artificially synthesized

<400>  23
cagccuccca agcagcugut t                                                21

<210>  24
<211>  21
<212>  DNA/RNA
<213>  Artificially synthesized

<400>  24
ccucccaagc agcugucaut t                                                21

<210>  25
<211>  21
<212>  DNA/RNA
<213>  Artificially synthesized

<400>  25
ucccaagcag cugucauuat t                                                21

<210>  26
<211>  21
<212>  DNA/RNA
<213>  Artificially synthesized

<400>  26
agcugucauu acaggccugt t                                                21

<210>  27
<211>  21
<212>  DNA/RNA
<213>  Artificially synthesized

<400>  27

gcugucauua caggccugct t                                                21

<210>  28
<211>  21
<212>  DNA/RNA
<213>  Artificially synthesized

<400>  28
cugucauuac aggccugcat t                                                21

<210>  29
<211>  21
<212>  DNA/RNA
<213>  Artificially synthesized

<400>  29
ggagaaacag gguuucacct t                                                21

<210>  30
<211>  21
<212>  DNA/RNA
<213>  Artificially synthesized

<400>  30
agaaacaggg uuucaccaut t                                                21

<210>  31
<211>  21
<212>  DNA/RNA
<213>  Artificially synthesized

<400>  31
aagugcuggg auuauaggct t                                                21

<210>  32
<211>  21
<212>  DNA/RNA
<213>  Artificially synthesized

<400>  32
uuauaggcau gagccaccgt t                                                21

<210>  33
<211>  21
<212>  DNA/RNA
<213>  Artificially synthesized

<400>  33
auucuccccu uccuccacat t                                                21

<210>  34
<211>  21
<212>  DNA/RNA
<213>  Artificially synthesized

<400>  34

ucuccccuuc cuccacagat t                                                21

<210>  35
<211>  21
<212>  DNA/RNA
<213>  Artificially synthesized

<400>  35
cauuuagcaa cccuagaugt t                                                21

<210>  36
<211>  21
<212>  DNA/RNA
<213>  Artificially synthesized

<400>  36
auuuagcaac ccuagaugct t                                                21

<210>  37
<211>  21
<212>  DNA/RNA
<213>  Artificially synthesized

<400>  37
uuuagcaacc cuagaugcut t                                                21

<210>  38
<211>  21
<212>  DNA/RNA
<213>  Artificially synthesized

<400>  38
uuagcaaccc uagaugcuut t                                                21

<210>  39
<211>  21
<212>  DNA/RNA
<213>  Artificially synthesized

<400>  39
uagcaacccu agaugcuuat t                                                21

<210>  40
<211>  21
<212>  DNA/RNA
<213>  Artificially synthesized

<400>  40
auacuggagg cccggugugt t                                                21

<210>  41
<211>  21
<212>  DNA/RNA
<213>  Artificially synthesized

<400>  41

gugugguggc ucacaccugt t                                                21

<210>  42
<211>  21
<212>  DNA/RNA
<213>  Artificially synthesized

<400>  42
ugguggcuca caccuguaat t                                                21

<210>  43
<211>  21
<212>  DNA/RNA
<213>  Artificially synthesized

<400>  43
gguggcucac accuguaaut t                                                21

<210>  44
<211>  21
<212>  DNA/RNA
<213>  Artificially synthesized

<400>  44
guggcucaca ccuguaauct t                                                21

<210>  45
<211>  21
<212>  DNA/RNA
<213>  Artificially synthesized

<400>  45
ggcucacacc uguaauccct t                                                21

<210>  46
<211>  21
<212>  DNA/RNA
<213>  Artificially synthesized

<400>  46
cacaccugua aucccagcat t                                                21

<210>  47
<211>  21
<212>  DNA/RNA
<213>  Artificially synthesized

<400>  47
acaccuguaa ucccagcact t                                                21

<210>  48
<211>  21
<212>  DNA/RNA
<213>  Artificially synthesized

<400>  48

accuguaauc ccagcacuut t                                                21

<210>  49
<211>  21
<212>  DNA/RNA
<213>  Artificially synthesized

<400>  49
guaaucccag cacuuugggt t                                                21

<210>  50
<211>  21
<212>  DNA/RNA
<213>  Artificially synthesized

<400>  50
uaaucccagc acuuugggat t                                                21

<210>  51
<211>  21
<212>  DNA/RNA
<213>  Artificially synthesized

<400>  51
aaucccagca cuuugggagt t                                                21

<210>  52
<211>  21
<212>  DNA/RNA
<213>  Artificially synthesized

<400>  52
cagcacuuug ggaggccgat t                                                21

<210>  53
<211>  21
<212>  DNA/RNA
<213>  Artificially synthesized

<400>  53
gaggcggucg gauuacgagt t                                                21

<210>  54
<211>  21
<212>  DNA/RNA
<213>  Artificially synthesized

<400>  54
ggcggucgga uuacgaggut t                                                21

<210>  55
<211>  21
<212>  DNA/RNA
<213>  Artificially synthesized

<400>  55

gcggucggau uacgagguct t                                                21

<210>  56
<211>  21
<212>  DNA/RNA
<213>  Artificially synthesized

<400>  56
cggucggauu acgaggucat t                                                21

<210>  57
<211>  21
<212>  DNA/RNA
<213>  Artificially synthesized

<400>  57
ggucggauua cgaggucagt t                                                21

<210>  58
<211>  21
<212>  DNA/RNA
<213>  Artificially synthesized

<400>  58
gucggauuac gaggucaggt t                                                21

<210>  59
<211>  21
<212>  DNA/RNA
<213>  Artificially synthesized

<400>  59
ucggauuacg aggucaggat t                                                21

<210>  60
<211>  21
<212>  DNA/RNA
<213>  Artificially synthesized

<400>  60
cggauuacga ggucaggagt t                                                21

<210>  61
<211>  21
<212>  DNA/RNA
<213>  Artificially synthesized

<400>  61
auuacgaggu caggaguuct t                                                21

<210>  62
<211>  21
<212>  DNA/RNA
<213>  Artificially synthesized

<400>  62

uuacgagguc aggaguucat t                                                21

<210>  63
<211>  21
<212>  DNA/RNA
<213>  Artificially synthesized

<400>  63
uacgagguca ggaguucaat t                                                21

<210>  64
<211>  21
<212>  DNA/RNA
<213>  Artificially synthesized

<400>  64
acgaggucag gaguucaagt t                                                21

<210>  65
<211>  21
<212>  DNA/RNA
<213>  Artificially synthesized

<400>  65
aggucaggag uucaagacct t                                                21

<210>  66
<211>  21
<212>  DNA/RNA
<213>  Artificially synthesized

<400>  66
aguucaagac cagccuggct t                                                21

<210>  67
<211>  21
<212>  DNA/RNA
<213>  Artificially synthesized

<400>  67
gaaaccccau cuuuacuaat t                                                21

<210>  68
<211>  21
<212>  DNA/RNA
<213>  Artificially synthesized

<400>  68
   auuagccggg ugugguggut t                                                21

<210>  69
<211>  21
<212>  DNA/RNA
<213>  Artificially synthesized

<400>  69
   guggugggcg ccuguaauct t                                                21

<210>  70
<211>  21
<212>  DNA/RNA
<213>  Artificially synthesized

<400>  70
ggcgccugua aucccagcut t                                                21

<210>  71
<211>  21
<212>  DNA/RNA
<213>  Artificially synthesized

<400>  71
uaaucccagc uacucggggt t                                                21

<210>  72
<211>  21
<212>  DNA/RNA
<213>  Artificially synthesized

<400>  72
gggcugaggc agaauugcut t                                                21

<210>  73
<211>  21
<212>  DNA/RNA
<213>  Artificially synthesized

<400>  73
ggcagaauug cuugaaccut t                                                21

<210>  74
<211>  21
<212>  DNA/RNA
<213>  Artificially synthesized

<400>  74
cagaauugcu ugaaccuggt t                                                21

<210>  75
<211>  21
<212>  DNA/RNA
<213>  Artificially synthesized

<400>  75
ugaaccuggg aggcagaggt t                                                21

<210>  76
<211>  21
<212>  DNA/RNA
<213>  Artificially synthesized

<400>  76

gaaccuggga ggcagaggut t                                                21

<210>  77
<211>  21
<212>  DNA/RNA
<213>  Artificially synthesized

<400>  77
accugggagg cagagguugt t                                                21

<210>  78
<211>  21
<212>  DNA/RNA
<213>  Artificially synthesized

<400>  78
ugcagugagc ugagaucact t                                                21

<210>  79
<211>  21
<212>  DNA/RNA
<213>  Artificially synthesized

<400>  79
cugagaucac gccacugcat t                                                21

<210>  80
<211>  21
<212>  DNA/RNA
<213>  Artificially synthesized

<400>  80
aucacgccac ugcauuccat t                                                21

<210>  81
<211>  21
<212>  DNA/RNA
<213>  Artificially synthesized

<400>  81
gggugacaga gcaauacuct t                                                21

<210>  82
<211>  21
<212>  DNA/RNA
<213>  Artificially synthesized

<400>  82
ugacagagca auacucugut t                                                21

<210>  83
<211>  21
<212>  DNA/RNA
<213>  Artificially synthesized

<400>  83

agagcaauac ucugucgcat t                                                21

<210>  84
<211>  21
<212>  DNA/RNA
<213>  Artificially synthesized

<400>  84
aaaagaauac uggaggcugt t                                                21

<210>  85
<211>  21
<212>  DNA/RNA
<213>  Artificially synthesized

<400>  85
aaagaauacu ggaggcuggt t                                                21

<210>  86
<211>  21
<212>  DNA/RNA
<213>  Artificially synthesized

<400>  86
auacuggagg cugggcgagt t                                                21

<210>  87
<211>  21
<212>  DNA/RNA
<213>  Artificially synthesized

<400>  87
cgagguggcu cacaccugut t                                                21

<210>  88
<211>  21
<212>  DNA/RNA
<213>  Artificially synthesized

<400>  88
gguggcucac accuguaaut t                                                21

<210>  89
<211>  21
<212>  DNA/RNA
<213>  Artificially synthesized

<400>  89
ggcucacacc uguaauccct t                                                21

<210>  90
<211>  21
<212>  DNA/RNA
<213>  Artificially synthesized

<400>  90

gcucacaccu guaaucccat t                                                21

<210>  91
<211>  21
<212>  DNA/RNA
<213>  Artificially synthesized

<400>  91
cacaccugua aucccagcat t                                                21

<210>  92
<211>  21
<212>  DNA/RNA
<213>  Artificially synthesized

<400>  92
uaaucccagc auuuugggat t                                                21

<210>  93
<211>  21
<212>  DNA/RNA
<213>  Artificially synthesized

<400>  93
gggcggaaua ucuugagcut t                                                21

<210>  94
<211>  21
<212>  DNA/RNA
<213>  Artificially synthesized

<400>  94
aauaucuuga gcucaggagt t                                                21

<210>  95
<211>  21
<212>  DNA/RNA
<213>  Artificially synthesized

<400>  95
uucgagacca gccuacacat t                                                21

<210>  96
<211>  21
<212>  DNA/RNA
<213>  Artificially synthesized

<400>  96
ccagccuaca caauaugcut t                                                21

<210>  97
<211>  21
<212>  DNA/RNA
<213>  Artificially synthesized

<400>  97

acacaauaug cuccaaacgt t                                                21

<210>  98
<211>  21
<212>  DNA/RNA
<213>  Artificially synthesized

<400>  98
cacaauaugc uccaaacgct t                                                21

<210>  99
<211>  21
<212>  DNA/RNA
<213>  Artificially synthesized

<400>  99
acaauaugcu ccaaacgcct t                                                21

<210>  100
<211>  21
<212>  DNA/RNA
<213>  Artificially synthesized

<400>  100
caaacgccgc cucuacaaat t                                                21

<210>  101
<211>  21
<212>  DNA/RNA
<213>  Artificially synthesized

<400>  101
cuguggyccu agcuacuugt t                                                21

<210>  102
<211>  21
<212>  DNA/RNA
<213>  Artificially synthesized

<400>  102
gggaggaucg cuugagcuct t                                                21

<210>  103
<211>  21
<212>  DNA/RNA
<213>  Artificially synthesized

<400>  103
gaggaucgcu ugagcucggt t                                                21

<210>  104
<211>  21
<212>  DNA/RNA
<213>  Artificially synthesized

<400>  104

ggaggucgag gcugcaaugt t                                              21


<210>  105
<211>  21
<212>  DNA/RNA
<213>  Artificially synthesized

<400>  105
ggucgaggcu gcaaugagct t                                              21


<210>  106
<211>  21
<212>  DNA/RNA
<213>  Artificially synthesized

<400>  106
caaugagccg agauggugct t                                              21


<210>  107
<211>  21
<212>  DNA/RNA
<213>  Artificially synthesized

<400>  107
aaugagccga gauggugcct t                                              21


<210>  108
<211>  21
<212>  DNA/RNA
<213>  Artificially synthesized

<400>  108
ccgagauggu gccacugcat t                                              21


<210>  109
<211>  21
<212>  DNA/RNA
<213>  Artificially synthesized

<400>  109
gagauggugc cacugcacut t                                              21


<210>  110
<211>  21
<212>  DNA/RNA
<213>  Artificially synthesized

<400>  110
agauggugcc acugcacuct t                                              21


<210>  111
<211>  21
<212>  DNA/RNA
<213>  Artificially synthesized

<400>  111

auggugccac ugcacucugt t                                                21

<210>  112
<211>  21
<212>  DNA/RNA
<213>  Artificially synthesized

<400>  112
ccacugcacu cugacgacat t                                                21

<210>  113
<211>  21
<212>  DNA/RNA
<213>  Artificially synthesized

<400>  113
cacugcacuc ugacgacagt t                                                21

<210>  114
<211>  21
<212>  DNA/RNA
<213>  Artificially synthesized

<400>  114
ucugacgaca gagcgagact t                                                21

<210>  115
<211>  21
<212>  DNA/RNA
<213>  Artificially synthesized

<400>  115
cugacgacag agcgagacut t                                                21

<210>  116
<211>  21
<212>  DNA/RNA
<213>  Artificially synthesized

<400>  116
gagacuccgu cucaaaacat t                                                21

<210>  117
<211>  21
<212>  DNA/RNA
<213>  Artificially synthesized

<400>  117
agacuccguc ucaaaacaat t                                                21

<210>  118
<211>  21
<212>  DNA/RNA
<213>  Artificially synthesized

<400>  118

ucuaguguuu aaggaucugt t                                                21

<210>  119
<211>  21
<212>  DNA/RNA
<213>  Artificially synthesized

<400>  119
uaguguuuaa ggaucugcct t                                                21

<210>  120
<211>  21
<212>  DNA/RNA
<213>  Artificially synthesized

<400>  120
uguuuaagga ucugccuuct t                                                21

<210>  121
<211>  21
<212>  DNA/RNA
<213>  Artificially synthesized

<400>  121
ggaucugccu uccuuccugt t                                                21

<210>  122
<211>  21
<212>  DNA/RNA
<213>  Artificially synthesized

<400>  122
uugucuuucc uuguuuguct t                                                21

<210>  123
<211>  21
<212>  DNA/RNA
<213>  Artificially synthesized

<400>  123
gucuuuccuu guuugucuut t                                                21

<210>  124
<211>  21
<212>  DNA/RNA
<213>  Artificially synthesized

<400>  124
caagcagguu uuaaauucct t                                                21

<210>  125
<211>  21
<212>  DNA/RNA
<213>  Artificially synthesized

<400>  125

gcagguuuua aauuccuagt t                                                21

<210>  126
<211>  21
<212>  DNA/RNA
<213>  Artificially synthesized

<400>  126
acauuuacuu uuccaagggt t                                                21

<210>  127
<211>  21
<212>  DNA/RNA
<213>  Artificially synthesized

<400>  127
acacuggagu ucgagacgat t                                                21

<210>  128
<211>  21
<212>  DNA/RNA
<213>  Artificially synthesized

<400>  128
cuggaguucg agacgaggct t                                                21

<210>  129
<211>  21
<212>  DNA/RNA
<213>  Artificially synthesized

<400>  129
uucgagacga ggccuaagct t                                                21

<210>  130
<211>  21
<212>  DNA/RNA
<213>  Artificially synthesized

<400>  130
gagacgaggc cuaagcaact t                                                21

<210>  131
<211>  21
<212>  DNA/RNA
<213>  Artificially synthesized

<400>  131
agacgaggcc uaagcaacat t                                                21

<210>  132
<211>  21
<212>  DNA/RNA
<213>  Artificially synthesized

<400>  132

gacgaggccu aagcaacaut t                                                21

<210>  133
<211>  21
<212>  DNA/RNA
<213>  Artificially synthesized

<400>  133
ccuaagcaac augccgaaat t                                                21

<210>  134
<211>  21
<212>  DNA/RNA
<213>  Artificially synthesized

<400>  134
cuaagcaaca ugccgaaact t                                                21

<210>  135
<211>  21
<212>  DNA/RNA
<213>  Artificially synthesized

<400>  135
uaagcaacau gccgaaacct t                                                21

<210>  136
<211>  21
<212>  DNA/RNA
<213>  Artificially synthesized

<400>  136
ugguggcgca cgccuauagt t                                                21

<210>  137
<211>  21
<212>  DNA/RNA
<213>  Artificially synthesized

<400>  137
gguggcgcac gccuauagut t                                                21

<210>  138
<211>  21
<212>  DNA/RNA
<213>  Artificially synthesized

<400>  138
cuauaguccu agcuacuggt t                                                21

<210>  139
<211>  21
<212>  DNA/RNA
<213>  Artificially synthesized

<400>  139

uauaguccua gcuacugggt t                                              21


<210>  140
<211>  21
<212>  DNA/RNA
<213>  Artificially synthesized

<400>  140
ugagguggga ggaucgcuut t                                              21


<210>  141
<211>  21
<212>  DNA/RNA
<213>  Artificially synthesized

<400>  141
cugcagugag ccgagaucgt t                                              21


<210>  142
<211>  21
<212>  DNA/RNA
<213>  Artificially synthesized

<400>  142
ugcagugagc cgagaucgct t                                              21


<210>  143
<211>  21
<212>  DNA/RNA
<213>  Artificially synthesized

<400>  143
ugcacuccag ccugagcgat t                                              21


<210>  144
<211>  21
<212>  DNA/RNA
<213>  Artificially synthesized

<400>  144
cacuccagcc ugagcgacat t                                              21


<210>  145
<211>  21
<212>  DNA/RNA
<213>  Artificially synthesized

<400>  145
acagggcgag gcucugucut t                                              21


<210>  146
<211>  21
<212>  DNA/RNA
<213>  Artificially synthesized

<400>  146

gggcgaggcu cugucucaat t                                                21

<210>  147
<211>  21
<212>  DNA/RNA
<213>  Artificially synthesized

<400>  147
ggcgaggcuc ugucucaaat t                                                21

<210>  148
<211>  21
<212>  DNA/RNA
<213>  Artificially synthesized

<400>  148
gcgaggcucu gucucaaaat t                                                21

<210>  149
<211>  21
<212>  DNA/RNA
<213>  Artificially synthesized

<400>  149
aggcucuguc ucaaaacaat t                                                21

<210>  150
<211>  21
<212>  DNA/RNA
<213>  Artificially synthesized

<400>  150
ggcucugucu caaaacaaat t                                                21

<210>  151
<211>  21
<212>  DNA/RNA
<213>  Artificially synthesized

<400>  151
gcucugucuc aaaacaaact t                                                21

<210>  152
<211>  21
<212>  DNA/RNA
<213>  Artificially synthesized

<400>  152
cucugucuca aaacaaacat t                                                21

<210>  153
<211>  21
<212>  DNA/RNA
<213>  Artificially synthesized

<400>  153

aacacaguga aaugaaaggt t                                              21

<210>  154
<211>  21
<212>  DNA/RNA
<213>  Artificially synthesized

<400>  154
acacagugaa augaaaggat t                                              21

<210>  155
<211>  21
<212>  DNA/RNA
<213>  Artificially synthesized

<400>  155
cagugaaaug aaaggauugt t                                              21

<210>  156
<211>  21
<212>  DNA/RNA
<213>  Artificially synthesized

<400>  156
gugaaaugaa aggauugagt t                                              21

<210>  157
<211>  21
<212>  DNA/RNA
<213>  Artificially synthesized

<400>  157
gaaaugaaag gauugagagt t                                              21

<210>  158
<211>  21
<212>  DNA/RNA
<213>  Artificially synthesized

<400>  158
aaagguacau cucacagaut t                                              21

<210>  159
<211>  21
<212>  DNA/RNA
<213>  Artificially synthesized

<400>  159
cagccugagu gacagagugt t                                              21

<210>  160
<211>  21
<212>  DNA/RNA
<213>  Artificially synthesized

<400>  160

ccagccugag ugacagagut t                                                21

<210>  161
<211>  21
<212>  DNA/RNA
<213>  Artificially synthesized

<400>  161
cuccagccug agugacagat t                                                21

<210>  162
<211>  21
<212>  DNA/RNA
<213>  Artificially synthesized

<400>  162
ucaggcugga gugcaguggt t                                                21

<210>  163
<211>  21
<212>  DNA/RNA
<213>  Artificially synthesized

<400>  163
agaucacgcc acugcacuct t                                                21

<210>  164
<211>  21
<212>  DNA/RNA
<213>  Artificially synthesized

<400>  164
ugcaguggcg ugaucuuggt t                                                21

<210>  165
<211>  21
<212>  DNA/RNA
<213>  Artificially synthesized

<400>  165
gccaagauca cgccacugct t                                                21

<210>  166
<211>  21
<212>  DNA/RNA
<213>  Artificially synthesized

<400>  166
agugagccaa gaucacgcct t                                                21

<210>  167
<211>  21
<212>  DNA/RNA
<213>  Artificially synthesized

<400>  167

ugcagugagc caagaucact t                                                21

<210>  168
<211>  21
<212>  DNA/RNA
<213>  Artificially synthesized

<400>  168
uugcagugag ccaagaucat t                                                21

<210>  169
<211>  21
<212>  DNA/RNA
<213>  Artificially synthesized

<400>  169
gguugcagug agccaagaut t                                                21

<210>  170
<211>  21
<212>  DNA/RNA
<213>  Artificially synthesized

<400>  170
gagguugcag ugagccaagt t                                                21

<210>  171
<211>  21
<212>  DNA/RNA
<213>  Artificially synthesized

<400>  171
ggagguugca gugagccaat t                                                21

<210>  172
<211>  21
<212>  DNA/RNA
<213>  Artificially synthesized

<400>  172
ucacuugaac ucgggaggct t                                                21

<210>  173
<211>  21
<212>  DNA/RNA
<213>  Artificially synthesized

<400>  173
aucacuugaa cucgggaggt t                                                21

<210>  174
<211>  21
<212>  DNA/RNA
<213>  Artificially synthesized

<400>  174

aaucacuuga acucgggagt t                                                21

<210>  175
<211>  21
<212>  DNA/RNA
<213>  Artificially synthesized

<400>  175
gaaucacuug aacucgggat t                                                21

<210>  176
<211>  21
<212>  DNA/RNA
<213>  Artificially synthesized

<400>  176
agccaggaga aucacuugat t                                                21

<210>  177
<211>  21
<212>  DNA/RNA
<213>  Artificially synthesized

<400>  177
ugagccagga gaaucacuut t                                                21

<210>  178
<211>  21
<212>  DNA/RNA
<213>  Artificially synthesized

<400>  178
cugagccagg agaaucacut t                                                21

<210>  179
<211>  21
<212>  DNA/RNA
<213>  Artificially synthesized

<400>  179
gcugagccag gagaaucact t                                                21

<210>  180
<211>  21
<212>  DNA/RNA
<213>  Artificially synthesized

<400>  180
acagcugcuu gggaggcugt t                                                21

<210>  181
<211>  21
<212>  DNA/RNA
<213>  Artificially synthesized

<400>  181

augacagcug cuugggaggt t                                              21

<210>  182
<211>  21
<212>  DNA/RNA
<213>  Artificially synthesized

<400>  182
uaaugacagc ugcuugggat t                                              21

<210>  183
<211>  21
<212>  DNA/RNA
<213>  Artificially synthesized

<400>  183
caggccugua augacagcut t                                              21

<210>  184
<211>  21
<212>  DNA/RNA
<213>  Artificially synthesized

<400>  184
gcaggccugu aaugacagct t                                              21

<210>  185
<211>  21
<212>  DNA/RNA
<213>  Artificially synthesized

<400>  185
ugcaggccug uaaugacagt t                                              21

<210>  186
<211>  21
<212>  DNA/RNA
<213>  Artificially synthesized

<400>  186
ggugaaaccc uguuucucct t                                              21

<210>  187
<211>  21
<212>  DNA/RNA
<213>  Artificially synthesized

<400>  187
auggugaaac ccuguuucut t                                              21

<210>  188
<211>  21
<212>  DNA/RNA
<213>  Artificially synthesized

<400>  188

gccuauaauc ccagcacuut t                                              21

<210>  189
<211>  21
<212>  DNA/RNA
<213>  Artificially synthesized

<400>  189
cgguggcuca ugccuauaat t                                              21

<210>  190
<211>  21
<212>  DNA/RNA
<213>  Artificially synthesized

<400>  190
uguggaggaa ggggagaaut t                                              21

<210>  191
<211>  21
<212>  DNA/RNA
<213>  Artificially synthesized

<400>  191
ucuguggagg aaggggagat t                                              21

<210>  192
<211>  21
<212>  DNA/RNA
<213>  Artificially synthesized

<400>  192
caucuagggu ugcuaaaugt t                                              21

<210>  193
<211>  21
<212>  DNA/RNA
<213>  Artificially synthesized

<400>  193
gcaucuaggg uugcuaaaut t                                              21

<210>  194
<211>  21
<212>  DNA/RNA
<213>  Artificially synthesized

<400>  194
agcaucuagg guugcuaaat t                                              21

<210>  195
<211>  21
<212>  DNA/RNA
<213>  Artificially synthesized

<400>  195

aagcaucuag gguugcuaat t                                                21

<210>  196
<211>  21
<212>  DNA/RNA
<213>  Artificially synthesized

<400>  196
uaagcaucua ggguugcuat t                                                21

<210>  197
<211>  21
<212>  DNA/RNA
<213>  Artificially synthesized

<400>  197
cacaccgggc cuccaguaut t                                                21

<210>  198
<211>  21
<212>  DNA/RNA
<213>  Artificially synthesized

<400>  198
caggugugag ccaccacact t                                                21

<210>  199
<211>  21
<212>  DNA/RNA
<213>  Artificially synthesized

<400>  199
uuacaggugu gagccaccat t                                                21

<210>  200
<211>  21
<212>  DNA/RNA
<213>  Artificially synthesized

<400>  200
auuacaggug ugagccacct t                                                21

<210>  201
<211>  21
<212>  DNA/RNA
<213>  Artificially synthesized

<400>  201
gauuacaggu gugagccact t                                                21

<210>  202
<211>  21
<212>  DNA/RNA
<213>  Artificially synthesized

<400>  202

gggauuacag gugugagcct t                                              21


<210>  203
<211>  21
<212>  DNA/RNA
<213>  Artificially synthesized

<400>  203
ugcugggauu acaggugugt t                                              21


<210>  204
<211>  21
<212>  DNA/RNA
<213>  Artificially synthesized

<400>  204
gugcugggau uacaggugut t                                              21


<210>  205
<211>  21
<212>  DNA/RNA
<213>  Artificially synthesized

<400>  205
aagugcuggg auuacaggut t                                              21


<210>  206
<211>  21
<212>  DNA/RNA
<213>  Artificially synthesized

<400>  206
cccaaagugc ugggauuact t                                              21


<210>  207
<211>  21
<212>  DNA/RNA
<213>  Artificially synthesized

<400>  207
ucccaaagug cugggauuat t                                              21


<210>  208
<211>  21
<212>  DNA/RNA
<213>  Artificially synthesized

<400>  208
cucccaaagu gcugggauut t                                              21


<210>  209
<211>  21
<212>  DNA/RNA
<213>  Artificially synthesized

<400>  209

ucggccuccc aaagugcugt t                                                21

<210>  210
<211>  21
<212>  DNA/RNA
<213>  Artificially synthesized

<400>  210
cucguaaucc gaccgccuct t                                                21

<210>  211
<211>  21
<212>  DNA/RNA
<213>  Artificially synthesized

<400>  211
accucguaau ccgaccgcct t                                                21

<210>  212
<211>  21
<212>  DNA/RNA
<213>  Artificially synthesized

<400>  212
gaccucguaa uccgaccgct t                                                21

<210>  213
<211>  21
<212>  DNA/RNA
<213>  Artificially synthesized

<400>  213
ugaccucgua auccgaccgt t                                                21

<210>  214
<211>  21
<212>  DNA/RNA
<213>  Artificially synthesized

<400>  214
cugaccucgu aauccgacct t                                                21

<210>  215
<211>  21
<212>  DNA/RNA
<213>  Artificially synthesized

<400>  215
ccugaccucg uaauccgact t                                                21

<210>  216
<211>  21
<212>  DNA/RNA
<213>  Artificially synthesized

<400>  216

uccugaccuc guaauccgat t                                                21

<210>  217
<211>  21
<212>  DNA/RNA
<213>  Artificially synthesized

<400>  217
cuccugaccu cguaauccgt t                                                21

<210>  218
<211>  21
<212>  DNA/RNA
<213>  Artificially synthesized

<400>  218
gaacuccuga ccucguaaut t                                                21

<210>  219
<211>  21
<212>  DNA/RNA
<213>  Artificially synthesized

<400>  219
ugaacuccug accucguaat t                                                21

<210>  220
<211>  21
<212>  DNA/RNA
<213>  Artificially synthesized

<400>  220
uugaacuccu gaccucguat t                                                21

<210>  221
<211>  21
<212>  DNA/RNA
<213>  Artificially synthesized

<400>  221
cuugaacucc ugaccucgut t                                                21

<210>  222
<211>  21
<212>  DNA/RNA
<213>  Artificially synthesized

<400>  222
ggucuugaac uccugaccut t                                                21

<210>  223
<211>  21
<212>  DNA/RNA
<213>  Artificially synthesized

<400>  223

gccaggcugg ucuugaacut t                                                21

<210>  224
<211>  21
<212>  DNA/RNA
<213>  Artificially synthesized

<400>  224
   uuaguaaaga ugggguuuct t                                                 21

<210>  225
<211>  21
<212>  DNA/RNA
<213>  Artificially synthesized

<400>  225
accaccacac ccggcuaaut t                                                21

<210>  226
<211>  21
<212>  DNA/RNA
<213>  Artificially synthesized

<400>  226
gauuacaggc gcccaccact t                                                21

<210>  227
<211>  21
<212>  DNA/RNA
<213>  Artificially synthesized

<400>  227
agcugggauu acaggcgcct t                                                21

<210>  228
<211>  21
<212>  DNA/RNA
<213>  Artificially synthesized

<400>  228
ccccgaguag cugggauuat t                                                21

<210>  229
<211>  21
<212>  DNA/RNA
<213>  Artificially synthesized

<400>  229
agcaauucug ccucagccct t                                                21

<210>  230
<211>  21
<212>  DNA/RNA
<213>  Artificially synthesized

<400>  230

agguucaagc aauucugcct t                                                21

<210>  231
<211>  21
<212>  DNA/RNA
<213>  Artificially synthesized

<400>  231
ccagguucaa gcaauucugt t                                                21

<210>  232
<211>  21
<212>  DNA/RNA
<213>  Artificially synthesized

<400>  232
ccucugccuc ccagguucat t                                                21

<210>  233
<211>  21
<212>  DNA/RNA
<213>  Artificially synthesized

<400>  233
accucugccu cccagguuct t                                                21

<210>  234
<211>  21
<212>  DNA/RNA
<213>  Artificially synthesized

<400>  234
caaccucugc cucccaggut t                                                21

<210>  235
<211>  21
<212>  DNA/RNA
<213>  Artificially synthesized

<400>  235
gugaucucag cucacugcat t                                                21

<210>  236
<211>  21
<212>  DNA/RNA
<213>  Artificially synthesized

<400>  236
ugcaguggcg ugaucucagt t                                                21

<210>  237
<211>  21
<212>  DNA/RNA
<213>  Artificially synthesized

<400>  237

uggaaugcag uggcgugaut t                                                21

<210>  238
<211>  21
<212>  DNA/RNA
<213>  Artificially synthesized

<400>  238
gaguauugcu cugucaccct t                                                21

<210>  239
<211>  21
<212>  DNA/RNA
<213>  Artificially synthesized

<400>  239
acagaguauu gcucugucat t                                                21

<210>  240
<211>  21
<212>  DNA/RNA
<213>  Artificially synthesized

<400>  240
ugcgacagag uauugcucut t                                                21

<210>  241
<211>  21
<212>  DNA/RNA
<213>  Artificially synthesized

<400>  241
cagccuccag uauucuuuut t                                                21

<210>  242
<211>  21
<212>  DNA/RNA
<213>  Artificially synthesized

<400>  242
ccagccucca guauucuuut t                                                21

<210>  243
<211>  21
<212>  DNA/RNA
<213>  Artificially synthesized

<400>  243
cucgcccagc cuccaguaut t                                                21

<210>  244
<211>  21
<212>  DNA/RNA
<213>  Artificially synthesized

<400>  244

acagguguga gccaccucgt t                                                21

<210>  245
<211>  21
<212>  DNA/RNA
<213>  Artificially synthesized

<400>  245
auuacaggug ugagccacct t                                                21

<210>  246
<211>  21
<212>  DNA/RNA
<213>  Artificially synthesized

<400>  246
gggauuacag gugugagcct t                                                21

<210>  247
<211>  21
<212>  DNA/RNA
<213>  Artificially synthesized

<400>  247
ugggauuaca ggugugagct t                                                21

<210>  248
<211>  21
<212>  DNA/RNA
<213>  Artificially synthesized

<400>  248
ugcugggauu acaggugugt t                                                21

<210>  249
<211>  21
<212>  DNA/RNA
<213>  Artificially synthesized

<400>  249
ucccaaaaug cugggauuat t                                                21

<210>  250
<211>  21
<212>  DNA/RNA
<213>  Artificially synthesized

<400>  250
agcucaagau auuccgccct t                                                21

<210>  251
<211>  21
<212>  DNA/RNA
<213>  Artificially synthesized

<400>  251

cuccugagcu caagauauut t                                                21

<210>  252
<211>  21
<212>  DNA/RNA
<213>  Artificially synthesized

<400>  252
uguguaggcu ggucucgaat t                                                21

<210>  253
<211>  21
<212>  DNA/RNA
<213>  Artificially synthesized

<400>  253
agcauauugu guaggcuggt t                                                21

<210>  254
<211>  21
<212>  DNA/RNA
<213>  Artificially synthesized

<400>  254
cguuuggagc auauugugut t                                                21

<210>  255
<211>  21
<212>  DNA/RNA
<213>  Artificially synthesized

<400>  255
gcguuuggag cauauugugt t                                                21

<210>  256
<211>  21
<212>  DNA/RNA
<213>  Artificially synthesized

<400>  256
ggcguuugga gcauauugut t                                                21

<210>  257
<211>  21
<212>  DNA/RNA
<213>  Artificially synthesized

<400>  257
uuuguagagg cggcguuugt t                                                21

<210>  258
<211>  21
<212>  DNA/RNA
<213>  Artificially synthesized

<400>  258

caaguagcua ggaccacagt t                                                21

<210>  259
<211>  21
<212>  DNA/RNA
<213>  Artificially synthesized

<400>  259
gagcucaagc gauccuccct t                                                21

<210>  260
<211>  21
<212>  DNA/RNA
<213>  Artificially synthesized

<400>  260
ccgagcucaa gcgauccuct t                                                21

<210>  261
<211>  21
<212>  DNA/RNA
<213>  Artificially synthesized

<400>  261
cauugcagcc ucgaccucct t                                                21

<210>  262
<211>  21
<212>  DNA/RNA
<213>  Artificially synthesized

<400>  262
gcucauugca gccucgacct t                                                21

<210>  263
<211>  21
<212>  DNA/RNA
<213>  Artificially synthesized

<400>  263
gcaccaucuc ggcucauugt t                                                21

<210>  264
<211>  21
<212>  DNA/RNA
<213>  Artificially synthesized

<400>  264
ggcaccaucu cggcucauut t                                                21

<210>  265
<211>  21
<212>  DNA/RNA
<213>  Artificially synthesized

<400>  265

ugcaguggca ccaucucggt t                                                21

<210>  266
<211>  21
<212>  DNA/RNA
<213>  Artificially synthesized

<400>  266
agugcagugg caccaucuct t                                                21

<210>  267
<211>  21
<212>  DNA/RNA
<213>  Artificially synthesized

<400>  267
gagugcagug gcaccaucut t                                                21

<210>  268
<211>  21
<212>  DNA/RNA
<213>  Artificially synthesized

<400>  268
cagagugcag uggcaccaut t                                                21

<210>  269
<211>  21
<212>  DNA/RNA
<213>  Artificially synthesized

<400>  269
ugucgucaga gugcaguggt t                                                21

<210>  270
<211>  21
<212>  DNA/RNA
<213>  Artificially synthesized

<400>  270
cugucgucag agugcagugt t                                                21

<210>  271
<211>  21
<212>  DNA/RNA
<213>  Artificially synthesized

<400>  271
gucucgcucu gucgucagat t                                                21

<210>  272
<211>  21
<212>  DNA/RNA
<213>  Artificially synthesized

<400>  272

agucucgcuc ugucgucagt t                                                21

<210>  273
<211>  21
<212>  DNA/RNA
<213>  Artificially synthesized

<400>  273
uguuuugaga cggagucuct t                                                21

<210>  274
<211>  21
<212>  DNA/RNA
<213>  Artificially synthesized

<400>  274
uuguuuugag acggagucut t                                                21

<210>  275
<211>  21
<212>  DNA/RNA
<213>  Artificially synthesized

<400>  275
cagauccuua aacacuagat t                                                21

<210>  276
<211>  21
<212>  DNA/RNA
<213>  Artificially synthesized

<400>  276
ggcagauccu uaaacacuat t                                                21

<210>  277
<211>  21
<212>  DNA/RNA
<213>  Artificially synthesized

<400>  277
gaaggcagau ccuuaaacat t                                                21

<210>  278
<211>  21
<212>  DNA/RNA
<213>  Artificially synthesized

<400>  278
caggaaggaa ggcagaucct t                                                21

<210>  279
<211>  21
<212>  DNA/RNA
<213>  Artificially synthesized

<400>  279

gacaaacaag gaaagacaat t                                              21


<210>  280
<211>  21
<212>  DNA/RNA
<213>  Artificially synthesized

<400>  280
aagacaaaca aggaaagact t                                              21


<210>  281
<211>  21
<212>  DNA/RNA
<213>  Artificially synthesized

<400>  281
ggaauuuaaa accugcuugt t                                              21


<210>  282
<211>  21
<212>  DNA/RNA
<213>  Artificially synthesized

<400>  282
cuaggaauuu aaaaccugct t                                              21


<210>  283
<211>  21
<212>  DNA/RNA
<213>  Artificially synthesized

<400>  283
cccuuggaaa aguaaaugut t                                              21


<210>  284
<211>  21
<212>  DNA/RNA
<213>  Artificially synthesized

<400>  284
ucgucucgaa cuccagugut t                                              21


<210>  285
<211>  21
<212>  DNA/RNA
<213>  Artificially synthesized

<400>  285
gccucgucuc gaacuccagt t                                              21


<210>  286
<211>  21
<212>  DNA/RNA
<213>  Artificially synthesized

<400>  286

gcuuaggccu cgucucgaat t                                                21

<210>  287
<211>  21
<212>  DNA/RNA
<213>  Artificially synthesized

<400>  287
guugcuuagg ccucgucuct t                                                21

<210>  288
<211>  21
<212>  DNA/RNA
<213>  Artificially synthesized

<400>  288
uguugcuuag gccucgucut t                                                21

<210>  289
<211>  21
<212>  DNA/RNA
<213>  Artificially synthesized

<400>  289
auguugcuua ggccucguct t                                                21

<210>  290
<211>  21
<212>  DNA/RNA
<213>  Artificially synthesized

<400>  290
uuucggcaug uugcuuaggt t                                                21

<210>  291
<211>  21
<212>  DNA/RNA
<213>  Artificially synthesized

<400>  291
guuucggcau guugcuuagt t                                                21

<210>  292
<211>  21
<212>  DNA/RNA
<213>  Artificially synthesized

<400>  292
gguuucggca uguugcuuat t                                                21

<210>  293
<211>  21
<212>  DNA/RNA
<213>  Artificially synthesized

<400>  293

cuauaggcgu gcgccaccat t                                                21

<210>  294
<211>  21
<212>  DNA/RNA
<213>  Artificially synthesized

<400>  294
acuauaggcg ugcgccacct t                                                21

<210>  295
<211>  21
<212>  DNA/RNA
<213>  Artificially synthesized

<400>  295
ccaguagcua ggacuauagt t                                                21

<210>  296
<211>  21
<212>  DNA/RNA
<213>  Artificially synthesized

<400>  296
cccaguagcu aggacuauat t                                                21

<210>  297
<211>  21
<212>  DNA/RNA
<213>  Artificially synthesized

<400>  297
aagcgauccu cccaccucat t                                                21

<210>  298
<211>  21
<212>  DNA/RNA
<213>  Artificially synthesized

<400>  298
cgaucucggc ucacugcagt t                                                21

<210>  299
<211>  21
<212>  DNA/RNA
<213>  Artificially synthesized

<400>  299
gcgaucucgg cucacugcat t                                                21

<210>  300
<211>  21
<212>  DNA/RNA
<213>  Artificially synthesized

<400>  300

ucgcucaggc uggagugcat t                                                21

<210>  301
<211>  21
<212>  DNA/RNA
<213>  Artificially synthesized

<400>  301
ugucgcucag gcuggagugt t                                                21

<210>  302
<211>  21
<212>  DNA/RNA
<213>  Artificially synthesized

<400>  302
agacagagcc ucgcccugut t                                                21

<210>  303
<211>  21
<212>  DNA/RNA
<213>  Artificially synthesized

<400>  303
uugagacaga gccucgccct t                                                21

<210>  304
<211>  21
<212>  DNA/RNA
<213>  Artificially synthesized

<400>  304
uuugagacag agccucgcct t                                                21

<210>  305
<211>  21
<212>  DNA/RNA
<213>  Artificially synthesized

<400>  305
uuuugagaca gagccucgct t                                                21

<210>  306
<211>  21
<212>  DNA/RNA
<213>  Artificially synthesized

<400>  306
uuguuuugag acagagccut t                                                21

<210>  307
<211>  21
<212>  DNA/RNA
<213>  Artificially synthesized

<400>  307

uuuguuuuga gacagagcct t                                                21

<210>  308
<211>  21
<212>  DNA/RNA
<213>  Artificially synthesized

<400>  308
guuuguuuug agacagagct t                                                21

<210>  309
<211>  21
<212>  DNA/RNA
<213>  Artificially synthesized

<400>  309
uguuuguuuu gagacagagt t                                                21

<210>  310
<211>  21
<212>  DNA/RNA
<213>  Artificially synthesized

<400>  310
ccuuucauuu cacuguguut t                                                21

<210>  311
<211>  21
<212>  DNA/RNA
<213>  Artificially synthesized

<400>  311
uccuuucauu ucacugugut t                                                21

<210>  312
<211>  21
<212>  DNA/RNA
<213>  Artificially synthesized

<400>  312
caauccuuuc auuucacugt t                                                21

<210>  313
<211>  21
<212>  DNA/RNA
<213>  Artificially synthesized

<400>  313
cucaauccuu ucauuucact t                                                21

<210>  314
<211>  21
<212>  DNA/RNA
<213>  Artificially synthesized

<400>  314

cucucaaucc uuucauuuct t                                                21

<210>  315
<211>  19
<212>  DNA
<213>  Artificially synthesized

<400>  315
atctgtgaga tgtaccttt                                                   19

<210>  316
<211>  19
<212>  DNA
<213>  Artificially synthesized

<400>  316
cactctgtca ctcaggctg                                                   19

<210>  317
<211>  19
<212>  DNA
<213>  Artificially synthesized

<400>  317
actctgtcac tcaggctgg                                                   19

<210>  318
<211>  19
<212>  DNA
<213>  Artificially synthesized

<400>  318
tctgtcactc aggctggag                                                   19

<210>  319
<211>  19
<212>  DNA
<213>  Artificially synthesized

<400>  319
tcaggctgga gtgcagtgg                                                   19

<210>  320
<211>  19
<212>  DNA
<213>  Artificially synthesized

<400>  320
gagtgcagtg gcgtgatct                                                   19

<210>  321
<211>  19
<212>  DNA
<213>  Artificially synthesized

<400>  321

tgcagtggcg tgatcttgg                                                    19

<210>  322
<211>  19
<212>  DNA
<213>  Artificially synthesized

<400>  322
gcagtggcgt gatcttggc                                                    19

<210>  323
<211>  19
<212>  DNA
<213>  Artificially synthesized

<400>  323
ggcgtgatct tggctcact                                                    19

<210>  324
<211>  19
<212>  DNA
<213>  Artificially synthesized

<400>  324
gtgatcttgg ctcactgca                                                    19

<210>  325
<211>  19
<212>  DNA
<213>  Artificially synthesized

<400>  325
tgatcttggc tcactgcaa                                                    19

<210>  326
<211>  19
<212>  DNA
<213>  Artificially synthesized

<400>  326
atcttggctc actgcaacc                                                    19

<210>  327
<211>  19
<212>  DNA
<213>  Artificially synthesized

<400>  327
cttggctcac tgcaacctc                                                    19

<210>  328
<211>  19
<212>  DNA
<213>  Artificially synthesized

<400>  328

ttggctcact gcaacctcc                                                    19


<210>  329
<211>  19
<212>  DNA
<213>  Artificially synthesized

<400>  329
gcctcccgag ttcaagtga                                                    19


<210>  330
<211>  19
<212>  DNA
<213>  Artificially synthesized

<400>  330
cctcccgagt tcaagtgat                                                    19


<210>  331
<211>  19
<212>  DNA
<213>  Artificially synthesized

<400>  331
ctcccgagtt caagtgatt                                                    19


<210>  332
<211>  19
<212>  DNA
<213>  Artificially synthesized

<400>  332
tcccgagttc aagtgattc                                                    19


<210>  333
<211>  19
<212>  DNA
<213>  Artificially synthesized

<400>  333
tcaagtgatt ctcctggct                                                    19


<210>  334
<211>  19
<212>  DNA
<213>  Artificially synthesized

<400>  334
aagtgattct cctggctca                                                    19


<210>  335
<211>  19
<212>  DNA
<213>  Artificially synthesized

<400>  335

agtgattctc ctggctcag                                                    19

<210>  336
<211>  19
<212>  DNA
<213>  Artificially synthesized

<400>  336
gtgattctcc tggctcagc                                                    19

<210>  337
<211>  19
<212>  DNA
<213>  Artificially synthesized

<400>  337
cagcctccca agcagctgt                                                    19

<210>  338
<211>  19
<212>  DNA
<213>  Artificially synthesized

<400>  338
cctcccaagc agctgtcat                                                    19

<210>  339
<211>  19
<212>  DNA
<213>  Artificially synthesized

<400>  339
tcccaagcag ctgtcatta                                                    19

<210>  340
<211>  19
<212>  DNA
<213>  Artificially synthesized

<400>  340
agctgtcatt acaggcctg                                                    19

<210>  341
<211>  19
<212>  DNA
<213>  Artificially synthesized

<400>  341
gctgtcatta caggcctgc                                                    19

<210>  342
<211>  19
<212>  DNA
<213>  Artificially synthesized

<400>  342

ctgtcattac aggcctgca                                                    19

<210>  343
<211>  19
<212>  DNA
<213>  Artificially synthesized

<400>  343
ggagaaacag ggtttcacc                                                    19

<210>  344
<211>  19
<212>  DNA
<213>  Artificially synthesized

<400>  344
agaaacaggg tttcaccat                                                    19

<210>  345
<211>  19
<212>  DNA
<213>  Artificially synthesized

<400>  345
aagtgctggg attataggc                                                    19

<210>  346
<211>  19
<212>  DNA
<213>  Artificially synthesized

<400>  346
ttataggcat gagccaccg                                                    19

<210>  347
<211>  19
<212>  DNA
<213>  Artificially synthesized

<400>  347
attctcccct tcctccaca                                                    19

<210>  348
<211>  19
<212>  DNA
<213>  Artificially synthesized

<400>  348
tctccccttc ctccacaga                                                    19

<210>  349
<211>  19
<212>  DNA
<213>  Artificially synthesized

<400>  349

catttagcaa ccctagatg                                                    19

<210>  350
<211>  19
<212>  DNA
<213>  Artificially synthesized

<400>  350
atttagcaac cctagatgc                                                    19

<210>  351
<211>  19
<212>  DNA
<213>  Artificially synthesized

<400>  351
tttagcaacc ctagatgct                                                    19

<210>  352
<211>  19
<212>  DNA
<213>  Artificially synthesized

<400>  352
ttagcaaccc tagatgctt                                                    19

<210>  353
<211>  19
<212>  DNA
<213>  Artificially synthesized

<400>  353
tagcaaccct agatgctta                                                    19

<210>  354
<211>  19
<212>  DNA
<213>  Artificially synthesized

<400>  354
atactggagg cccggtgtg                                                    19

<210>  355
<211>  19
<212>  DNA
<213>  Artificially synthesized

<400>  355
gtgtggtggc tcacacctg                                                    19

<210>  356
<211>  19
<212>  DNA
<213>  Artificially synthesized

<400>  356

tggtggctca cacctgtaa                                                    19

<210>  357
<211>  19
<212>  DNA
<213>  Artificially synthesized

<400>  357
ggtggctcac acctgtaat                                                    19

<210>  358
<211>  19
<212>  DNA
<213>  Artificially synthesized

<400>  358
gtggctcaca cctgtaatc                                                    19

<210>  359
<211>  19
<212>  DNA
<213>  Artificially synthesized

<400>  359
ggctcacacc tgtaatccc                                                    19

<210>  360
<211>  19
<212>  DNA
<213>  Artificially synthesized

<400>  360
cacacctgta atcccagca                                                    19

<210>  361
<211>  19
<212>  DNA
<213>  Artificially synthesized

<400>  361
acacctgtaa tcccagcac                                                    19

<210>  362
<211>  19
<212>  DNA
<213>  Artificially synthesized

<400>  362
acctgtaatc ccagcactt                                                    19

<210>  363
<211>  19
<212>  DNA
<213>  Artificially synthesized

<400>  363

gtaatcccag cactttggg                                                    19

<210>  364
<211>  19
<212>  DNA
<213>  Artificially synthesized

<400>  364
taatcccagc actttggga                                                    19

<210>  365
<211>  19
<212>  DNA
<213>  Artificially synthesized

<400>  365
aatcccagca ctttgggag                                                    19

<210>  366
<211>  19
<212>  DNA
<213>  Artificially synthesized

<400>  366
cagcactttg ggaggccga                                                    19

<210>  367
<211>  19
<212>  DNA
<213>  Artificially synthesized

<400>  367
gaggcggtcg gattacgag                                                    19

<210>  368
<211>  19
<212>  DNA
<213>  Artificially synthesized

<400>  368
ggcggtcgga ttacgaggt                                                    19

<210>  369
<211>  19
<212>  DNA
<213>  Artificially synthesized

<400>  369
gcggtcggat tacgaggtc                                                    19

<210>  370
<211>  19
<212>  DNA
<213>  Artificially synthesized

<400>  370

cggtcggatt acgaggtca                                                    19

<210>  371
<211>  19
<212>  DNA
<213>  Artificially synthesized

<400>  371
ggtcggatta cgaggtcag                                                    19

<210>  372
<211>  19
<212>  DNA
<213>  Artificially synthesized

<400>  372
gtcggattac gaggtcagg                                                    19

<210>  373
<211>  19
<212>  DNA
<213>  Artificially synthesized

<400>  373
tcggattacg aggtcagga                                                    19

<210>  374
<211>  19
<212>  DNA
<213>  Artificially synthesized

<400>  374
cggattacga ggtcaggag                                                    19

<210>  375
<211>  19
<212>  DNA
<213>  Artificially synthesized

<400>  375
attacgaggt caggagttc                                                    19

<210>  376
<211>  19
<212>  DNA
<213>  Artificially synthesized

<400>  376
ttacgaggtc aggagttca                                                    19

<210>  377
<211>  19
<212>  DNA
<213>  Artificially synthesized

<400>  377

tacgaggtca ggagttcaa                                                    19

<210>  378
<211>  19
<212>  DNA
<213>  Artificially synthesized

<400>  378
acgaggtcag gagttcaag                                                    19

<210>  379
<211>  19
<212>  DNA
<213>  Artificially synthesized

<400>  379
aggtcaggag ttcaagacc                                                    19

<210>  380
<211>  19
<212>  DNA
<213>  Artificially synthesized

<400>  380
agttcaagac cagcctggc                                                    19

<210>  381
<211>  19
<212>  DNA
<213>  Artificially synthesized

<400>  381
gaaaccccat ctttactaa                                                    19

<210>  382
<211>  19
<212>  DNA
<213>  Artificially synthesized

<400>  382
attagccggg tgtggtggt                                                    19

<210>  383
<211>  19
<212>  DNA
<213>  Artificially synthesized

<400>  383
gtggtgggcg cctgtaatc                                                    19

<210>  384
<211>  19
<212>  DNA
<213>  Artificially synthesized

<400>  384

ggcgcctgta atcccagct                                                    19


<210>  385
<211>  19
<212>  DNA
<213>  Artificially synthesized

<400>  385
taatcccagc tactcgggg                                                    19


<210>  386
<211>  19
<212>  DNA
<213>  Artificially synthesized

<400>  386
gggctgaggc agaattgct                                                    19


<210>  387
<211>  19
<212>  DNA
<213>  Artificially synthesized

<400>  387
ggcagaattg cttgaacct                                                    19


<210>  388
<211>  19
<212>  DNA
<213>  Artificially synthesized

<400>  388
cagaattgct tgaacctgg                                                    19


<210>  389
<211>  19
<212>  DNA
<213>  Artificially synthesized

<400>  389
tgaacctggg aggcagagg                                                    19


<210>  390
<211>  19
<212>  DNA
<213>  Artificially synthesized

<400>  390
gaacctggga ggcagaggt                                                    19


<210>  391
<211>  19
<212>  DNA
<213>  Artificially synthesized

<400>  391

acctgggagg cagaggttg                                                    19

<210>  392
<211>  19
<212>  DNA
<213>  Artificially synthesized

<400>  392
tgcagtgagc tgagatcac                                                    19

<210>  393
<211>  19
<212>  DNA
<213>  Artificially synthesized

<400>  393
ctgagatcac gccactgca                                                    19

<210>  394
<211>  19
<212>  DNA
<213>  Artificially synthesized

<400>  394
atcacgccac tgcattcca                                                    19

<210>  395
<211>  19
<212>  DNA
<213>  Artificially synthesized

<400>  395
gggtgacaga gcaatactc                                                    19

<210>  396
<211>  19
<212>  DNA
<213>  Artificially synthesized

<400>  396
tgacagagca atactctgt                                                    19

<210>  397
<211>  19
<212>  DNA
<213>  Artificially synthesized

<400>  397
agagcaatac tctgtcgca                                                    19

<210>  398
<211>  19
<212>  DNA
<213>  Artificially synthesized

<400>  398

aaaagaatac tggaggctg                                                    19

<210>  399
<211>  19
<212>  DNA
<213>  Artificially synthesized

<400>  399
aaagaatact ggaggctgg                                                    19

<210>  400
<211>  19
<212>  DNA
<213>  Artificially synthesized

<400>  400
atactggagg ctgggcgag                                                    19

<210>  401
<211>  19
<212>  DNA
<213>  Artificially synthesized

<400>  401
cgaggtggct cacacctgt                                                    19

<210>  402
<211>  19
<212>  DNA
<213>  Artificially synthesized

<400>  402
ggtggctcac acctgtaat                                                    19

<210>  403
<211>  19
<212>  DNA
<213>  Artificially synthesized

<400>  403
ggctcacacc tgtaatccc                                                    19

<210>  404
<211>  19
<212>  DNA
<213>  Artificially synthesized

<400>  404
gctcacacct gtaatccca                                                    19

<210>  405
<211>  19
<212>  DNA
<213>  Artificially synthesized

<400>  405

cacacctgta atcccagca                                                    19

<210>  406
<211>  19
<212>  DNA
<213>  Artificially synthesized

<400>  406
taatcccagc attttggga                                                    19

<210>  407
<211>  19
<212>  DNA
<213>  Artificially synthesized

<400>  407
gggcggaata tcttgagct                                                    19

<210>  408
<211>  19
<212>  DNA
<213>  Artificially synthesized

<400>  408
aatatcttga gctcaggag                                                    19

<210>  409
<211>  19
<212>  DNA
<213>  Artificially synthesized

<400>  409
ttcgagacca gcctacaca                                                    19

<210>  410
<211>  19
<212>  DNA
<213>  Artificially synthesized

<400>  410
ccagcctaca caatatgct                                                    19

<210>  411
<211>  19
<212>  DNA
<213>  Artificially synthesized

<400>  411
acacaatatg ctccaaacg                                                    19

<210>  412
<211>  19
<212>  DNA
<213>  Artificially synthesized

<400>  412

cacaatatgc tccaaacgc                                                    19

<210>  413
<211>  19
<212>  DNA
<213>  Artificially synthesized

<400>  413
acaatatgct ccaaacgcc                                                    19

<210>  414
<211>  19
<212>  DNA
<213>  Artificially synthesized

<400>  414
caaacgccgc ctctacaaa                                                    19

<210>  415
<211>  19
<212>  DNA
<213>  Artificially synthesized

<400>  415
ctgtggtcct agctacttg                                                    19

<210>  416
<211>  19
<212>  DNA
<213>  Artificially synthesized

<400>  416
gggaggatcg cttgagctc                                                    19

<210>  417
<211>  19
<212>  DNA
<213>  Artificially synthesized

<400>  417
gaggatcgct tgagctcgg                                                    19

<210>  418
<211>  19
<212>  DNA
<213>  Artificially synthesized

<400>  418
ggaggtcgag gctgcaatg                                                    19

<210>  419
<211>  19
<212>  DNA
<213>  Artificially synthesized

<400>  419

ggtcgaggct gcaatgagc                                                    19

<210>  420
<211>  19
<212>  DNA
<213>  Artificially synthesized

<400>  420
caatgagccg agatggtgc                                                    19

<210>  421
<211>  19
<212>  DNA
<213>  Artificially synthesized

<400>  421
aatgagccga gatggtgcc                                                    19

<210>  422
<211>  19
<212>  DNA
<213>  Artificially synthesized

<400>  422
ccgagatggt gccactgca                                                    19

<210>  423
<211>  19
<212>  DNA
<213>  Artificially synthesized

<400>  423
gagatggtgc cactgcact                                                    19

<210>  424
<211>  19
<212>  DNA
<213>  Artificially synthesized

<400>  424
agatggtgcc actgcactc                                                    19

<210>  425
<211>  19
<212>  DNA
<213>  Artificially synthesized

<400>  425
atggtgccac tgcactctg                                                    19

<210>  426
<211>  19
<212>  DNA
<213>  Artificially synthesized

<400>  426

ccactgcact ctgacgaca                                                    19

<210>  427
<211>  19
<212>  DNA
<213>  Artificially synthesized

<400>  427
cactgcactc tgacgacag                                                    19

<210>  428
<211>  19
<212>  DNA
<213>  Artificially synthesized

<400>  428
tctgacgaca gagcgagac                                                    19

<210>  429
<211>  19
<212>  DNA
<213>  Artificially synthesized

<400>  429
ctgacgacag agcgagact                                                    19

<210>  430
<211>  19
<212>  DNA
<213>  Artificially synthesized

<400>  430
gagactccgt ctcaaaaca                                                    19

<210>  431
<211>  19
<212>  DNA
<213>  Artificially synthesized

<400>  431
agactccgtc tcaaaacaa                                                    19

<210>  432
<211>  19
<212>  DNA
<213>  Artificially synthesized

<400>  432
tctagtgttt aaggatctg                                                    19

<210>  433
<211>  19
<212>  DNA
<213>  Artificially synthesized

<400>  433

tagtgtttaa ggatctgcc                                                    19

<210>  434
<211>  19
<212>  DNA
<213>  Artificially synthesized

<400>  434
tgtttaagga tctgccttc                                                    19

<210>  435
<211>  19
<212>  DNA
<213>  Artificially synthesized

<400>  435
ggatctgcct tccttcctg                                                    19

<210>  436
<211>  19
<212>  DNA
<213>  Artificially synthesized

<400>  436
ttgtctttcc ttgtttgtc                                                    19

<210>  437
<211>  19
<212>  DNA
<213>  Artificially synthesized

<400>  437
gtctttcctt gtttgtctt                                                    19

<210>  438
<211>  19
<212>  DNA
<213>  Artificially synthesized

<400>  438
caagcaggtt ttaaattcc                                                    19

<210>  439
<211>  19
<212>  DNA
<213>  Artificially synthesized

<400>  439
gcaggtttta aattcctag                                                    19

<210>  440
<211>  19
<212>  DNA
<213>  Artificially synthesized

<400>  440

acatttactt ttccaaggg                                                    19

<210>  441
<211>  19
<212>  DNA
<213>  Artificially synthesized

<400>  441
acactggagt tcgagacga                                                    19

<210>  442
<211>  19
<212>  DNA
<213>  Artificially synthesized

<400>  442
ctggagttcg agacgaggc                                                    19

<210>  443
<211>  19
<212>  DNA
<213>  Artificially synthesized

<400>  443
ttcgagacga ggcctaagc                                                    19

<210>  444
<211>  19
<212>  DNA
<213>  Artificially synthesized

<400>  444
gagacgaggc ctaagcaac                                                    19

<210>  445
<211>  19
<212>  DNA
<213>  Artificially synthesized

<400>  445
agacgaggcc taagcaaca                                                    19

<210>  446
<211>  19
<212>  DNA
<213>  Artificially synthesized

<400>  446
gacgaggcct aagcaacat                                                    19

<210>  447
<211>  19
<212>  DNA
<213>  Artificially synthesized

<400>  447

cctaagcaac atgccgaaa                                                    19

<210>  448
<211>  19
<212>  DNA
<213>  Artificially synthesized

<400>  448
ctaagcaaca tgccgaaac                                                    19

<210>  449
<211>  19
<212>  DNA
<213>  Artificially synthesized

<400>  449
taagcaacat gccgaaacc                                                    19

<210>  450
<211>  19
<212>  DNA
<213>  Artificially synthesized

<400>  450
tggtggcgca cgcctatag                                                    19

<210>  451
<211>  19
<212>  DNA
<213>  Artificially synthesized

<400>  451
ggtggcgcac gcctatagt                                                    19

<210>  452
<211>  19
<212>  DNA
<213>  Artificially synthesized

<400>  452
ctatagtcct agctactgg                                                    19

<210>  453
<211>  19
<212>  DNA
<213>  Artificially synthesized

<400>  453
tatagtccta gctactggg                                                    19

<210>  454
<211>  19
<212>  DNA
<213>  Artificially synthesized

<400>  454

tgaggtggga ggatcgctt 19

<210> 455
<211> 19
<212> DNA
<213> Artificially synthesized

<400> 455
ctgcagtgag ccgagatcg 19

<210> 456
<211> 19
<212> DNA
<213> Artificially synthesized

<400> 456
tgcagtgagc cgagatcgc 19

<210> 457
<211> 19
<212> DNA
<213> Artificially synthesized

<400> 457
tgcactccag cctgagcga 19

<210> 458
<211> 19
<212> DNA
<213> Artificially synthesized

<400> 458
cactccagcc tgagcgaca 19

<210> 459
<211> 19
<212> DNA
<213> Artificially synthesized

<400> 459
acagggcgag gctctgtct 19

<210> 460
<211> 19
<212> DNA
<213> Artificially synthesized

<400> 460
gggcgaggct ctgtctcaa 19

<210> 461
<211> 19
<212> DNA
<213> Artificially synthesized

<400> 461

ggcgaggctc tgtctcaaa                                                    19

<210>  462
<211>  19
<212>  DNA
<213>  Artificially synthesized

<400>  462
gcgaggctct gtctcaaaa                                                    19

<210>  463
<211>  19
<212>  DNA
<213>  Artificially synthesized

<400>  463
aggctctgtc tcaaaacaa                                                    19

<210>  464
<211>  19
<212>  DNA
<213>  Artificially synthesized

<400>  464
ggctctgtct caaaacaaa                                                    19

<210>  465
<211>  19
<212>  DNA
<213>  Artificially synthesized

<400>  465
gctctgtctc aaaacaaac                                                    19

<210>  466
<211>  19
<212>  DNA
<213>  Artificially synthesized

<400>  466
ctctgtctca aaacaaaca                                                    19

<210>  467
<211>  19
<212>  DNA
<213>  Artificially synthesized

<400>  467
aacacagtga aatgaaagg                                                    19

<210>  468
<211>  19
<212>  DNA
<213>  Artificially synthesized

<400>  468

acacagtgaa atgaaagga                                                    19

<210>  469
<211>  19
<212>  DNA
<213>  Artificially synthesized

<400>  469
cagtgaaatg aaaggattg                                                    19

<210>  470
<211>  19
<212>  DNA
<213>  Artificially synthesized

<400>  470
gtgaaatgaa aggattgag                                                    19

<210>  471
<211>  19
<212>  DNA
<213>  Artificially synthesized

<400>  471
gaaatgaaag gattgagag                                                    19

<210>  472
<211>  19
<212>  DNA/RNA
<213>  Artificially synthesized

<400>  472
acuacugagu gacaguagatt                                                    21

<210>  473
<211>  19
<212>  DNA/RNA
<213>  Artificially synthesized

<400>  473
ucuacuguca cucaguagutt                                                    21

<210>  474
<211>  19
<212>  DNA/RNA
<213>  Artificially synthesized

<400>  474
gggaugauac agcacugautt                                                    21

<210>  475
<211>  19
<212>  DNA/RNA
<213>  Artificially synthesized

<400>  475

aucagugcug uaucaucccctt                                                21

<210>  476
<211>  159
<212>  DNA
<213>  Artificially synthesized

<400>  476
agtcgcactc tgtcactcag gctggagtgc agtggcgtga tcttggctca ctgcaacctc      60

cgcctcccga gttcaagtga ttctcctggc tcagcctccc aagcagctgt cattacaggc     120

ctgcaccacc acacccggct gatttttgta tttttagga                            159

<210>  477
<211>  82
<212>  DNA
<213>  Artificially synthesized

<400>  477
aatactggag gcccggtgtg gtggctcaca cctgtaatcc cagcactttg ggaggccgag      60

gcggtcggat tacgaggtca gg                                               82

<210>  478
<211>  815
<212>  DNA
<213>  Artificially synthesized

<400>  478
ctggccaaca tggtgaaacc ccatctttac taaaaataca aaaattagcc gggtgtggtg      60

gtgggcgcct gtaatcccag ctactcgggg ggctgaggca gaattgcttg aacctgggag     120

gcagaggttg cagtgagctg agatcacgcc actgcattcc agcctgggtg acagagcaat     180

actctgtcgc aaaaaaaaaa aagaatactg gaggctgggc gaggtggctc acacctgtaa     240

tcccagcatt ttgggatgcc agaggcgggc ggaatatctt gagctcagga gttcgagacc     300

agcctacaca atatgctcca aacgccgcct ctacaaaaca tacagaaact agccgggtgt     360

ggtggcgtgc ccctgtggtc ctagctactt gggaggttga ggcgggagga tcgcttgagc     420

tcgggaggtc gaggctgcaa tgagccgaga tggtgccact gcactctgac gacagagcga     480

gactccgtct caaaacaaac aacaaataag gttgggggat caaatatctt ctagtgttta     540

aggatctgcc ttccttcctg cccccatgtt tgtctttcct tgtttgtctt tatatagatc     600

aagcaggttt taaattccta gtaggagctt acatttactt ttccaagggg gagggggaat     660

aaatatctac acacacacac acacacacac acacacacac acactggagt tcgagacgag     720

gcctaagcaa catgccgaaa ccccgtctct actaaataca aaaaatagct gagcttggtg     780

gcgcacgcct atagtcctag ctactgggga ggctg                                815

<210>  479

<211>  108
<212>  DNA
<213>  Artificially synthesized

<400>  479
ctgcagtgag ccgagatcgc gccgctgcac tccagcctga gcgacagggc gaggctctgt      60

ctcaaaacaa acaaacaaaa aaaaaaggaa aggaaatata acacagtg                  108


<210>  480
<211>  18
<212>  DNA
<213>  Artificially synthesized

<400>  480
cacaggccag agcgatga                                                    18


<210>  481
<211>  23
<212>  DNA
<213>  Artificially synthesized

<400>  481
cgaagtttca caaatgtcac cat                                              23


<210>  482
<211>  21
<212>  DNA
<213>  Artificially synthesized

<400>  482
atggacagga ctgaacgtct t                                                21


<210>  483
<211>  20
<212>  DNA
<213>  Artificially synthesized

<400>  483
tccagcaggt cagcaaagaa                                                  20


<210>  484
<211>  20
<212>  DNA
<213>  Artificially synthesized

<400>  484
ataatcccaa gcggtttgct                                                  20


<210>  485
<211>  20
<212>  DNA
<213>  Artificially synthesized

<400>  485
ctgccagtct ggactgttct                                                  20

<210>  486
<211>  20
<212>  DNA
<213>  Artificially synthesized

<400>  486
ccaccacctc ccatatgtcc                                                    20


<210>  487
<211>  22
<212>  DNA
<213>  Artificially synthesized

<400>  487
gctctatgcc agcatttctc ct                                                 22


<210>  488
<211>  20
<212>  DNA
<213>  Artificially synthesized

<400>  488
cccccaccac ctcccatatg                                                    20


<210>  489
<211>  25
<212>  DNA
<213>  Artificially synthesized

<400>  489
cccttctcac agctcataaa attac                                              25


<210>  490
<211>  33
<212>  DNA
<213>  Artificially synthesized

<400>  490
taagcaggat ccatggcgat gagcagcggc ggc                                     33


<210>  491
<211>  33
<212>  DNA
<213>  Artificially synthesized

<400>  491
taagcagaat tcttaattta aggaatgtga gca                                     33